# EUROPEAN PATENT APPLICATION

(11) **EP 3 662 911 A1**
(43) Date of publication of application: **10.06.2020**
(21) Application number: 19206787.4
(22) Date of filing: 30.10.2013
(51) Int. Cl.: A61K 31/706, A61K 31/4184

(54) **COMBINATION OF AZACITIDINE AND PRACINOSTAT TO TREAT MYELODYSPLASTIC SYNDROME (MDS)**

(30) Priority: 30.10.2012 US 201261720346 P
(62) Divisional of application: 13851971.5
(71) Applicant: MEI Pharma, Inc., San Diego, CA 92130 (US)
(72) Inventor: GOLD, Daniel, P., San Diego, CA California 92130 (US); GARCIA-MANERO, Guillermo, Houston, TX Texas 77030 (US)
(74) Representative: Avidity IP

(57) **Abstract**

Provided herein are methods of treating and sensitizing cancer comprising administering a heterocyclic compound and a DNA hypomethylating agent.

## Description

### CROSS-REFERENCE TO RELATED APPLICATIONS

This application claims the benefit of U.S. Application Serial No. 61/720,346, filed October 30, 2012, of which is hereby incorporated by reference in its entirety.

### BACKGROUND OF THE INVENTION

Local chromatin architecture is generally recognized as an important factor in the regulation of gene expression. The architecture of chromatin, a protein-DNA complex, is strongly influenced by post-translational modifications of the histones which are the protein components. Reversible acetylation of histones is a key component in the regulation of gene expression by altering the accessibility of transcription factors to DNA. In general, increased levels of histone acetylation are associated with increased transcriptional activity, whereas decreased levels of acetylation are associated with repression of gene expression [Wadem P.A. Hum. Mol. Genet. 10, 693-698 (2001), De Ruijter A.J.M. et al, Biochem. J., 370, 737-749 (2003)]. In normal cells, histone deacetylases (HDACs) and histone acetyltransferase together control the level of acetylation of histones to maintain a balance. Inhibition of HDACs results in the accumulation of acetylated histones, which results in a variety of cell type dependent cellular responses, such as apoptosis, necrosis, differentiation, cell survival, inhibition of proliferation and cytostasis.

Inhibitors of HDAC have been studied for their therapeutic effects on cancer cells. For example, suberoylanilide hydroxamic acid (SAHA) is a potent inducer of differentiation and/or apoptosis in murine erythroleukemia, bladder, and myeloma cell lines [Richon V.M. et al, Proc. Natl. Acad. Sci. USA, 93: 5705-5708 (1996), Richon V.M. et al, Proc. Natl. Acad. Sci. USA, 95: 3003-3007 (1998)]. SAHA has been shown to suppress the growth of prostate cancer cells *in vitro* and *in vivo* [Butler L.M. et al, Cancer Res. 60, 5165-5170 (2000)]. Other inhibitors of HDAC that have been widely studied for their anti-cancer activities are trichostatin A (TSA) and trapoxin B [Yoshida M. et al, J. Biol. Chem., 265, 17174 (1990), Kijima M. et al, J. Biol. Chem., 268, 22429 (1993)]. Trichostatin A is a reversible inhibitor of mammalian HDAC. Trapoxin B is a cyclic tetrapeptide, which is an irreversible inhibitor of mammalian HDAC. However, due to the *in vivo* instability of these compounds they are less desirable as anti-cancer drugs. Recently, other small molecule HDAC inhibitors have become available for clinical evaluation [US 6,552,065]. Additional HDAC inhibiting compounds have been reported in the literature [Bouchain G. et al, J. Med. Chem., 46, 820-830 (2003)] and patents [WO 03/066579A2]. The in vivo activity of such inhibitors can be directly monitored by their ability to increase the amount of acetylated histones in the biological sample. HDAC inhibitors have been reported to interfere with neurodegenerative processes, for instance, HDAC inhibitors arrest polyglutamine-dependent neurodegeneration [Nature, 413(6857): 739-43, 18 October, 2001]. In addition, HDAC inhibitors have also been known to inhibit production of cytokines such as TNF, IFN, IL-1 which are known to be implicated in inflammatory diseases and/or immune system disorders. [J. Biol. Chem. 1990; 265(18): 10232-10237; Science, 1998; 281: 1001-1005; Dinarello C.A. and Moldawer L.L. Proinflammatory and anti-inflammatory cytokines in rheumatoid arthritis, A primer for clinicians, 3rd Edition, Amgen Inc., 2002].

Nevertheless, there is still a need to provide further HDAC inhibitor combinations that would be expected to have useful, improved pharmaceutical properties in the treatment of diseases such as cancer, neurodegenerative diseases, disorders involving angiogenesis and inflammatory and/or immune system disorders.

### SUMMARY OF THE INVENTION

Provided herein in certain embodiments is a method of treating a disease or disorder associated with dysregulation of histone deacetylase, comprising administering to a subject in need thereof an effective amount of: (i) a DNA hypomethylating agent; and (ii) a compound of formula (I): 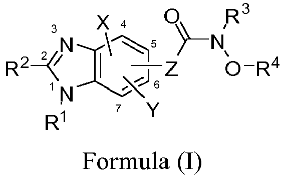 wherein R
¹ is an optionally substituted heteroaryl group, an optionally substituted heterocycloalkyl group or a group of formula:

   -(CR²⁰R²¹)ₘ-(CR²²R²³)ₙ-(CR²⁴R²⁵)ₒ-NR²⁶R²⁷;
R² is selected from the group consisting of: H, alkyl, alkenyl, alkynyl, heteroalkyl, cycloalkyl, cycloalkenyl, cycloalkylalkyl, alkoxyalkyl, R¹¹S(O)R¹³-, R¹¹S(O)₂R¹³-, R¹¹C(O)N(R¹²)R¹³-, R¹¹SO₂N(R¹²)R¹³-, R¹¹N(R¹²)C(O)R¹³-, R¹¹N(R¹²)SO₂R¹³-, R¹¹N(R¹²)C(O)N(R¹²)R¹³- and acyl, each of which may be optionally substituted;
R³ is selected from the group consisting of H, C₁ -C₆ alkyl, and acyl, each of which may be optionally substituted;
X and Y are the same or different and are independently selected from the group consisting of: H, halogen, -CN, -NO₂, -CF₃, -OCF₃, alkyl, alkenyl, alkynyl, haloalkyl, haloalkenyl, haloalkynyl, heteroalkyl, cycloalkyl, cycloalkenyl, heterocycloalkyl, heterocycloalkenyl, aryl, heteroaryl, hydroxy, hydroxyalkyl, alkoxy, alkoxyalkyl, alkoxyaryl, alkoxyheteroaryl, alkenyloxy, alkynyloxy, cycloalkyloxy, cycloalkenyloxy, heterocycloalkyloxy, heterocycloalkenyloxy, aryloxy, heteroaryloxy, arylalkyl, heteroarylalkyl, arylalkyloxy, -amino, alkylamino, acylamino, aminoalkyl, arylamino, sulfonyl, alkylsulfonyl, arylsulfonyl, aminosulfonyl, aminoalkyl, alkoxyalky, -COOH-C(O)OR⁵, -COR⁵, - SH, -SR⁶, -OR⁶ acyl and -NR⁷R⁸, each of which may be optionally substituted;
R⁴ is selected from the group consisting of: H, alkyl, alkenyl, alkynyl, haloalkyl, heteroalkyl, cycloalkyl, heterocycloalkyl, aryl, heteroaryl, cycloalkylalkyl, heterocycloalkylalkyl, arylalkyl, heteroarylalkyl and acyl, each of which may be optionally substituted;
each R⁵ is independently selected from the group consisting of: H, alkyl, alkenyl, alkynyl, haloalkyl, heteroalkyl, cycloalkyl, heterocycloalkyl, aryl, heteroaryl, cycloalkylalkyl, heterocycloalkylalkyl, arylalkyl, heteroarylalkyl and acyl, each of which may be optionally substituted;
each R⁶ is independently selected from the group consisting of: H, alkyl, alkenyl, alkynyl, haloalkyl, heteroalkyl, cycloalkyl, heterocycloalkyl, aryl, heteroaryl, cycloalkylalkyl, heterocycloalkylalkyl, arylalkyl, heteroarylalkyl and acyl; each of which may be optionally substituted;
each R⁷ and R⁸ is independently selected from the group consisting of: H, alkyl, alkenyl, alkynyl, haloalkyl, heteroalkyl, cycloalkyl, heterocycloalkyl, aryl, heteroaryl, cycloalkylalkyl, heterocycloalkylalkyl, arylalkyl, heteroarylalkyl and acyl, each of which may be optionally substituted;
each R¹¹ and R¹² is independently selected from the group consisting of H, alkyl, alkenyl, and alkynyl, each of which may be optionally substituted;
each R¹³ is a bond or is independently selected from the group consisting of: alkyl, alkenyl, and alkynyl, each of which may be optionally substituted;
each R²⁰, R²¹, R²², R²³, R²⁴ and R²⁵ is independently selected from the group consisting of: H, halogen, -CN, -NO₂, -CF₃, -OCF₃, alkyl, alkenyl, alkynyl, haloalkyl, haloalkenyl, haloalkynyl, heteroalkyl, cycloalkyl, cycloalkenyl, heterocycloalkyl, heterocycloalkenyl, aryl, heteroaryl, cycloalkylalkyl, heterocycloalkylalkyl, arylalkyl, heteroarylalkyl, arylalkenyl, cycloalkylheteroalkyl, heterocycloalkylheteroalkyl, heteroaryiheteroalkyl, arylheteroalkyl, hydroxy, hydroxyalkyl, alkoxy, alkoxyalkyl, alkoxyaryl, alkoxyheteroaryl, alkenyloxy, alkynyloxy, cycloalkylkoxy, heterocycloalkyloxy, aryloxy, arylalkyloxy, phenoxy, benzyloxy heteroaryloxy, amino, alkylamino, acylamino, aminoalkyl, arylamino, alkoxycarbonyl, alkylaminocarbonyl, sulfonyl, alkylsulfonyl, aminosulfonyl, arylsulfonyl, arylsulfinyl -COOH, -C(O)OR⁵,-COR⁵, -SH, -SR⁶, -OR⁶ and acyl, each of which may be optionally substituted; or
R²⁰ and R²¹ when taken together may form a group of formula =O or =S, and/or
R²² and R²³ when taken together may form a group of formula =O or =S, and/or
R²⁴ and R²⁵ when taken together may form a group of formula =O or =S;
each R²⁶ and R²⁷ is independently selected from the group consisting of: H, halogen, alkyl, alkenyl, alkynyl, haloalkyl, haloalkenyl, heteroalkyl, cycloalkyl, cycloalkenyl, heterocycloalkyl, heterocycloalkenyl, aryl, heteroaryl, cycloalkylalkyl, heterocycloalkylalkyl, arylalkyl, heteroarylalkyl, arylalkenyl, cycloalkylheteroalkyl, heterocycloalkylheteroalkyl, heteroarylheteroalkyl, arylheteroalkyl, hydroxy, hydroxyalkyl, alkoxy, alkoxyalkyl, alkoxyaryl, alkenyloxy, alkynyloxy, cycloalkylkoxy, heterocycloalkyloxy, aryloxy, arylalkyloxy, heteroaryloxy, amino, alkylamino, aminoalkyl, acylamino, arylamino, phenoxy, benzyloxy, COOH, alkoxycarbonyl, alkylaminocarbonyl, sulfonyl, alkylsulfonyl, alkylsulfinyl, arylsulfonyl, arylsulfinyl, aminosulfonyl, SW and acyl, each of which may be optionally substituted, or R²⁶ and R²⁷ when taken together with the nitrogen atom to which they are attached form an optionally substituted heterocycloalkyl group;
Z is selected from the group consisting of -CH₂-, -CH₂CH₂-, -CH=CH-, C₃-C₆ alkylene, C₃-C₆ alkenylene, C₃-C₆ alkynylene, C₃-C₆ cycloalkyl, unsubstituted or substituted with one or more substituents independently selected from the group consisting of C₁-C₄ alkyl;
m, n and o are integers independently selected from the group consisting of 0, 1, 2, 3 and 4;
or a pharmaceutically acceptable salt or prodrug thereof.

In some embodiments, the compound of formula (I) has the structure of formula (Id): 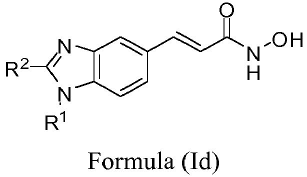 wherein
R¹ is a group having the formula:

   -(CR²⁰R²¹)*ₘ*-(CR²²R²³)*ₙ*-(CR²⁴R²⁵)*ₒ*-NR²⁶R²⁷;
R² is alkyl, fluoroalkyl, cyano, C₂-C₆alkenyl, C₂-C₆alkynyl, or heteroalkyl optionally substituted with =O;
each R²⁰, R²¹, R²², R²³, R²⁴, and R²⁵ is independently H or methyl;
each R²⁶ and R²⁷ is independently H, hydroxylalkyl, or alkyl; and
m, n, and o are independently integers of 0, 1, 2, 3, or 4;
or a pharmaceutically acceptable salt or prodrug thereof.

In some embodiments, the compound of formula (I) has the structure of formula (Ie) or (If): 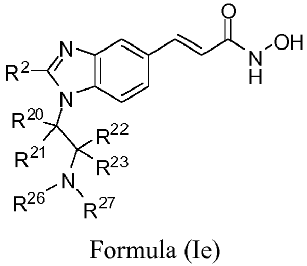 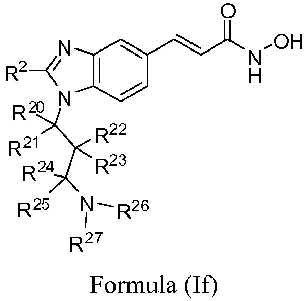 wherein
R² is alkyl, fluoroalkyl, cyano, C₂-C₆alkenyl, C₂-C₆alkynyl, or heteroalkyl optionally substituted with =O;
each R²⁰, R²¹, R²², R²³, R²⁴, and R²⁵ is independently H or methyl; and
each R²⁶ and R²⁷ is independently H, hydroxylalkyl, or alkyl;
or a pharmaceutically acceptable salt or prodrug thereof.

Described herein in some embodiments is a compound of formulas (I), (Id), (Ie), or (If) wherein R²⁶ and R²⁷ are independently H or alkyl. In specific embodiments, R²⁶ and R²⁷ are independently H, methyl, ethyl, isopropyl, propyl, butyl, isobutyl, pentyl, hexyl or heptyl. In some embodiments, R¹ has the structure:

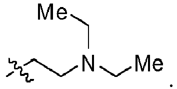

In some embodiments, R² is ethyl, 1-methyl-ethyl, 2,2,2-trifluoroethyl, propyl, 2-methyl-propyl, 2,2-dimethyl-propyl, 3,3,3-trifluoro-propyl, butyl, 3,3-dimethyl-butyl, pentyl, 2,4,4-trimethyl-pentyl, hexyl or octyl. In some specific embodiments, R² is butyl.

In some embodiments, the compound of formula (I) is 3-[2-Butyl-1-(2-diethylaminoethyl)-1H-benzimidazol-5-yl]-N-hydroxy-acrylamide. In some embodiments, the compound of formula (I) has the structure:

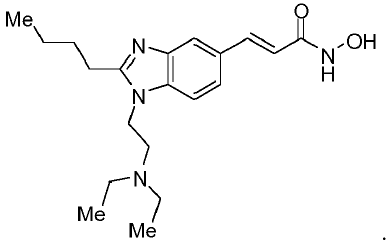

In some embodiments, the DNA hypomethylating agent is 5-azacytidine (azacitidine), 5-azadeoxycytidine (decitabine), SGI-110, zebularine or procaine. In certain specific embodiments, the DNA hypomethylating agent is 5-azacytidine (azacitidine).

In some embodiments, the disease or disorder associated with dysregulation of histone deacetylase is cancer. In some embodiments, the cancer is a hematological malignancy. In some embodiments, wherein the hematological malignancy is acute myeloid leukemia (AML), chronic myeloid leukemia (CML), chronic myelomonocytic leukemia, thrombolytic leukemia, a myelodysplastic syndrome (MDS), a myeloproliferative disorder, refractory anemia, a preleukemia syndrome, a lymphoid leukemia, lymphoma, non-Hodgkin's lymphoma, or an undifferentiated leukemia. In some specific embodiments, the cancer is myelodysplastic syndrome (MDS) or acute myeloid leukemia (AML). In other specific embodiments, the non-Hodgkin's lymphoma is diffuse large B-cell lymphoma (DLBCL), mantle cell lymphoma (MCL), or chronic lymphocytic leukemia (CLL). In some embodiments, the cancer is refractory, non-responsive or resistant to chemotherapy and/or haploidentical stem cell transplantation. In some embodiments, the cancer is resistant to azacitidine, decitabine, SGI-110, lenalidomide, TXA-127, or combinations thereof. In some embodiments, the cancer is resistant to azacitidine, decitabine, lenalidomide, TXA-127, or combinations thereof.

In some embodiments, a compound of formula (I), (Id), (Ie) or (If) is administered in an amount from 5 mg to 120 mg. In some embodiments, the compound of formula (I) is administered in an amount of about 60 mg. In some embodiments, the DNA hypomethylating agent is administered in an amount from 5 mg/m² to 125 mg/m². In other embodiments, the DNA hypomethylating agent is administered in an amount of 75 mg/m². In some embodiments, the compound of formula (I) is administered orally and the DNA hypomethylating agent is administered intravenously or subcutaneously. In some embodiments, the DNA hypomethylating agent is administered intravenously.

Provided herein in certain embodiments are methods of treating chemoresistant cancer comprising administering to a subject in need thereof an effective amount of: (i) a DNA hypomethylating agent; and (ii) a compound of formula (I): 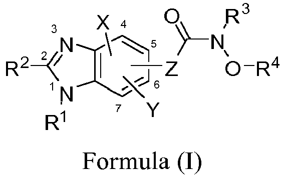 wherein
R¹ is an optionally substituted heteroaryl group, an optionally substituted heterocycloalkyl group or a group of formula:

   -(CR²⁰R²¹)ₘ-(CR²²R²³)ₙ-(CR²⁴R²⁵)ₒ-NR²⁶R²⁷;
R² is selected from the group consisting of: H, alkyl, alkenyl, alkynyl, heteroalkyl, cycloalkyl, cycloalkenyl, cycloalkylalkyl, alkoxyalkyl, R¹¹S(O)R¹³-, R¹¹S(O)₂R¹³-, R¹¹C(O)N(R¹²)R¹³-, R¹¹SO₂N(R¹²)R¹³-, R¹¹N(R¹²)C(O)R¹³-, R¹¹N(R¹²)SO₂R¹³-, R¹¹N(R¹²)C(O)N(R¹²)R¹³- and acyl, each of which may be optionally substituted;
R³ is selected from the group consisting of H, C₁ -C₆ alkyl, and acyl, each of which may be optionally substituted;
X and Y are the same or different and are independently selected from the group consisting of: H, halogen, -CN, -NO₂, -CF₃, -OCF₃, alkyl, alkenyl, alkynyl, haloalkyl, haloalkenyl, haloalkynyl, heteroalkyl, cycloalkyl, cycloalkenyl, heterocycloalkyl, heterocycloalkenyl, aryl, heteroaryl, hydroxy, hydroxyalkyl, alkoxy, alkoxyalkyl, alkoxyaryl, alkoxyheteroaryl, alkenyloxy, alkynyloxy, cycloalkyloxy, cycloalkenyloxy, heterocycloalkyloxy, heterocycloalkenyloxy, aryloxy, heteroaryloxy, arylalkyl, heteroarylalkyl, arylalkyloxy, -amino, alkylamino, acylamino, aminoalkyl, arylamino, sulfonyl, alkylsulfonyl, arylsulfonyl, aminosulfonyl, aminoalkyl, alkoxyalky, -COOH-C(O)OR⁵, -COR⁵,-SH, -SR⁶, -OR⁶ acyl and -NR⁷R⁸, each of which may be optionally substituted;
R⁴ is selected from the group consisting of: H, alkyl, alkenyl, alkynyl, haloalkyl, heteroalkyl, cycloalkyl, heterocycloalkyl, aryl, heteroaryl, cycloalkylalkyl, heterocycloalkylalkyl, arylalkyl, heteroarylalkyl and acyl, each of which may be optionally substituted;
each R⁵ is independently selected from the group consisting of: H, alkyl, alkenyl, alkynyl, haloalkyl, heteroalkyl, cycloalkyl, heterocycloalkyl, aryl, heteroaryl, cycloalkylalkyl, heterocycloalkylalkyl, arylalkyl, heteroarylalkyl and acyl, each of which may be optionally substituted;
each R⁶ is independently selected from the group consisting of: H, alkyl, alkenyl, alkynyl, haloalkyl, heteroalkyl, cycloalkyl, heterocycloalkyl, aryl, heteroaryl, cycloalkylalkyl, heterocycloalkylalkyl, arylalkyl, heteroarylalkyl and acyl; each of which may be optionally substituted;
each R⁷ and R⁸ is independently selected from the group consisting of: H, alkyl, alkenyl, alkynyl, haloalkyl, heteroalkyl, cycloalkyl, heterocycloalkyl, aryl, heteroaryl, cycloalkylalkyl, heterocycloalkylalkyl, arylalkyl, heteroarylalkyl and acyl, each of which may be optionally substituted;
each R¹¹ and R¹² is independently selected from the group consisting of H, alkyl, alkenyl, and alkynyl, each of which may be optionally substituted;
each R¹³ is a bond or is independently selected from the group consisting of: alkyl, alkenyl, and alkynyl, each of which may be optionally substituted;
each R²⁰, R²¹, R²², R²³, R²⁴ and R²⁵ is independently selected from the group consisting of: H, halogen, -CN, -NO₂, -CF₃, -OCF₃, alkyl, alkenyl, alkynyl, haloalkyl, haloalkenyl, haloalkynyl, heteroalkyl, cycloalkyl, cycloalkenyl, heterocycloalkyl, heterocycloalkenyl, aryl, heteroaryl, cycloalkylalkyl, heterocycloalkylalkyl, arylalkyl, heteroarylalkyl, arylalkenyl, cycloalkylheteroalkyl, heterocycloalkylheteroalkyl, heteroaryiheteroalkyl, arylheteroalkyl, hydroxy, hydroxyalkyl, alkoxy, alkoxyalkyl, alkoxyaryl, alkoxyheteroaryl, alkenyloxy, alkynyloxy, cycloalkylkoxy, heterocycloalkyloxy, aryloxy, arylalkyloxy, phenoxy, benzyloxy heteroaryloxy, amino, alkylamino, acylamino, aminoalkyl, arylamino, alkoxycarbonyl, alkylaminocarbonyl, sulfonyl, alkylsulfonyl, aminosulfonyl, arylsulfonyl, arylsulfinyl -COOH, -C(O)OR⁵,-COR⁵, -SH, -SR⁶, -OR⁶ and acyl, each of which may be optionally substituted; or
R²⁰ and R²¹ when taken together may form a group of formula =O or =S, and/or
R²² and R²³ when taken together may form a group of formula =O or =S, and/or
R²⁴ and R²⁵ when taken together may form a group of formula =O or =S;
each R²⁶ and R²⁷ is independently selected from the group consisting of: H, halogen, alkyl, alkenyl, alkynyl, haloalkyl, haloalkenyl, heteroalkyl, cycloalkyl, cycloalkenyl, heterocycloalkyl, heterocycloalkenyl, aryl, heteroaryl, cycloalkylalkyl, heterocycloalkylalkyl, arylalkyl, heteroarylalkyl, arylalkenyl, cycloalkylheteroalkyl, heterocycloalkylheteroalkyl, heteroarylheteroalkyl, arylheteroalkyl, hydroxy, hydroxyalkyl, alkoxy, alkoxyalkyl, alkoxyaryl, alkenyloxy, alkynyloxy, cycloalkylkoxy, heterocycloalkyloxy, aryloxy, arylalkyloxy, heteroaryloxy, amino, alkylamino, aminoalkyl, acylamino, arylamino, phenoxy, benzyloxy, COOH, alkoxycarbonyl, alkylaminocarbonyl, sulfonyl, alkylsulfonyl, alkylsulfinyl, arylsulfonyl, arylsulfinyl, aminosulfonyl, SW and acyl, each of which may be optionally substituted, or R²⁶ and R²⁷ when taken together with the nitrogen atom to which they are attached form an optionally substituted heterocycloalkyl group;
Z is selected from the group consisting of -CH₂-, -CH₂CH₂-, -CH=CH-, C₃-C₆ alkylene, C₃-C₆ alkenylene, C₃-C₆ alkynylene, C₃-C₆ cycloalkyl, unsubstituted or substituted with one or more substituents independently selected from the group consisting of C₁-C₄ alkyl;
m, n and o are integers independently selected from the group consisting of 0, 1, 2, 3 and 4;
or a pharmaceutically acceptable salt or prodrug thereof.

In some embodiments, the compound of formula (I) has the structure of formula (Id): 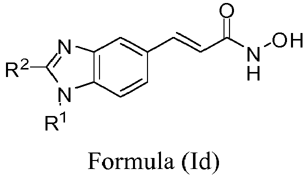 wherein
R¹ is a group having the formula:

   -(CR²⁰R²¹)*ₘ*-(CR²²R²³)*ₙ*-(CR²⁴R²⁵)*ₒ*-NR²⁶R²⁷;
R² is alkyl, fluoroalkyl, cyano, C₂-C₆alkenyl, C₂-C₆alkynyl, or heteroalkyl optionally substituted with =O;
each R²⁰, R²¹, R²², R²³, R²⁴, and R²⁵ is independently H or methyl;
each R²⁶ and R²⁷ is independently H, hydroxylalkyl, or alkyl; and
m, n, and o are independently integers of 0, 1, 2, 3, or 4;
or a pharmaceutically acceptable salt or prodrug thereof.

In some embodiments, the cancer is a hematological malignancy. In some embodiments, the cancer is myelodysplastic syndrome (MDS) or acute myeloid leukemia (AML). In certain embodiments, the cancer is resistant to azacitidine, decitabine, SGI-110, lenalidomide, TXA-127, or combinations thereof. In some embodiments, the cancer is resistant to azacitidine, decitabine, lenalidomide, TXA-127, or combinations thereof.

In some embodiments, the compound of formula (I) is 3-[2-Butyl-1-(2-diethylaminoethyl)-1H-benzimidazol-5-yl]-N-hydroxy-acrylamide. In some embodiments, the compound of formula (I) or (Id) has the structure:

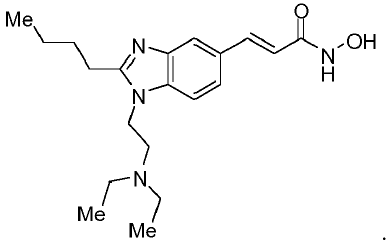

In some embodiments, the DNA hypomethylating agent is 5-azacytidine (azacitidine).

Also described herein in some embodiments are kits comprising one or more containers filled with a DNA hypomethylating agent and one or more containers filled with a compound of formula (I): 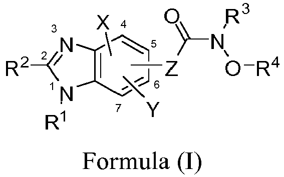 wherein
R¹ is an optionally substituted heteroaryl group, an optionally substituted heterocycloalkyl group or a group of formula:

   -(CR²⁰R²¹)ₘ-(CR²²R²³)ₙ-(CR²⁴R²⁵)ₒ-NR²⁶R²⁷;
R² is selected from the group consisting of: H, alkyl, alkenyl, alkynyl, heteroalkyl, cycloalkyl, cycloalkenyl, cycloalkylalkyl, alkoxyalkyl, R¹¹S(O)R¹³-, R¹¹S(O)₂R¹³-, R¹¹C(O)N(R¹²)R¹³-, R¹¹SO₂N(R¹²)R¹³-, R¹¹N(R¹²)C(O)R¹³-, R¹¹N(R¹²)SO₂R¹³-, R¹¹N(R¹²)C(O)N(R¹²)R¹³- and acyl, each of which may be optionally substituted;
R³ is selected from the group consisting of H, C₁ -C₆ alkyl, and acyl, each of which may be optionally substituted;
X and Y are the same or different and are independently selected from the group consisting of: H, halogen, -CN, -NO₂, -CF₃, -OCF₃, alkyl, alkenyl, alkynyl, haloalkyl, haloalkenyl, haloalkynyl, heteroalkyl, cycloalkyl, cycloalkenyl, heterocycloalkyl, heterocycloalkenyl, aryl, heteroaryl, hydroxy, hydroxyalkyl, alkoxy, alkoxyalkyl, alkoxyaryl, alkoxyheteroaryl, alkenyloxy, alkynyloxy, cycloalkyloxy, cycloalkenyloxy, heterocycloalkyloxy, heterocycloalkenyloxy, aryloxy, heteroaryloxy, arylalkyl, heteroarylalkyl, arylalkyloxy, -amino, alkylamino, acylamino, aminoalkyl, arylamino, sulfonyl, alkylsulfonyl, arylsulfonyl, aminosulfonyl, aminoalkyl, alkoxyalky, -COOH-C(O)OR⁵, -COR⁵,-SH, -SR⁶, -OR⁶ acyl and -NR⁷R⁸, each of which may be optionally substituted;
R⁴ is selected from the group consisting of: H, alkyl, alkenyl, alkynyl, haloalkyl, heteroalkyl, cycloalkyl, heterocycloalkyl, aryl, heteroaryl, cycloalkylalkyl, heterocycloalkylalkyl, arylalkyl, heteroarylalkyl and acyl, each of which may be optionally substituted;
each R⁵ is independently selected from the group consisting of: H, alkyl, alkenyl, alkynyl, haloalkyl, heteroalkyl, cycloalkyl, heterocycloalkyl, aryl, heteroaryl, cycloalkylalkyl, heterocycloalkylalkyl, arylalkyl, heteroarylalkyl and acyl, each of which may be optionally substituted;
each R⁶ is independently selected from the group consisting of: H, alkyl, alkenyl, alkynyl, haloalkyl, heteroalkyl, cycloalkyl, heterocycloalkyl, aryl, heteroaryl, cycloalkylalkyl, heterocycloalkylalkyl, arylalkyl, heteroarylalkyl and acyl; each of which may be optionally substituted;
each R⁷ and R⁸ is independently selected from the group consisting of: H, alkyl, alkenyl, alkynyl, haloalkyl, heteroalkyl, cycloalkyl, heterocycloalkyl, aryl, heteroaryl, cycloalkylalkyl, heterocycloalkylalkyl, arylalkyl, heteroarylalkyl and acyl, each of which may be optionally substituted;
each R¹¹ and R¹² is independently selected from the group consisting of H, alkyl, alkenyl, and alkynyl, each of which may be optionally substituted;
each R¹³ is a bond or is independently selected from the group consisting of: alkyl, alkenyl, and alkynyl, each of which may be optionally substituted;
each R²⁰, R²¹, R²², R²³, R²⁴ and R²⁵ is independently selected from the group consisting of: H, halogen, -CN, -NO₂, -CF₃, -OCF₃, alkyl, alkenyl, alkynyl, haloalkyl, haloalkenyl, haloalkynyl, heteroalkyl, cycloalkyl, cycloalkenyl, heterocycloalkyl, heterocycloalkenyl, aryl, heteroaryl, cycloalkylalkyl, heterocycloalkylalkyl, arylalkyl, heteroarylalkyl, arylalkenyl, cycloalkylheteroalkyl, heterocycloalkylheteroalkyl, heteroaryiheteroalkyl, arylheteroalkyl, hydroxy, hydroxyalkyl, alkoxy, alkoxyalkyl, alkoxyaryl, alkoxyheteroaryl, alkenyloxy, alkynyloxy, cycloalkylkoxy, heterocycloalkyloxy, aryloxy, arylalkyloxy, phenoxy, benzyloxy heteroaryloxy, amino, alkylamino, acylamino, aminoalkyl, arylamino, alkoxycarbonyl, alkylaminocarbonyl, sulfonyl, alkylsulfonyl, aminosulfonyl, arylsulfonyl, arylsulfinyl -COOH, -C(O)OR⁵,-COR⁵, -SH, -SR⁶, -OR⁶ and acyl, each of which may be optionally substituted; or
R²⁰ and R²¹ when taken together may form a group of formula =O or =S, and/or
R²² and R²³ when taken together may form a group of formula =O or =S, and/or
R²⁴ and R²⁵ when taken together may form a group of formula =O or =S;
each R²⁶ and R²⁷ is independently selected from the group consisting of: H, halogen, alkyl, alkenyl, alkynyl, haloalkyl, haloalkenyl, heteroalkyl, cycloalkyl, cycloalkenyl, heterocycloalkyl, heterocycloalkenyl, aryl, heteroaryl, cycloalkylalkyl, heterocycloalkylalkyl, arylalkyl, heteroarylalkyl, arylalkenyl, cycloalkylheteroalkyl, heterocycloalkylheteroalkyl, heteroarylheteroalkyl, arylheteroalkyl, hydroxy, hydroxyalkyl, alkoxy, alkoxyalkyl, alkoxyaryl, alkenyloxy, alkynyloxy, cycloalkylkoxy, heterocycloalkyloxy, aryloxy, arylalkyloxy, heteroaryloxy, amino, alkylamino, aminoalkyl, acylamino, arylamino, phenoxy, benzyloxy, COOH, alkoxycarbonyl, alkylaminocarbonyl, sulfonyl, alkylsulfonyl, alkylsulfinyl, arylsulfonyl, arylsulfinyl, aminosulfonyl, SW and acyl, each of which may be optionally substituted, or R²⁶ and R²⁷ when taken together with the nitrogen atom to which they are attached form an optionally substituted heterocycloalkyl group;
Z is selected from the group consisting of -CH₂-, -CH₂CH₂-, -CH=CH-, C₃-C₆ alkylene, C₃-C₆ alkenylene, C₃-C₆ alkynylene, C₃-C₆ cycloalkyl, unsubstituted or substituted with one or more substituents independently selected from the group consisting of C₁-C₄ alkyl;
m, n and o are integers independently selected from the group consisting of 0, 1, 2, 3 and 4;
or a pharmaceutically acceptable salt or prodrug thereof.

In some embodiments, the compound of formula (I) has the structure of formula (Id): 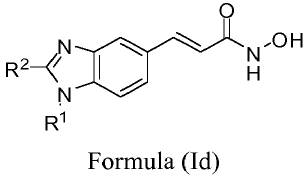 wherein
R¹ is a group having the formula:

   -(CR²⁰R²¹)*ₘ*-(CR²²R²¹)*ₙ*-(CR²⁴R²⁵)*ₒ*-NR²⁶R²⁷;
R² is alkyl, fluoroalkyl, cyano, C₂-C₆alkenyl, C₂-C₆alkynyl, or heteroalkyl optionally substituted with =O;
each R²⁰, R²¹, R²², R²³, R²⁴, and R²⁵ is independently H or methyl;
each R²⁶ and R²⁷ is independently H, hydroxylalkyl, or alkyl; and
m, n, and o are independently integers of 0, 1, 2, 3, or 4;
or a pharmaceutically acceptable salt or prodrug thereof.

In some embodiments, the kit comprises 5-azacytidine (azacitidine) as the DNA hypomethylating agent and the compound of formula (I) having the structure:

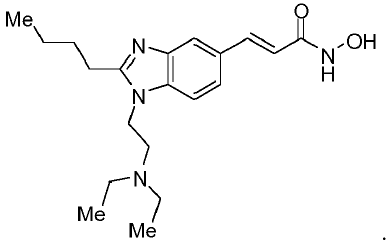

### INCORPORATION BY REFERENCE

All publications, patents, and patent applications mentioned in this specification are herein incorporated by reference to the same extent as if each individual publication, patent, or patent application was specifically and individually indicated to be incorporated by reference.

### DETAILED DESCRIPTION OF THE INVENTION

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that methods and structures within the scope of these claims and their equivalents be covered thereby.

There is a continuing need to develop and provide effective therapies for the treatment of disease and disorders associated with dysregulation of histone deacetylase (e.g., cancer). Described herein in some embodiments is a combination therapy for treating cancer.

### Certain Definitions

Unless otherwise noted, terminology used herein should be given its normal meaning as understood by one of skill in the art.

As used herein, the term unsubstituted means that there is no substituent or that the only substituents are hydrogen.

The term "optionally substituted" as used throughout the specification denotes that the group may or may not be further substituted or fused (so as to form a condensed polycyclic system), with one or more substituent groups. Preferably the substituent groups are one or more groups independently selected from the group consisting of halogen, =O, =S, -CN, - NO₂, -CF₃,-OCF₃, alkyl, alkenyl, alkynyl, haloalkyl, haloalkenyl, haloalkynyl, heteroalkyl, cycloalkyl, cycloalkenyl, heterocycloalkyl, heterocycloalkenyl, aryl, heteroaryl, cycloalkylalkyl, heterocycloalkylalkyl, heteroarylalkyl, arylalkyl, cycloalkylalkenyl, heterocycloalkylalkenyl, arylalkenyl, heteroarylalkenyl, cycloalkylheteroalkyl, heterocycloalkylheteroalkyl, arylheteroalkyl, heteroarylheteroalkyl, hydroxy, hydroxyalkyl, alkoxy, alkoxyalkyl, alkoxycycloalkyl, alkoxyheterocycloalkyl, alkoxyaryl, alkoxyheteroaryl, alkoxycarbonyl, alkylaminocarbonyl, alkenyloxy, alkynyloxy, cycloalkyloxy, cycloalkenyloxy, heterocycloalkyloxy, heterocycloalkenyloxy, aryloxy, phenoxy, benzyloxy, heteroaryloxy, arylalkyloxy, arylalkyl, heteroarylalkyl, cycloalkylalkyl, heterocycloalkylalkyl, arylalkyloxy, amino, alkylamino, acylamino, aminoalkyl, arylamino, sulfonylamino, sulfinylamino, sulfonyl, alkylsulfonyl, arylsulfonyl, aminosulfonyl, sulfinyl, alkylsulfinyl, arylsulfinyl, aminosulfinylaminoalkyl, -COOH, -COR⁵, -C(O)OR⁵, CONHR⁵, NHCOR⁵, NHCOOR⁵, NHCONHR⁵, C(=NOH)R⁵, -SH, -SR⁵, -OR⁵ and acyl.

"Alkyl" as a group or part of a group refers to a straight or branched aliphatic hydrocarbon group, preferably a C₁-C₁₄ alkyl, more preferably C₁-C₁₀ alkyl, preferably C₁-C₆ or C₁-C₃ unless otherwise noted. Examples of suitable straight and branched C₁-C₆ alkyl substituents include methyl, ethyl, n-propyl, 2-propyl, n-butyl, sec-butyl, t-butyl, hexyl, and the like. The group may be a terminal group or a bridging group.

"Alkylamino" includes both monoalkylamino and dialkylamino, unless specified. "Monoalkylamino" means a -NH-Alkyl group, in which alkyl is as defined above. "Dialkylamino" means a -N(alkyl)₂ group, in which each alkyl may be the same or different and are each as defined herein for alkyl. The alkyl group is preferably a C₁-C₆ alkyl group. The group may be a terminal group or a bridging group.

"Arylamino" includes both mono-arylamino and di-arylamino unless specified. Mono-arylamino means a group of formula aryl NH-, in which aryl is as defined herein. di-arylamino means a group of formula (aryl₂) N- where each aryl may be the same or different and are each as defined herein for aryl. The group may be a terminal group or a bridging group.

"Acyl" means an alkyl-CO- group in which the alkyl group is as described herein. Examples of acyl include acetyl and benzoyl. The alkyl group is preferably a C₁-C₆ alkyl group. The group may be a terminal group or a bridging group.

"Alkenyl" as a group or part of a group denotes an aliphatic hydrocarbon group containing at least one carbon-carbon double bond and which may be straight or branched preferably having 2-14 carbon atoms, more preferably 2-12 carbon atoms, most preferably 2-6 carbon atoms, in the normal chain. The group may contain a plurality of double bonds in the normal chain and the orientation about each is independently E or Z. Exemplary alkenyl groups include, but are not limited to, ethenyl, propenyl, butenyl, pentenyl, hexenyl, heptenyl, octenyl and nonenyl. The group may be a terminal group or a bridging group.

"Alkoxy" refers to an -O-alkyl group in which alkyl is defined herein. Preferably the alkoxy is a C₁-C₆alkoxy. Examples include, but are not limited to, methoxy and ethoxy. The group may be a terminal group or a bridging group.

"Alkenyloxy" refers to an -O- alkenyl group in which alkenyl is as defined herein. Preferred alkenyloxy groups are C₁-C₆ alkenyloxy groups. The group may be a terminal group or a bridging group.

"Alkynyloxy" refers to an -O-alkynyl group in which alkynyl is as defined herein. Preferred alkynyloxy groups are C₁-C₆ alkynyloxy groups. The group may be a terminal group or a bridging group.

"Alkoxycarbonyl" refers to an -C(O)-O-alkyl group in which alkyl is as defined herein. The alkyl group is preferably a C₁-C₆ alkyl group. Examples include, but not limited to, methoxycarbonyl and ethoxycarbonyl. The group may be a terminal group or a bridging group.

"Akylsulfinyl" means a -S(O)-alkyl group in which alkyl is as defined above. The alkyl group is preferably a C₁-C₆ alkyl group. Exemplary alkylsulfinyl groups include, but not limited to, methylsulfinyl and ethylsulfinyl. The group may be a terminal group or a bridging group.

"Alkylsulfonyl" refers to a -S(O)₂-alkyl group in which alkyl is as defined above. The alkyl group is preferably a C₁-C₆ alkyl group. Examples include, but not limited to methylsulfonyl and ethylsulfonyl. The group may be a terminal group or a bridging group.

"Alkynyl as a group or part of a group means an aliphatic hydrocarbon group containing a carbon-carbon triple bond and which may be straight or branched preferably having from 2-14 carbon atoms, more preferably 2-12 carbon atoms, more preferably 2-6 carbon atoms in the normal chain. Exemplary structures include, but are not limited to, ethynyl and propynyl. The group may be a terminal group or a bridging group.

"Alkylaminocarbonyl" refers to an alkylamino-carbonyl group in which alkylamino is as defined above. The group may be a terminal group or a bridging group.

"Cycloalkyl" refers to a saturated or partially saturated, monocyclic or fused or spiro polycyclic, carbocycle preferably containing from 3 to 9 carbons per ring, such as cyclopropyl, cyclobutyl, cyclopentyl, cyclohexyl and the like, unless otherwise specified. It includes monocyclic systems such as cyclopropyl and cyclohexyl, bicyclic systems such as decalin, and polycyclic systems such as adamantane. The group may be a terminal group or a bridging group.

"Cycloalkenyl" means a non-aromatic monocyclic or multicyclic ring system containing at least one carbon-carbon double bond and preferably having from 5-10 carbon atoms per ring. Exemplary monocyclic cycloalkenyl rings include cycloheptenyl, cyclohexenyl or cycloheptenyl. The cycloalkenyl group may be substituted by one or more substituent groups. The group may be a terminal group or a bridging group.

The above discussion of alkyl and cycloalkyl substituents also applies to the alkyl portions of other substituents, such as without limitation, alkoxy, alkyl amines, alkyl ketones, arylalkyl, heteroarylalkyl, alkylsulfonyl and alkyl ester substituents and the like.

"Cycloalkylalkyl" means a cycloalkyl-alkyl- group in which the cycloalkyl and alkyl moieties are as previously described. Exemplary monocycloalkylalkyl groups include cyclopropylmethyl, cyclopentylmethyl, cyclohexylmethyl and cycloheptylmethyl. The group may be a terminal group or a bridging group.

"Halogen" represents chlorine, fluorine, bromine or iodine.

"Heterocycloalkyl" refers to a saturated or partially saturated monocyclic, bicyclic, or polycyclic ring containing at least one heteroatom selected from nitrogen, sulfur, oxygen, preferably from 1 to 3 heteroatoms in at least one ring. Each ring is preferably from 3 to 10 membered, more preferably 4 to 7 membered. Examples of suitable heterocycloalkyl substituents include pyrrolidyl, tetrahydrofuryl, tetrahydrothiofuranyl, piperidyl, piperazyl, tetrahydropyranyl, morphilino, 1,3-diazapane, 1,4-diazapane, 1,4-oxazepane, and 1,4-oxathiapane. The group may be a terminal group or a bridging group.

"Heterocycloalkenyl" refers to a heterocycloalkyl as described above but containing at least one double bond. The group may be a terminal group or a bridging group.

"Heterocycloalkylalkyl" refers to a heterocycloalkyl-alkyl group in which the heterocycloalkyl and alkyl moieties are as previously described. Exemplary heterocycloalkylalkyl groups include (2-tetrahydrofuryl)methyl, (2-tetrahydrothiofuranyl)methyl. The group may be a terminal group or a bridging group.

"Heteroalkyl" refers to a straight- or branched-chain alkyl group preferably having from 2 to 14 atoms, more preferably 2 to 10 atoms in the chain, one or more of which is a heteroatom selected from S, O, and N. Exemplary heteroalkyls include alkyl ethers, secondary and tertiary alkyl amines, alkyl sulfides, and the like. The group may be a terminal group or a bridging group.

"Aryl" as a group or part of a group denotes (i) an optionally substituted monocyclic, or fused polycyclic, aromatic carbocycle (ring structure having ring atoms that are all carbon) preferably having from 5 to 12 atoms per ring. Examples of aryl groups include phenyl, naphthyl, and the like; (ii) an optionally substituted partially saturated bicyclic aromatic carbocyclic moiety in which a phenyl and a C₅₋₇ cycloalkyl or C₅₋₇ cycloalkenyl group are fused together to form a cyclic structure, such as tetrahydronaphthyl, indenyl or indanyl. The group may be a terminal group or a bridging group.

"Arylalkenyl" means an aryl-alkenyl- group in which the aryl and alkenyl are as previously described. Exemplary arylalkenyl groups include phenylallyl. The group may be a terminal group or a bridging group.

"Arylalkyl" means an aryl-alkyl- group in which the aryl and alkyl moieties are as previously described. Preferred arylalkyl groups contain a C₁₋₅ alkyl moiety. Exemplary arylalkyl groups include benzyl, phenethyl and naphthelenemethyl. The group may be a terminal group or a bridging group.

"Arylacyl" means an aryl-acyl- group in which the aryl and acyl moieties are as previously described. In general the aryl moiety is attached to the alkyl portion of the acyl moiety, typically to the terminal carbon of the alkyl portion of the acyl moiety. Preferred arylacyl groups contain a C₁-C₅ alkyl moiety in the acyl moiety. Exemplary arylacyl groups include 2-phenyl-acetyl. The group may be a terminal group or a bridging group.

"Heteroaryl" either alone or part of a group refers to groups containing an aromatic ring (preferably a 5 or 6 membered aromatic ring) having one or more heteroatoms as ring atoms in the aromatic ring with the remainder of the ring atoms being carbon atoms. Suitable heteroatoms include nitrogen, oxygen and sulphur. Examples of heteroaryl include thiophene, benzothiophene, benzofuran, benzimidazole, benzoxazole, benzothiazole, benzisothiazole, naphtho[2,3-b]thiophene, furan, isoindolizine, xantholene, phenoxatine, pyrrole, imidazole, pyrazole, pyridine, pyrazine, pyrimidine, pyridazine, indole, isoindole, 1H-indazole, purine, quinoline, isoquinoline, phthalazine, naphthyridine, quinoxaline, cinnoline, carbazole, phenanthridine, acridine, phenazine, thiazole, isothiazole, phenothiazine, oxazole, isooxazole, furazane, phenoxazine, 2-,3- or4-pyridyl, 2-, 3-, 4-, 5-, or 8-quinolyl, 1-, 3-, 4-, or 5-isoquinolinyl, 1-, 2-, or 3-indolyl, and 2-, or 3-thienyl. The group may be a terminal group or a bridging group.

"Heteroarylalkyl" means a heteroaryl-alkyl group in which the heteroaryl and alkyl moieties are as previously described. Preferred heteroarylalkyl groups contain a lower alkyl moiety. Exemplary heteroarylalkyl groups include pyridylmethyl. The group may be a terminal group or a bridging group.

"Lower alkyl" as a group means unless otherwise specified, an aliphatic hydrocarbon group which may be straight or branched having 1 to 6 carbon atoms in the chain, more preferably 1 to 4 carbons such as methyl, ethyl, propyl (n-propyl or isopropyl) or butyl (n-butyl, isobutyl or tertiary-butyl). The group may be a terminal group or a bridging group.

In Formula (I), as well as in Formulae (1a) - (If) defining sub-sets of compounds within Formula (I), there is shown a benzimidazole ring system. Within this ring system, there are substitutable positions at the 4-, 5-, 6-, and 7-ring positions. In each of Formulae (I), (Ia), and (Ib), there is a requirement for attachment of an acidic moiety at one of the ring positions. This acidic moiety may be provided by but is not limited to groups containing, a hydroxamic acid or salt derivatives of such acid which when hydrolysed would provide the acidic moiety. In some embodiments the acidic moiety may be attached to the ring position through an alkylene group such as -CH₂- or -CH₂-CH₂-, or an alkenylene group such as -CH=CH-. Preferred positions for attachment of the acidic moiety are the 5- and 6-ring positions.

It is understood that included in the family of compounds of Formula (I) are isomeric forms including diastereoisomers, enantiomers, tautomers, and geometrical isomers in "E" or "Z" configurational isomer or a mixture of E and Z isomers. It is also understood that some isomeric forms such as diastereomers, enantiomers, and geometrical isomers can be separated by physical and/or chemical methods and by those skilled in the art.

Some of the compounds of the disclosed embodiments may exist as single stereoisomers, racemates, and/or mixtures of enantiomers and for diastereomers. All such single stereoisomers, racemates and mixtures thereof are intended to be within the scope of the subject matter described and claimed.

Additionally, Formula (I) is intended to cover, where applicable, solvated as well as unsolvated forms of the compounds. Thus, each formula includes compounds having the indicated structure, including the hydrated as well as the non-hydrated forms.

In addition to compounds of the Formula (I), the HDAC inhibiting agents of the various embodiments include pharmaceutically acceptable salts, prodrugs, and active metabolites of such compounds, and pharmaceutically acceptable salts of such metabolites.

The term "pharmaceutically acceptable salts" refers to salts that retain the desired biological activity of the above-identified compounds, and include pharmaceutically acceptable acid addition salts and base addition salts. Suitable pharmaceutically acceptable acid addition salts of compounds of Formula (I) may be prepared from an inorganic acid or from an organic acid. Examples of such inorganic acids are hydrochloric, sulfuric, and phosphoric acid. Appropriate organic acids may be selected from aliphatic, cycloaliphatic, aromatic, heterocyclic carboxylic and sulfonic classes of organic acids, examples of which are formic, acetic, propionic, succinic, glycolic, gluconic, lactic, malic, tartaric, citric, fumaric, maleic, alkyl sulfonic, arylsulfonic. Suitable pharmaceutically acceptable base addition salts of compounds of Formula (I) include metallic salts made from lithium, sodium, potassium, magnesium, calcium, aluminium, and zinc, and organic salts made from organic bases such as choline, diethanolamine, morpholine. Other examples of organic salts are: ammonium salts, quaternary salts such as tetramethylammonium salt; amino acid addition salts such as salts with glycine and arginine. Additional information on pharmaceutically acceptable salts can be found in Remington's Pharmaceutical Sciences, 19th Edition, Mack Publishing Co., Easton, PA 1995. In the case of agents that are solids, it is understood by those skilled in the art that the inventive compounds, agents and salts may exist in different crystalline or polymorphic forms, all of which are intended to be within the scope of the present invention and specified formulae.

"Prodrug" means a compound which is convertible *in vivo* by metabolic means (e.g. by hydrolysis, reduction or oxidation) to a compound of formula (I). For example an ester prodrug of a compound of formula (I) containing a hydroxyl group may be convertible by hydrolysis *in vivo* to the parent molecule. Suitable esters of compounds of formula (I) containing a hydroxyl group, are for example acetates, citrates, lactates, tartrates, malonates, oxalates, salicylates, propionates, succinates, fumarates, maleates, methylene-bis-β-hydroxynaphthoates, gestisates, isethionates, di-*p*-toluoyltartrates, methanesulphonates, ethanesulphonates, benzenesulphonates, *p*-toluenesulphonates, cyclohexylsuiphamates and quinates. As another example an ester prodrug of a compound of formula (I) containing a carboxy group may be convertible by hydrolysis in vivo to the parent molecule. (Examples of ester prodrugs are those described by F. J. Leinweber, Drug Metab. Res., 18:379, 1987).

The term "therapeutically effective amount" or "effective amount" is an amount sufficient to effect beneficial or desired results. An effective amount can be administered in one or more administrations. An effective amount is typically sufficient to palliate, ameliorate, stabilize, reverse, slow or delay the progression of the disease state. A therapeutically effective amount can be readily determined by an attending diagnostician by the use of conventional techniques and by observing results obtained under analogous circumstances. In determining the therapeutically effective amount a number of factors are to be considered including but not limited to, the species of animal, its size, age and general health, the specific condition involved, the severity of the condition, the response of the patient to treatment, the particular compound administered, the mode of administration, the bioavailability of the preparation administered, the dose regime selected, the use of other medications and other relevant circumstances.

### HDAC Inhibiting Agents

In one aspect the present invention provides a compound of the formula (I): 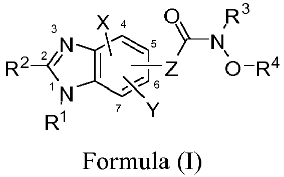 wherein R
¹ is an optionally substituted heteroaryl group, an optionally substituted heterocycloalkyl group or a group of formula:

   -(CR²⁰R²¹)ₘ-(CR²²R²³)ₙ-(CR²⁴R²⁵)ₒ-NR²⁶R²⁷;
R² is selected from the group consisting of: H, alkyl, alkenyl, alkynyl, heteroalkyl, cycloalkyl, cycloalkenyl, cycloalkylalkyl, alkoxyalkyl, R¹¹S(O)R¹³-, R¹¹S(O)₂R¹³-, R¹¹C(O)N(R¹²)R¹³-, R¹¹SO₂N(R¹²)R¹³-, R¹¹N(R¹²)C(O)R¹³-, R¹¹N(R¹²)SO₂R¹³-, R¹¹N(R¹²)C(O)N(R¹²)R¹³- and acyl, each of which may be optionally substituted;
R³ is selected from the group consisting of H, C₁ -C₆ alkyl, and acyl, each of which may be optionally substituted;
X and Y are the same or different and are independently selected from the group consisting of: H, halogen, -CN, -NO₂, -CF₃, -OCF₃, alkyl, alkenyl, alkynyl, haloalkyl, haloalkenyl, haloalkynyl, heteroalkyl, cycloalkyl, cycloalkenyl, heterocycloalkyl, heterocycloalkenyl, aryl, heteroaryl, hydroxy, hydroxyalkyl, alkoxy, alkoxyalkyl, alkoxyaryl, alkoxyheteroaryl, alkenyloxy, alkynyloxy, cycloalkyloxy, cycloalkenyloxy, heterocycloalkyloxy, heterocycloalkenyloxy, aryloxy, heteroaryloxy, arylalkyl, heteroarylalkyl, arylalkyloxy, -amino, alkylamino, acylamino, aminoalkyl, arylamino, sulfonyl, alkylsulfonyl, arylsulfonyl, aminosulfonyl, aminoalkyl, alkoxyalky, -COOH-C(O)OR⁵, -COR⁵,-SH, -SR⁶, -OR⁶ acyl and -NR⁷R⁸, each of which may be optionally substituted;
R⁴ is selected from the group consisting of: H, alkyl, alkenyl, alkynyl, haloalkyl, heteroalkyl, cycloalkyl, heterocycloalkyl, aryl, heteroaryl, cycloalkylalkyl, heterocycloalkylalkyl, arylalkyl, heteroarylalkyl and acyl, each of which may be optionally substituted;
each R⁵ is independently selected from the group consisting of: H, alkyl, alkenyl, alkynyl, haloalkyl, heteroalkyl, cycloalkyl, heterocycloalkyl, aryl, heteroaryl, cycloalkylalkyl, heterocycloalkylalkyl, arylalkyl, heteroarylalkyl and acyl, each of which may be optionally substituted;
each R⁶ is independently selected from the group consisting of: H, alkyl, alkenyl, alkynyl, haloalkyl, heteroalkyl, cycloalkyl, heterocycloalkyl, aryl, heteroaryl, cycloalkylalkyl, heterocycloalkylalkyl, arylalkyl, heteroarylalkyl and acyl; each of which may be optionally substituted;
each R⁷ and R⁸ is independently selected from the group consisting of: H, alkyl, alkenyl, alkynyl, haloalkyl, heteroalkyl, cycloalkyl, heterocycloalkyl, aryl, heteroaryl, cycloalkylalkyl, heterocycloalkylalkyl, arylalkyl, heteroarylalkyl and acyl, each of which may be optionally substituted;
each R¹¹ and R¹² is independently selected from the group consisting of H, alkyl, alkenyl, and alkynyl, each of which may be optionally substituted;
each R¹³ is a bond or is independently selected from the group consisting of: alkyl, alkenyl, and alkynyl, each of which may be optionally substituted;
each R²⁰, R²¹, R²², R²³, R²⁴ and R²⁵ is independently selected from the group consisting of: H, halogen, -CN, -NO₂, -CF₃, -OCF₃, alkyl, alkenyl, alkynyl, haloalkyl, haloalkenyl, haloalkynyl, heteroalkyl, cycloalkyl, cycloalkenyl, heterocycloalkyl, heterocycloalkenyl, aryl, heteroaryl, cycloalkylalkyl, heterocycloalkylalkyl, arylalkyl, heteroarylalkyl, arylalkenyl, cycloalkylheteroalkyl, heterocycloalkylheteroalkyl, heteroaryiheteroalkyl, arylheteroalkyl, hydroxy, hydroxyalkyl, alkoxy, alkoxyalkyl, alkoxyaryl, alkoxyheteroaryl, alkenyloxy, alkynyloxy, cycloalkylkoxy, heterocycloalkyloxy, aryloxy, arylalkyloxy, phenoxy, benzyloxy heteroaryloxy, amino, alkylamino, acylamino, aminoalkyl, arylamino, alkoxycarbonyl, alkylaminocarbonyl, sulfonyl, alkylsulfonyl, aminosulfonyl, arylsulfonyl, arylsulfinyl -COOH, -C(O)OR⁵,-COR⁵, -SH, -SR⁶, -OR⁶ and acyl, each of which may be optionally substituted; or
R²⁰ and R²¹ when taken together may form a group of formula =O or =S, and/or
R²² and R²³ when taken together may form a group of formula =O or =S, and/or
R²⁴ and R²⁵ when taken together may form a group of formula =O or =S;
each R²⁶ and R²⁷ is independently selected from the group consisting of: H, halogen, alkyl, alkenyl, alkynyl, haloalkyl, haloalkenyl, heteroalkyl, cycloalkyl, cycloalkenyl, heterocycloalkyl, heterocycloalkenyl, aryl, heteroaryl, cycloalkylalkyl, heterocycloalkylalkyl, arylalkyl, heteroarylalkyl, arylalkenyl, cycloalkylheteroalkyl, heterocycloalkylheteroalkyl, heteroarylheteroalkyl, arylheteroalkyl, hydroxy, hydroxyalkyl, alkoxy, alkoxyalkyl, alkoxyaryl, alkenyloxy, alkynyloxy, cycloalkylkoxy, heterocycloalkyloxy, aryloxy, arylalkyloxy, heteroaryloxy, amino, alkylamino, aminoalkyl, acylamino, arylamino, phenoxy, benzyloxy, COOH, alkoxycarbonyl, alkylaminocarbonyl, sulfonyl, alkylsulfonyl, alkylsulfinyl, arylsulfonyl, arylsulfinyl, aminosulfonyl, SW and acyl, each of which may be optionally substituted, or R²⁶ and R²⁷ when taken together with the nitrogen atom to which they are attached form an optionally substituted heterocycloalkyl group;
Z is selected from the group consisting of -CH₂-, -CH₂CH₂-, -CH=CH-, C₃-C₆ alkylene, C₃-C₆ alkenylene, C₃-C₆ alkynylene, C₃-C₆ cycloalkyl, unsubstituted or substituted with one or more substituents independently selected from the group consisting of C₁-C₄ alkyl;
m, n and o are integers independently selected from the group consisting of 0, 1, 2, 3 and 4;
or a pharmaceutically acceptable salt or prodrug thereof.

In one embodiment of the invention R⁴ is H and the compounds are those of formula (Ia): 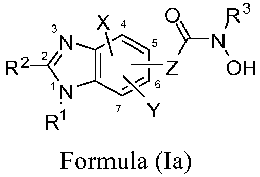
or a pharmaceutically acceptable salt or prodrug thereof
wherein R¹, R², R³, X, Y and Z are as defined for compounds of formula (I).

In another embodiment R³ and R⁴ are H and the compounds are of formula (Ib). 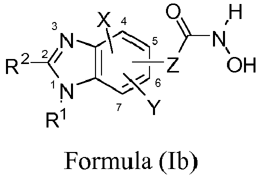
or a pharmaceutically acceptable salt or prodrug thereof
wherein R¹, R², X, Y and Z are as defined for compounds of formula (I).

As with any group of structurally related compounds which possess a particular utility, certain groups are preferred for the compounds of the Formula (I), (Ia) and (Ib) in their end use application.

In one embodiment the group R¹ is a group of formula

-(CR²⁰R²¹)ₘ-(CR²²R²³)ₙ-(CR²⁴R²⁵)ₒ-NR²⁶R²⁷

in which m, n and o are integers independently selected from the group consisting of 0, 1, 2, 3 and 4.

Accordingly, in one embodiment the compounds of the invention are compounds of formula (Ic): 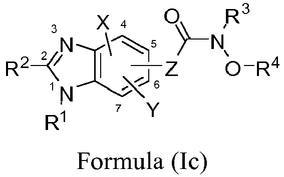 wherein R¹ is a group of formula:

-(CR²⁰R²¹)ₘ-(CR²²R²³)ₙ(CR²⁴R²⁵)_{O-}NR²⁶R²⁷

and R², R³, R⁴, X, Y, Z, R²⁰, R²¹, R²², R²³, R²⁴, R²⁵, R²⁶, R²⁷, m, n and o are as defined for compounds of formula (I).

As the values of m, n and o are integers ranging from 0 to 4 the sum of m+n+o is an integer selected from the group consisting of 0, 1, 2, 3, 4, 5, 6, 7, 8, 9, 10, 11, and 12. In one embodiment the sum of m+n+o is an integer selected from the group consisting of 0, 1, 2, 3, 4, 5, 6, 7 and 8. In another embodiment the sum of m+n+o is an integer selected from the group consisting of 0, 1, 2, 3 and 4. In another embodiment the sum of m+n+o is an integer selected from the group consisting of 2 and 3.

In specific embodiments, the sum of m+n+o is 2. When this occurs R¹ is selected from the group consisting of:

-(CR²⁰R²¹)₂₋NR²⁶R²⁷;

-(CR²²R²³)₂₋NR²⁶R²⁷;

-(CR²⁴R²⁵)₂₋NR²⁶R²⁷;

-(CR²⁰R²¹)-(CR²²R²³)-NR²⁶R²⁷;

-(CR²⁰R²¹)-(CR²⁴R²⁵)-NR²⁶R²⁷;

-(CR²²R²³)-(CR²⁴R²⁵)-NR²⁶R²⁷;

In some embodiments, R¹ is the group:

-(CR²⁰R²¹)-(CR²²R²³)-NR²⁶R²⁷;

In some embodiments, the compound of formula (I) has the structure of formula (Id): 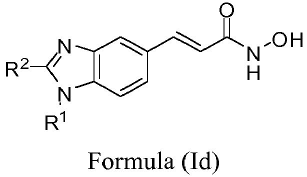 wherein
R¹ is a group having the formula:

   -(CR²⁰R²¹)*ₘ*-(CR²²R²³)*ₙ*-(CR²⁴R²⁵)*ₒ*-NR²⁶R²⁷;
R² is alkyl, fluoroalkyl, cyano, C₂-C₆alkenyl, C₂-C₆alkynyl, or heteroalkyl optionally substituted with =O;
each R²⁰, R²¹, R²², R²³, R²⁴, and R²⁵ is independently H or methyl;
each R²⁶ and R²⁷ is independently H, hydroxylalkyl, or alkyl; and
m, n, and o are independently integers of 0, 1, 2, 3, or 4;
or a pharmaceutically acceptable salt or prodrug thereof.

In some embodiments, the compound of formula (I) has the structure of formula (Ie) or (If): 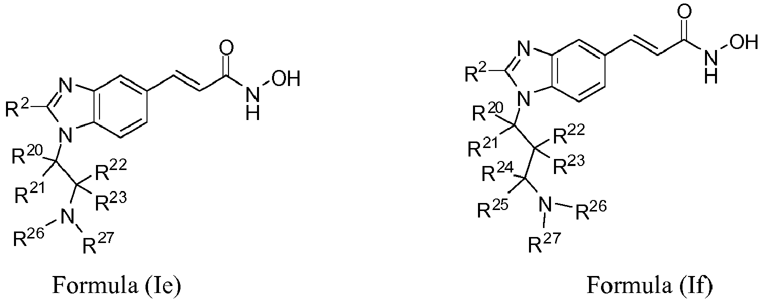 wherein
R² is alkyl, fluoroalkyl, cyano, C₂-C₆alkenyl, C₂-C₆alkynyl, or heteroalkyl optionally substituted with =O;
each R²⁰, R²¹, R²², R²³, R²⁴, and R²⁵ is independently H or methyl; and
each R²⁶ and R²⁷ is independently H, hydroxylalkyl, or alkyl;
or a pharmaceutically acceptable salt or prodrug thereof.

In some embodiments, the compound of formula (I) has the structure of the formula (II): 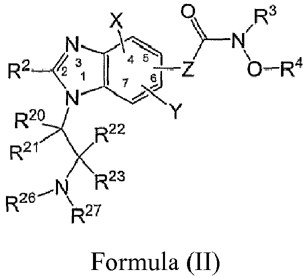 wherein X, Y, Z, R², R³, R⁴, R²⁰, R²¹, R²², R²³, R²⁶ and a R²⁷ are as defined in formula (I).

In a specific form of this embodiment R⁴ is H which provides compounds of formula (IIa): 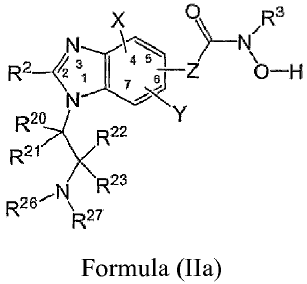 wherein X, Y, Z, R², R³, R²⁰, R²¹, R²², R²³, R²⁶ and R²⁷ are as defined in formula (I).

In another specific form R³ is H leading to compounds of formula (IIb): 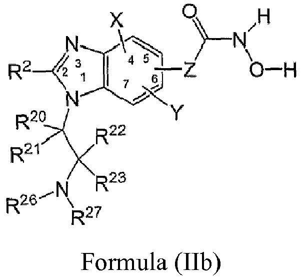 wherein X, Y, Z, R², R²³, R²¹, R²², R²³, R²⁶ and R²⁷ are as defined in formula (I).

In an even more specific form of this embodiment R²⁰, R²¹, R²² and R²³ are H providing compounds of formula (IIc): 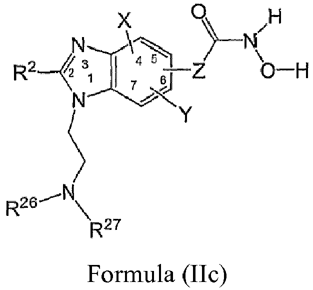 wherein X, Y, Z, R², R²⁶ and R²⁷ are as defined in formula (I).

In another embodiment the sum of m+n+o is 3. When this occurs R¹ is selected from the group consisting of:

-(CR²⁰R²¹)₃₋NR²⁶R²⁷;

-(CR²²R²³)₃₋NR²⁶R²⁷;

-(CR²⁴R²⁵)₃₋NR²⁶R²⁷;

-(CR²⁰R²¹)₂₋(CR²²R²³)-NR²⁶R²⁷;

-(CR²⁰R²¹)₂₋(CR²⁴R²⁵)NR²⁶R²⁷;

-(CR²⁰R²¹)-(CR²²R²³)₂₋NR²⁶R²⁷;

-(CR²²R²³)₂₋(CR²⁴R²⁵)-NR²⁶R²⁷;

-(CR²⁰R²¹)-(CR²⁴R²⁵)₂₋NR²⁶R²⁷;

-(CR²²R²³)-(CR²⁴R²⁵)₂₋NR²⁶R²⁷;

-(CR²⁰R²¹)-(CR²²R²³)-(CR²⁴R²⁵)-NR²⁶R²⁷;

In some embodiments, R¹ is a group of the formula:

-(CR²⁰R²¹)-(CR²²R²³)-(CR²⁴R²⁵)-NR²⁶R²⁷.

This provides compounds of the formula (III): 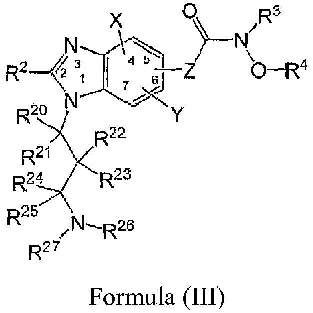 wherein X, Y, Z, R², R³, R⁴, R²⁰, R²¹, R²², R²³, R²⁴, R²⁵, R²⁶ and R²⁷ are as defined in formula (I).

In a specific form of this embodiment R⁴ is H which provides compounds of formula (IIIa). 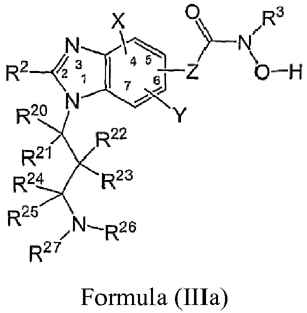 wherein X, Y, Z, R², R³, R²⁰, R²¹, R²², R²¹, R²⁴, R²⁵, R²⁶ and R²⁷ are as defined in formula (I).

In another specific form R³ is H leading to compounds of formula (IIIb): 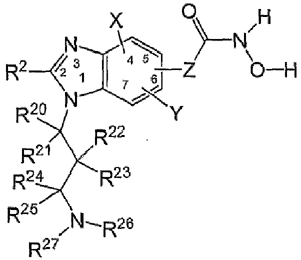 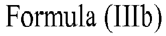 wherein X, Y, Z, R², R²⁰, R²¹, R²², R²³, R²⁴, R²⁵, R²⁶ and R²⁷ are as defined in formula (I).

In an even more specific form of this embodiment R²⁰, R²¹, R²⁴ and R²⁵ are H, and R²² and R²³ are methyl providing compounds of formula (IIIc). 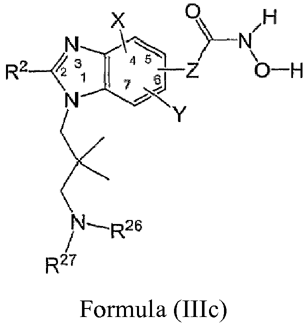 wherein X, Y, Z, R², R²⁶ and R²⁷ are as defined in formula (I).

In each of the above embodiments of the invention R²⁰ and R²¹ may represent a number of different variables. In one embodiment R²⁰ and R²¹ are independently selected from the group consisting of H, alkyl, alkenyl and alkynyl. In another embodiment R²⁰ and R²¹ are independently selected from the group consisting of H and alkyl. In yet another embodiment R²⁰ and R²¹ are independently selected from the group consisting of H, methyl, ethyl, isopropyl, propyl, 2-ethyl-propyl, 3,3-dimethyl-propyl, butyl, isobutyl, 3,3- dimethyl-butyl, 2-ethyl-butyl, pentyl, 2-methyl, pentyl, pent-4-enyl, hexyl, heptyl and octyl. In a specific embodiment R²⁰ and R²¹ are both H.

In each of the above embodiments of the invention R²² and R²³ may represent a number of different variables. In one embodiment R²² and R²³ are independently selected from the group consisting of H, alkyl, alkenyl and alkynyl. In another embodiment R²² and R²³ are independently selected from the group consisting of H and alkyl. In yet another embodiment R²² and R²³ are independently selected from the group consisting of H, methyl, ethyl, isopropyl, propyl, 2-ethyl-propyl, 3,3-dimethyl-propyl, butyl, isobutyl, 3,3- dimethyl-butyl, 2-ethyl-butyl, pentyl, 2-methyl, pentyl, pent-4-enyl, hexyl, heptyl and octyl. In a further embodiment R²² and R²³ are independently selected from the group consisting of alkyl. In a most specific embodiment R²² and R²³ are both methyl.

In each of the above embodiments of the invention R²⁴ and R²⁵ may represent a number of different variables. In one embodiment R²⁴ and R²⁵ are preferably independently selected from the group consisting of H, alkyl, alkenyl and alkynyl. In another embodiment R²⁴ and R²⁵ are independently selected from the group consisting of H and alkyl. In yet another embodiment R²⁴ and R²⁵ are independently selected from the group consisting of H, methyl, ethyl, isopropyl, propyl, 2-ethyl-propyl, 3,3-dimethyl-propyl, butyl, isobutyl, 3,3-dimethyl-butyl, 2-ethyl-butyl, pentyl, 2-methyl, pentyl, pent-4-enyl, hexyl, heptyl and octyl. In a specific embodiment R²⁴ and R²⁵ are both H.

In each of the above embodiments there are a number of values for R²⁶ and R²⁷. In one embodiment R²⁶ and R²⁷ are independently selected from the group consisting of: H, alkyl, alkenyl, alkynyl, alkoxyalkyl, and acyl. In another embodiment R²⁶ and R²⁷ are independently selected from the group consisting of: H, alkyl and acyl. In a further embodiment R²⁶ and R²⁷ are independently selected from the group consisting of H, methyl, ethyl, isopropyl, propyl, 2-ethyl-propyl, 3,3-dimethyl-propyl, butyl, isobutyl, 3,3-dimethyl-butyl, 2-ethyl-butyl, pentyl, 2-methyl, pentyl, pent-4-enyl, hexyl, heptyl, octyl, acetyl and 2-methoxy-ethyl.

In another embodiment R¹ is a heterocycloalkyl group which may optionally be substituted.

In one form of this embodiment the heterocycloalkyl group is selected from the group consisting of: 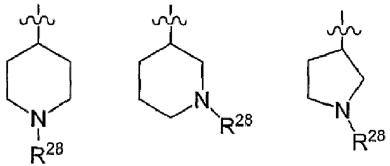 wherein R²⁸ is selected from the group consisting of H, halogen, alkyl, alkenyl, alkynyl, haloalkyl, haloalkenyl, heteroalkyl, cycloalkyl, cycloalkenyl, heterocycloalkyl, heterocycloalkenyl, aryl, heteroaryl, cycloalkylalkyl, heterocycloalkylalkyl, arylalkyl, heteroarylalkyl, arylalkenyl, cycloalkylheteroalkyl, heterocycloalkylheteroalkyl, heteroarylheteroalkyl, arylheteroalkyl, hydroxy, hydroxyalkyl, alkoxy, alkoxyalkyl, alkoxyaryl, alkenyloxy, alkynyloxy, cycloalkylkoxy, heterocycloalkyloxy, aryloxy, arylalkyloxy, heteroaryloxy, amino, alkylamino, aminoalkyl, acylamino, arylamino, phenoxy, benzyloxy, COOH, alkoxycarbonyl, alkylaminocarbonyl, arylacyl, sulfonyl, alkylsulfonyl, alkylsulfinyl, arylsulfonyl, arylsulfinyl, aminosulfonyl, SR⁵ and acyl, each of which may be optionally substituted.

In one embodiment R²⁸ is selected from the group consisting of H, alkyl, alkenyl, arylalkyl and arylacyl. Specific values of R²⁸ are H, methyl; ethyl; propyl; 2-methyl-propyl, 2-2-dimethyl-propyl; isopropyl; 3,3,3-triflouro-propyl; butyl; isobutyl; 3,3-dimethyl-butyl; pentyl; 2,4,4-trimethyl-pentyl; penten-4-yl, hexyl; heptyl, octyl, nonyl, 2-methoxy nonyl, benzyl, 2-phenyl-ethyl, 2-phenyl-acetyl, 3-phenyl-propyl,

In another embodiment the heterocycloalkyl group is pyrrolidyl, tetrahydrofuryl, tetrahydrothiofuranyl, piperidyl, piperazyl, tetrahydropyranyl, morphilino, 1,3-diazapane, 1,4-diazapane, 1,4-oxazepane, and 1,4-oxathiapane. In one specific embodiment al is selected from the group consisting of piperidine-3-yl, piperidine-4-yl and pyrollidin-3-y.

In another embodiment R¹ is a heteroaryl group.

In another embodiment R¹ is a group selected from the group consisting of:

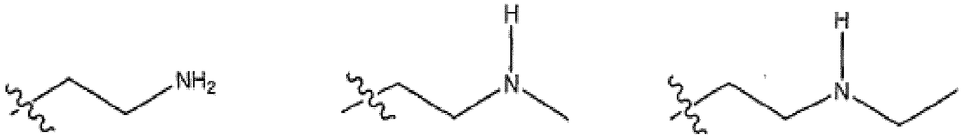

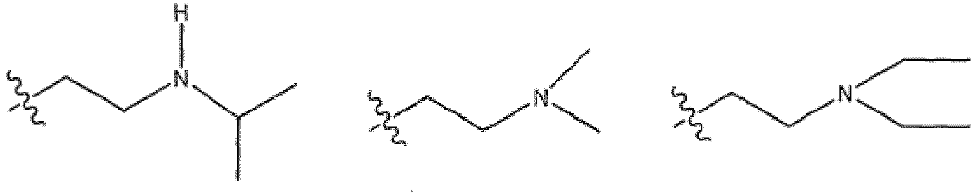

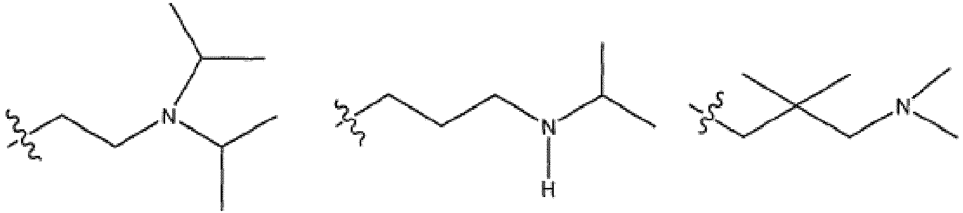

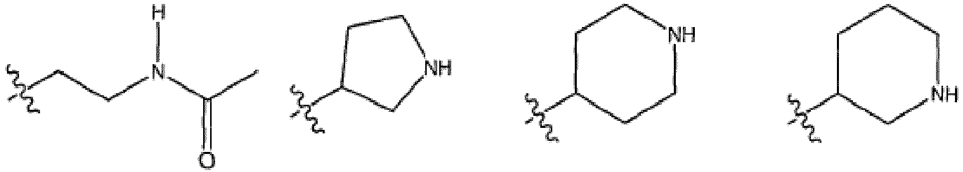

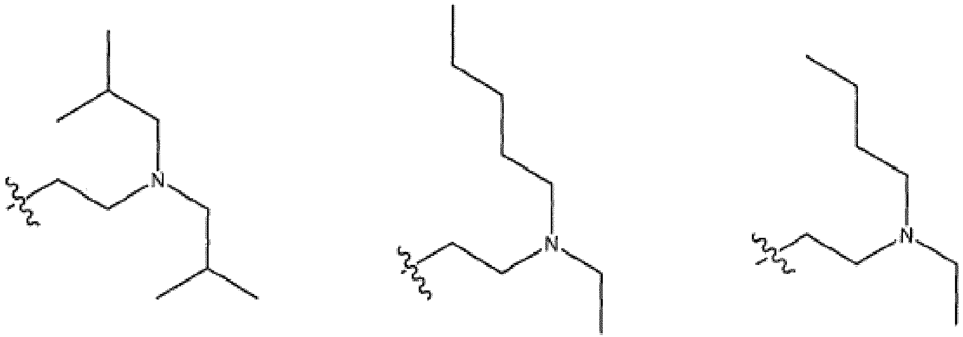

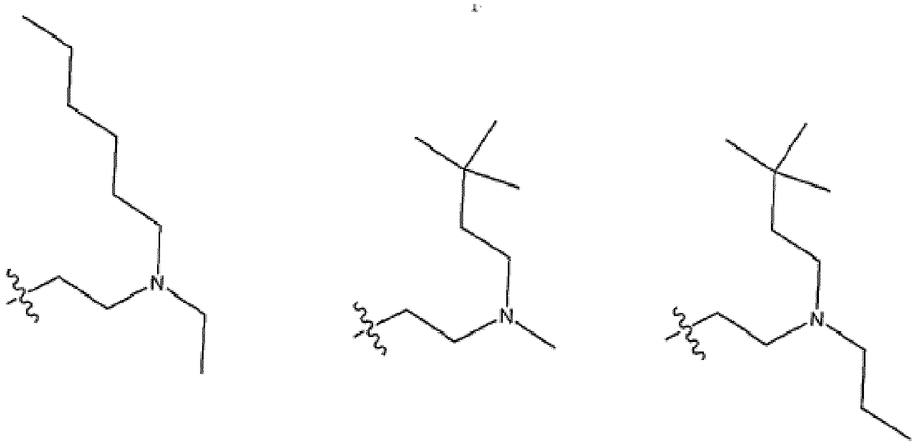

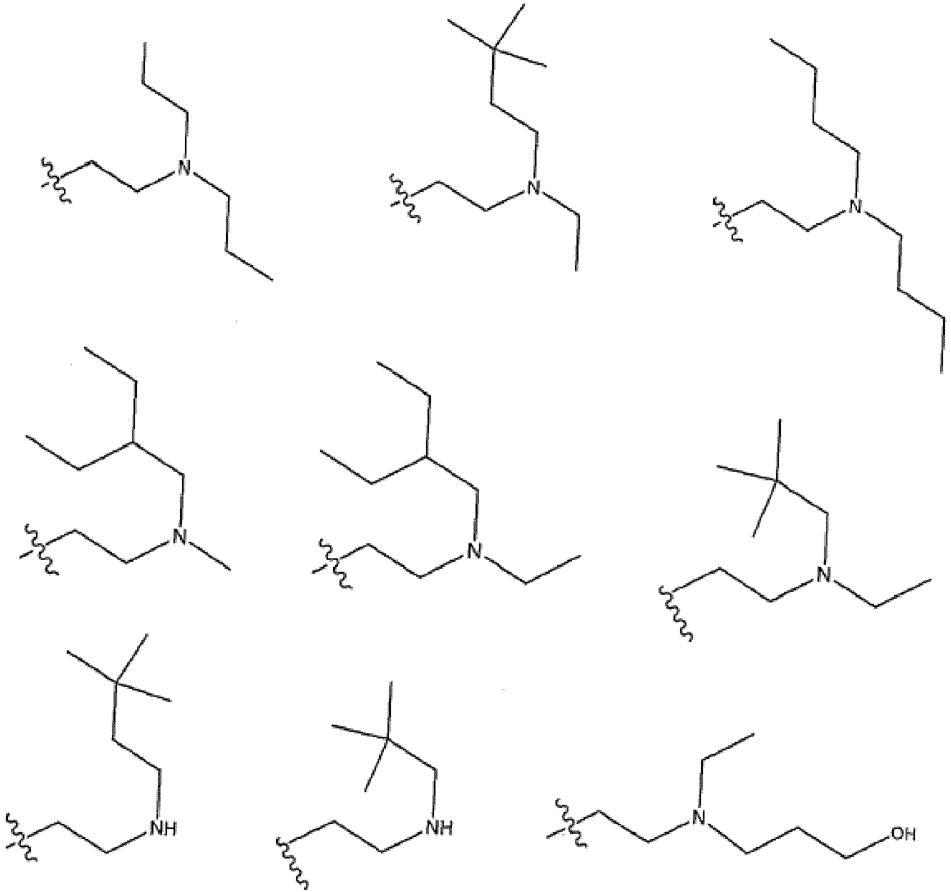

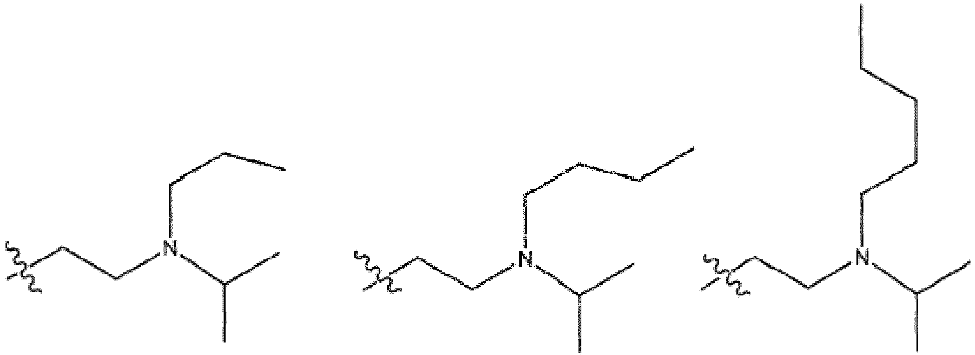

In one specific embodiment R¹ is a group of formula:

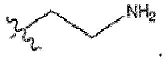

In another specific embodiment R¹ is a group of formula:

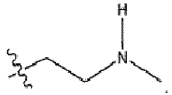

In another specific embodiment R¹ is a group of formula:

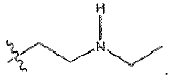

In yet another specific embodiment R¹ is a group of formula:

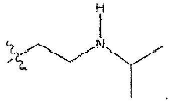

In another specific embodiment R¹ is a group of formula:

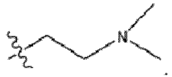

In another specific embodiment R¹ is a group of formula:

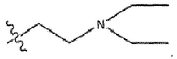

In another specific embodiment R¹ is a group of formula:

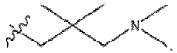

In another specific embodiment R¹ is a group of formula:

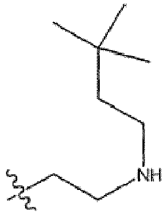

In another specific embodiment R¹ is a group of formula:

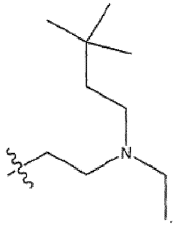

In one embodiment R² is selected from the group consisting of H, alkyl, cycloalkyl, heteroalkyl, alkenyl, alkynyl, alkoxyalkyl and cycloalkylalkyl, each of which may be optionally substituted.

In one form of this embodiment R² is alkyl. In one embodiment the alkyl is a C₁-C₁₀ alkyl. In another form of this embodiment the alkyl is a C₁-C₆ alkyl group. In another form of this embodiment R² is selected from the group consisting of: methyl; ethyl; propyl; 2-methyl-propyl, 2-2-dimethyl-propyl; isopropyl; 3,3,3-triflouro-propyl; butyl; isobutyl; 3,3- dimethyl-butyl; pentyl; 2,4,4-trimethyl-pentyl; hexyl; heptyl, octyl, nonyl, and 2-methoxy nonyl.

In one form of this embodiment R² is alkenyl. In one form of this embodiment the alkenyl is a C₁-Cₗ₀ alkenyl. In another form of this embodiment the alkenyl is a C₁-C₆ alkenyl group. In another form of this embodiment R² is selected from the group consisting of: ethenyl, prop-1-enyl, prop-2-enyl, but-1-enyl, but-2-enyl but-3-enyl, pent-1- enyl, pent-2-enyl, pent-3-enyl, pent-4-enyl, hex-1-enyl, hex-2-enyl, hex-3-enyl, hex-4-enyl and hex-5-enyl.

In another embodiment R² is selected from the group consisting of R¹¹S(O)R¹³-, R¹¹S(O)₂R¹³-, R¹¹C(O)N(R¹²)R¹³-, R¹¹SO₂N(R¹²)R¹³-, R¹¹N(R¹²)C(O)R¹³-, R¹¹N(R¹²)SO₂R¹³-, and R¹¹N(R¹²)C(O)N(R¹²) R¹³-. In one form of this embodiment R² is a group of the formula R¹¹C(O)N(R¹²)R¹³-. In one form of this embodiment R¹³ is a C₁-C₆ alkyl. In a specific form of this embodiment R¹³ is methyl or ethyl. In one form of this embodiment R¹² is H or C₁-C₆alkyl. A specific value for R¹² is H. In one form of this embodiment R¹¹ is C₁-C₆ alkyl group. Specific values for R¹¹ include t-butyl and propyl. Specific examples of groups of this type include: (CH₃)₃CCH₂CONH(CH₂)₂-; (CH₃)₃CCONH(CH₂)₂-; (CH₃)₃CCONH(CH₂)- and CH₃(CH₂)₂CONH(CH₂)-.

Specific values of R² are selected from the group consisting of: H; methyl; ethoxymethyl; [Bicylco[2.2.1]2-ylmethyl; Adamantan-2-ylmethyl; 2-methansulfanyl-ethyl; 2,2,2-triflouro-ethyl; propyl; 2-2-dimethyl-propyl; isopropyl; 3,3,3-triflouro-propyl; butyl; isobutyl; 3,3-dimethyl-butyl; but-3-enyl; but-3-yny; pentyl; 2,4,4-trimethyl-pentyl; Bicyclo[2.2.1]hept-5-en-2yl; hexyl; hex-3-enyl; octyl; non-3-enyl; non-6-enyl; 2-methoxynonyl, 2-phenyl-cyclopropyl; cyclohexyl; (CH₃)₃CCH₂CONH(CH₂)₂-; (CH₃)₃CCONH(CH₂)₂-; (CH₃)₃CCONH(CH₂)- and CH₃(CH₂)₂CONH(CH₂)-.

In one embodiment X and Y may be the same or different and are selected from the group consisting of H, halogen, C₁-C₄ alkyl, -CF₃, -NO₂, -C(O)R⁵, -CR⁶, -SR⁶, -CN and NR⁷R⁸.

In one embodiment X is H. In one embodiment Y is H. In one embodiment X and Y (if present) are at the 4 and 7 positions of the aromatic ring.

In one embodiment R³ is H, C₁-C₆ alkyl, or acyl. In another embodiment R³ is H or C₁-C₄ alkyl. In one embodiment, R³ is H.

In one embodiment R⁴ is H or C₁-C₄ alkyl.. In one embodiment, R⁴ is H.

In one embodiment R⁵ is C₁-C₄ alkyl, heteroalkyl, or acyl.. In one embodiment, R⁵ is methyl.

In one embodiment R⁶ is C₁-C₄ alkyl, heteroalkyl or acyl.. In one embodiment, R⁶ is C₁-C₄ alkyl;
In one embodiment R⁷ and R⁶ are selected from the group consisting of H, C₁-C₆ alkyl, C₄-C₉cycloalkyl, C₄-C₉heterocycloalkyl, aryl, heteroaryl, arylalkyl, and heteroarylalkyl.

Many if not all of the variables discussed above may be optionally substituted. If the variable is optionally substituted then in one embodiment the optional substituent is selected from the group consisting of: halogen, =O, =S, -CN, - NO₂, -CF₃, -OCF₃, alkyl, alkenyl, alkynyl, haloalkyl, haloalkenyl, haloalkynyl, heteroalkyl, cycloalkyl, cycloalkenyl, heterocycloalkyl, heterocycloalkenyl, aryl, heteroaryl, hydroxy, hydroxyalkyl, alkoxy, alkoxyalkyl, alkoxyaryl, alkoxyheteroaryl, alkenyloxy, alkynyloxy, cycloalkyloxy, cycloalkenyloxy, heterocycloalkyloxy, heterocycloalkenyloxy, aryloxy, heteroaryloxy, arylalkyl, heteroarylalkyl, arylalkyloxy, -amino, alkylamino, acylamino, aminoalkyl, arylamino, sulfonyl, alkylsulfonyl, arylsulfonyl, aminosulfonyl, aminoalkyl, alkoxyalky, -COOH, -COR⁵, -C(O)OR⁵, -SH, -SR⁵, -OR⁶ and acyl.

In a further embodiment the optional substituents are selected from the group consisting of: halogen, =O, =S, -CN, -NO₂, alkyl, alkenyl, heteroalkyl, haloalkyl, alkynyl, aryl, cycloalkyl, heterocycloalkyl, heteroaryl, hydroxy, hydroxyalkyl, alkoxy, alkylamino, aminoalkyl, acylamino, phenoxy, alkoxyalkyl, benzyloxy, alkylsulfonyl, arylsulfonyl, aminosulfonyl, -C(O)OR⁵, COOH, SH, and acyl.

In one embodiment the Z moiety is at the 5 or 6 position. In a specific embodiment the Z moiety is at the 5 position. In one embodiment the Z moiety is a group of formula -CH=CH-. If the Z moiety is a group of this type it is preferably in the "E" configuration.

Preferred HDAC inhibiting agents include those having an IC₅₀ value of 10 µM or less.

In addition to compounds of Formula (I), the embodiments disclosed are also directed to pharmaceutically acceptable salts, pharmaceutically acceptable prodrugs, and pharmaceutically active metabolites of such compounds, and pharmaceutically acceptable salts of such metabolites. Such compounds, salts, prodrugs and metabolites are at times collectively referred to herein as "HDAC inhibiting agents" or "HDAC inhibitors".

Specific compounds of the invention include the following:

| | |
|---|---|
| 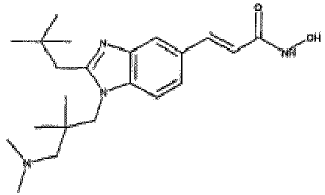 | 3-[1-(3-Dimethylamino-2,2-dimethyl-propyl)-2-(2,2-dimethyl-propyl)-1H-benzimidazol-5-yl]-N-hydroxy-acrylamide |
| 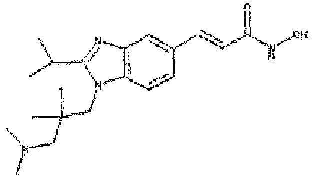 | 3-[1-(3-Dimethylamino-2,2-dimethyl-propyl)-2-isopropyl-1H-benzimidazol-5-yl]-N-hydroxy-acrylamide |
| 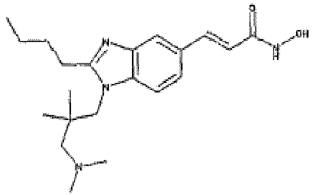 | 3-[2-Butyl-1-(3-dimethylamino-2,2-dimethylpropyl)-1H-benzimidazol-5-yl]-N-hydroxy-acrylamide |
| 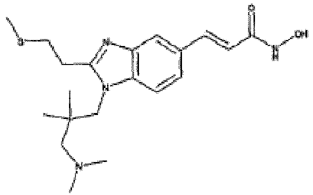 | 3-[1-(3-Dimethylamino-2,2-dimethyl-propyl)-2-(2-methylsulfanyl-ethyl)-1H-benzimidazol-5-yl]-N-hydroxy-acrylamide |
| 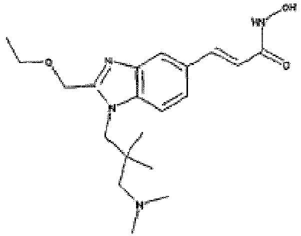 | 3-[1-(3-Dimethylamino-2,2-dimethyl-propyl)-2-ethoxymethyl-1H-benzimidazol-5-yl]-N-hydroxy-acrylamide |
| 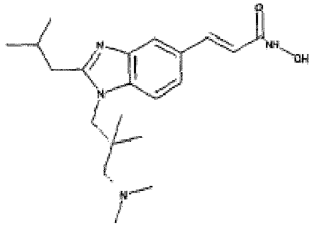 | 3-[1-(3-Dimethylamino-2,2-dimethyl-propyl)-2-isobutyl-1H-benzimidazol-5-yl]-N-hydroxy-acrylamide |
| 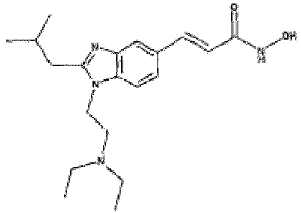 | 3-[1-(2-Diethylamino-ethyl)-2-isobutyl-1H-benzimidazol-5-yl]-N-hydroxy-acrylamide |
| 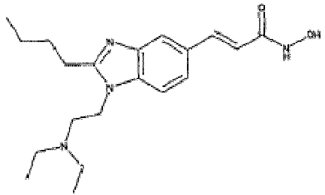 | 3-[2-Butyl-1-(2-diethylamino-ethyl)-1H-benzimidazol-5-yl]-N-hydroxy-acrylamide |
| 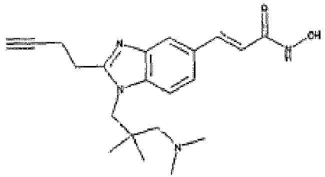 | 3-[2-But-3-ynyl-1-(3-dimethylamino-2,2-dimethyl-propyl)-1H-benzimidazol-5-yl]-N-hydroxy-acrylamide |
| 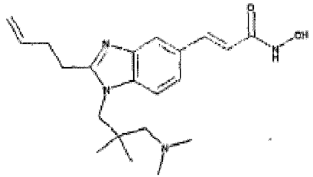 | 3-[2-But-3-enyl-1-(3-dimethylamino-2,2-dimethyl-propyl)-1H-benzimidazol-5-yl]-N-hydroxy-acrylamide |
| 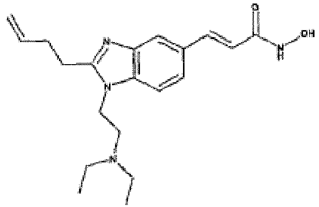 | 3-[2-But-3-enyl-1-(2-diethylamino-ethyl)-1H-benzimidazol-5-yl]-N-hydroxy-acrylamide |
| 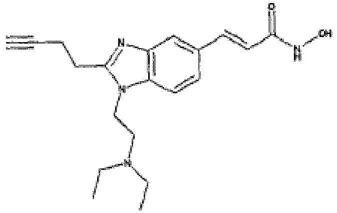 | 3-[2-But-3-ynyl-1-(2-diethylamino-ethyl)-1H-benzimidazol-5-yl]-N-hydroxy-acrylamide |
| 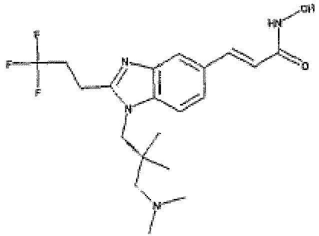 | 3-[1-(3-Dimethylamino-2,2-dimethyl-propyl)-2-(3,3,3-trifluoro-propyl)-1H-benzimidazol-5-yl]-N-hydroxy-acrylamide |
| 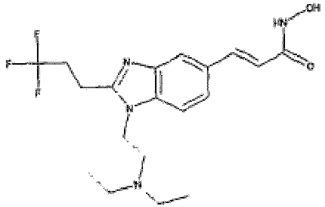 | 3-[1-(2-Diethylamino-ethyl)-2-(3,3,3-trifluoro-propyl)-1H-benzimidazol-5-yl]-N-hydroxy-acrylamide |
| 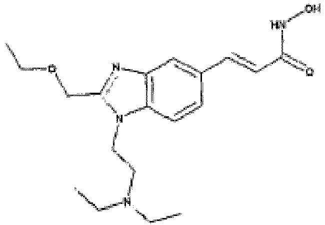 | 3-[1-(2-Diethylamino-ethyl)-2-ethoxymethyl-1H-benzimidazol-5-yl]-N-hydroxy-acrylamide |
| 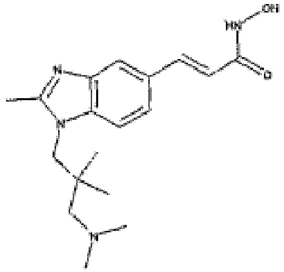 | 3-[1-(3-Dimethylamino-2,2-dimethyl-propyl)-2-methyl-1H-benzimidazol-5-yl]-N-hydroxy-acrylamide |
| 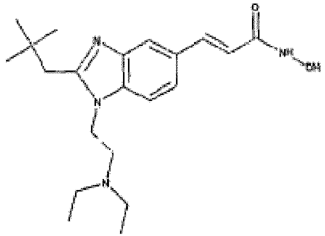 | 3-[1-(2-Diethylamino-ethyl)-2-(2,2-dimethyl-propyl)-1H-benzimidazol-5-yl]-N-hydroxy-acrylamide |
| 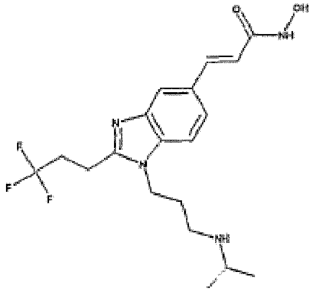 | N-Hydroxy-3-[1-(3-isopropylamino-propyl)-2-(3,3,3-trifluoro-propyl)-1H-benzimidazol-5-yl]-acrylamide |
| 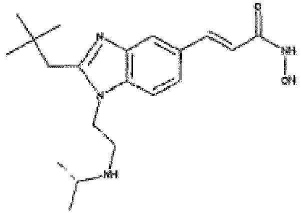 | 3-[2-(2,2-Dimethyl-propyl)-1-(2-isopropylaminoethyl)-1 H-benzimidazol-5-yl]-N-hydroxy-acrylamide |
| 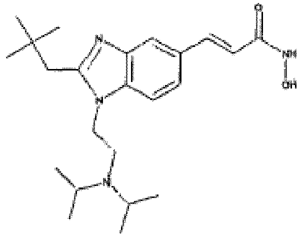 | 3-[1-(2-Disopropylamino-ethyl)-2-(2,2-dimethylpropyl)-1H-benzimidazol-5-yl]-N-hydroxy-acrylamide |
| 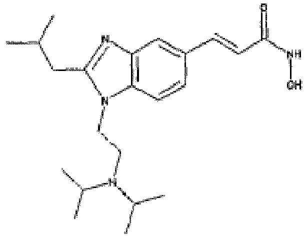 | 3-[1-(2-Disopropylamino-ethyl)-2-isobutyl-1H-benzimidazol-5-yl]-N-hydroxy-acrylamide |
| 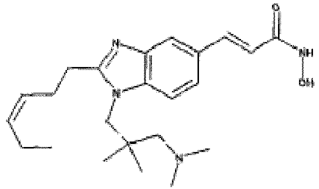 | 3-[1-(3-Dimethylamino-2,2-dimethyl-propyl)-2-hex-3-enyl-1H-benzimidazol-5-yl]-N-hydroxy-acrylamide |
| 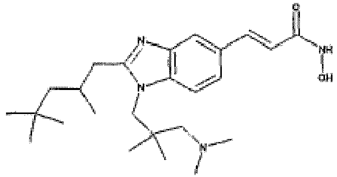 | 3-[1-(3-Dimethylamino-2,2-dimethyl-propyl)-2-(2,4,4-trimethyl-pentyl)-1H-benzimidazol-5-yl]-N-hydroxy-acrylamide |
| 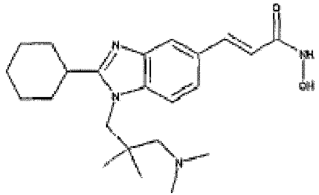 | 3-[2-Cyclohexyl-1-(3-dimethylamino-2,2-dimethyl-propyl)-1H-benzimidazol-5-yl]-N-hydroxy-acrylamide |
| 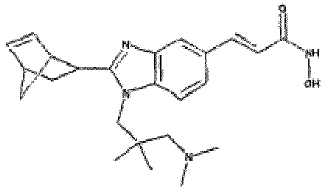 | 3-[2-Bicyclo[2.2.1]hept-5-en-2-yl-1-(3-dimethylamino-2,2-dimethyl-propyl)-1H-benzimidazol-5-yl]-N-hydroxy-acrylamide |
| 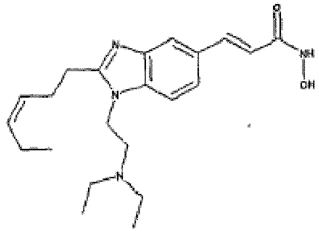 | 3-[ 1-(2-Diethylamino-ethyl)-2-hex-3-enyl-1H-benzimidazol-5-yl]-N-hydroxy-acrylamide |
| 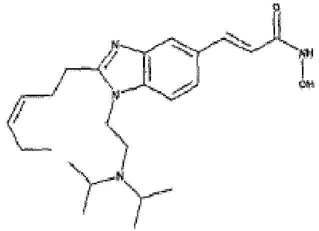 | 3-[1-(2-Diisopropylamino-ethyl)-2-hex-3-enyl-1H-benzimidazol-5-yl]-N-hydroxy-acrylamide |
| 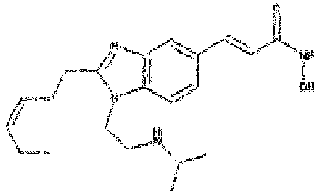 | 3 -[2-Hex-3-enyl-1-(2-isopropylamino-ethyl)-1H-benzimidazol-5-yl]-N-hydroxy-acrylamide |
| 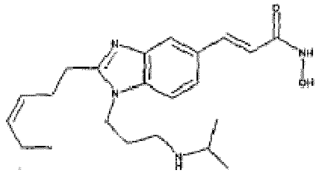 | 3-[2-Hex-3-enyl-1-(3-isopropylamino-propyl)-1H-enzimidazol-5-yl]-N-hydroxy-acrylamide |
| 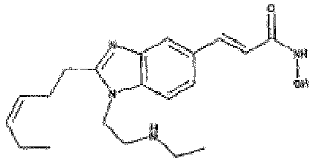 | 3-[1-(2-Ethylamino-ethyl)-2-hex-3-enyl-1H-benzimidazol-5-yl]-N-hydroxy-acrylamide |
| 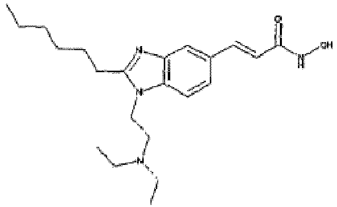 | 3-[1-(2-Diethylamino-ethyl)-2-hexyl-1H-benzimidazol-5-yl]-N-hydroxy-acrylamide |
| 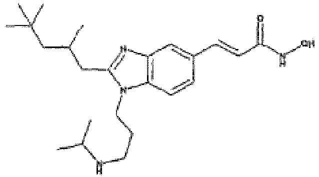 | N-Hydroxy-3-[1-(3-isopropylamino-propyl)-2-(2,4,4-trimethyl-pentyl)-1H-benzimidazol-5-yl]-acrylamide |
| 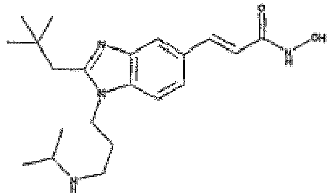 | 3-[2-(2,2-Dimethyl-propyl)-1-(3-isopropylaminopropyl)-1H-benzimidazol-5-yl]-N-hydroxy-acrylamide |
| 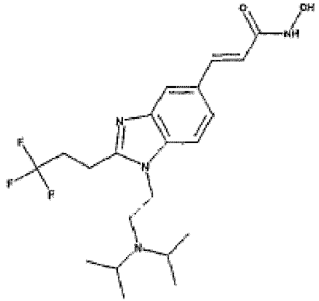 | 3-[1-(2-Diisopropylammo-ethyl)-2-(3,3,3-trifluoro-propyl)-1H-benzimidazol-5-yl]-N-hydroxy-acrylamide |
| 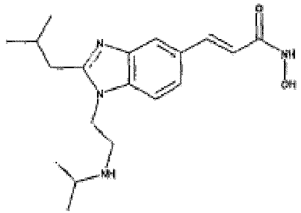 | N-Hydroxy-3-[2-isobutyl-1-(2-isopropylaminoethyl)-1H-benzimidazol-5-yl]-acrylamide |
| 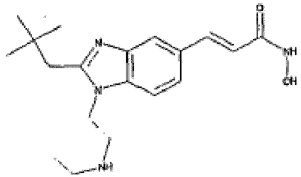 | 3-[2-(2,2-Dimethyl-propyl)-1-(2-ethylaminoethyl)-1H-benzimidazol-5-yl]-N-hydroxy-acrylamide |
| 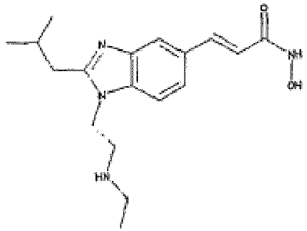 | 3-[1-(2-Ethylamino-ethyl)-2-isobutyl-1H-benzimidazol-5-yl]-N-hydroxy-acrylamide |
| 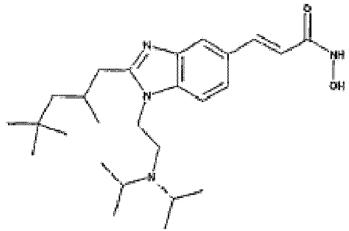 | 3-[1-(2-Diisopropylamino-ethyl)-2-(2,4,4-trimethyl-pentyl)-1H-benzimidazol-5-yl]-N-hydroxy-acrylamide |
| 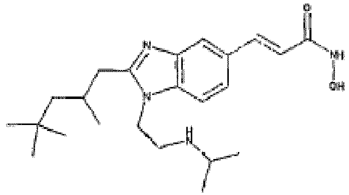 | N-Hydroxy-3-[1-(2-isopropylamino-ethyl)-2-(2,4,4-trimethyl-pentyl)-1H-benzimidazol-5-yl]-acrylamide |
| 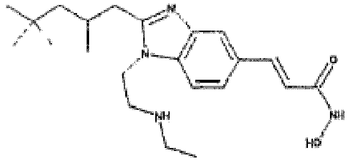 | 3-[1-(2-Ethylamino-ethyl)-2-(2,4,4-trimethyl-pentyl)-1H-benzimidazol-5-yl]-N-hydroxy-acrylamide |
| 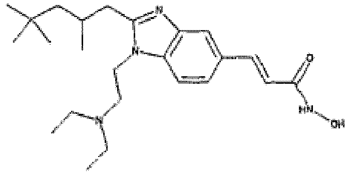 | 3-[1-(2-Diethylamino-ethyl)-2-(2,4,4-trimethyl-pentyl)-1H-benzimidazol-5-yl]-N-hydroxy-acrylamide |
| 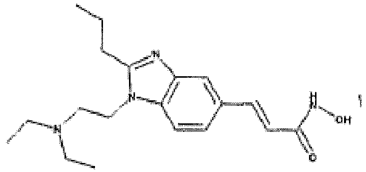 | 3-[1-(2-Diethylamino-ethyl)-2-propyl-1H-benzimidazol-5-yl]-N-hydroxy-acrylamide |
| 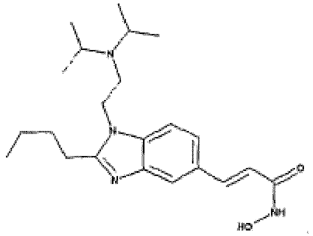 | 3[2-Butyl-1-(2-diisopropylamino-ethyl)-1H-benzimidazol-5-yl]-N-hydroxy-acrylamide |
| 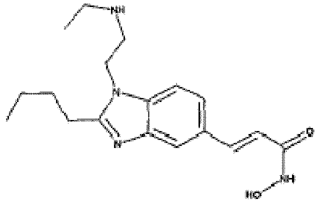 | 3[2-Butyl-1-(2-ethylamino-ethyl)-1H-benzimidazol-5-yl]-N-hydroxy-acrylamide |
| 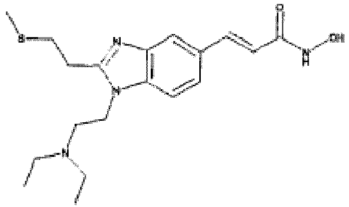 | 3-[1-(2-Diethylamino-ethyl)-2-(2-methylsulfanyl-ethyl)-1H-benzimidazol-5-yl]-N-hydroxy-acrylamide |
| 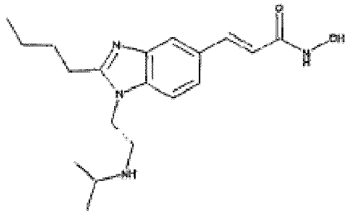 | 3[2-Butyl-1-(2-isopropylamino-ethyl)-1H-benzimidazol-5-yl]-N-hydroxy-acrylamide |
| 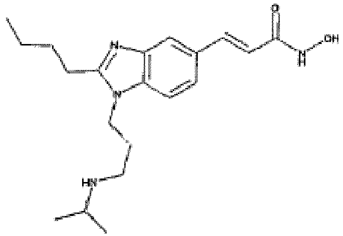 | 3[2-Butyl-1-(3-isopropylamino-propyl)-1H-benzimidazol-5-yl]-N-hydroxy-acrylamide |
| 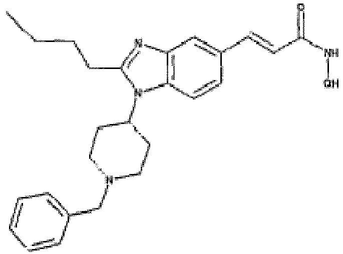 | 3-[1-(1-Benzyl-piperidin-4-yl)-2-butyl-1H-benzimidazol-5-yl]-N-hydroxy-acrylamide |
| 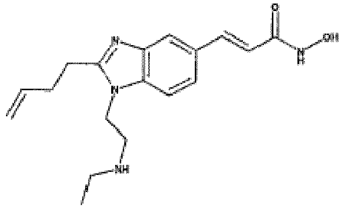 | 3-[2-But-3-enyl-1-(2-ethylamino-ethyl)-1H-benzimidazol-5-yl]-N-hydroxy-acrylamide |
| 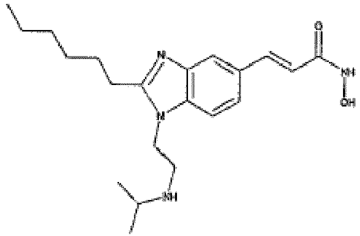 | 3[2-Hexyl-1-(2-isopropylamino-ethyl)-1H-benzimidazol-5-yl]-N-hydroxy-acrylamide |
| 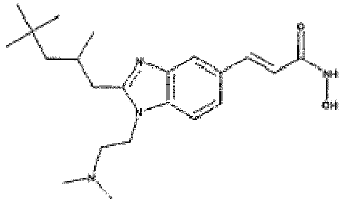 | 3-[1-(2-Dimethylamino-ethyl)-2-(2,4,4-trimethyl-pentyl)-1H-benzimidazol-5-yl]-N-hydroxy-acrylamide |
| 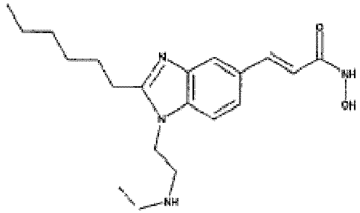 | 3-[1-(2-Ethylamino-ethyl)-2-hexyl-1H-benzimidazol-5-yl]-N-hydroxy-acrylamide |
| 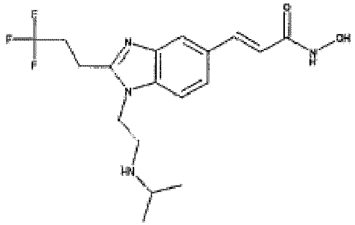 | N-Hydroxy-3-[1-(2-isopropylamino-ethyl)-2-(3,3,3-trifluoro-propyl)-1H-benzimidazol-5-yl]-acrylamide |
| 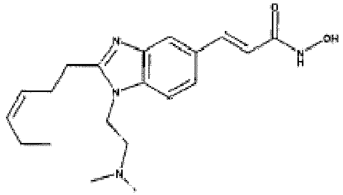 | 3-[1-(2-Dimethylamino-ethyl)-2-hex-3-enyl-1H-benzimidazol-5-yl]-N-hydroxy-acrylamide |
| 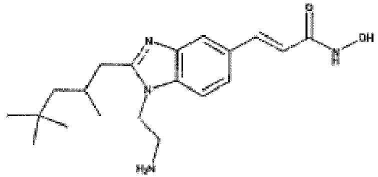 | 3-[1-(2-Amino-ethyl)-2-(2,4,4-trimethyl-pentyl)-1H-benzimidazol-5-yl]-N-hydroxy-acrylamide |
| 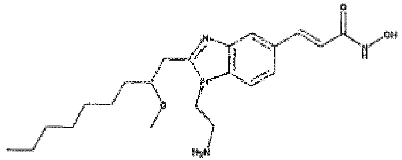 | 3-[1-(2-Amino-ethyl)-2-(2-methoxy-nonyl)-1H-benzimidazol-5-yl]-N-hydroxy-acrylamide |
| 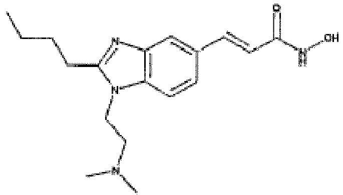 | 3-[2-Butyl-1-(2-dimethylamino-ethyl)-1H-benzimidazol-5-yl]-N-hydroxy-acrylamide |
| 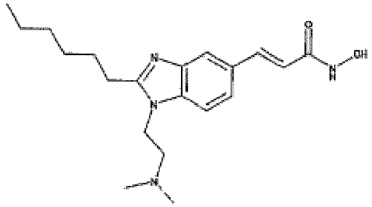 | 3-[1-(2-Dimethylamino-ethyl)-2-hexyl-1H-benzimidazol-5-yl]-N-hydroxy-acrylamide |
| 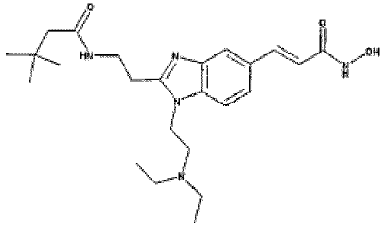 | N-{2-[1-(2-Diethylamino-ethyl)-5-(2-hydroxycarbamoyl-vinyl)-1H-benzimidazol-2-yl]-ethyl}-3,3-dimethyl-butyramide |
| 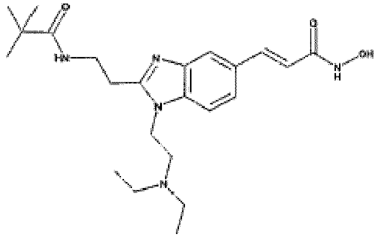 | 3-{1-(2-Diethylamino-ethyl)-2[2-(2,2-dimethyl-propionylamino)-ethyl]-1H-benzimidazol-5-yl)-N-hydroxy-acrylamide |
| 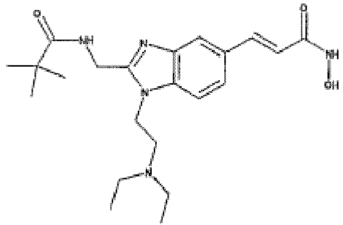 | 3-{1-(2-Diethylamino-ethyl)-2-[(2,2-dimethyl-propionylamino)-methyl]-1H-benzimidazol-5-yl} - N-hydroxy-acrylamide |
| 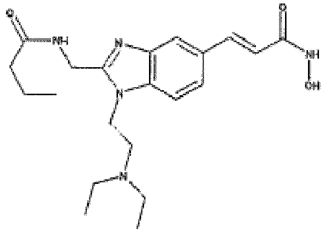 | N[ 1-(2-Diethylamino-ethyl)-5-(2-hydroxycarbamoyl-vinyl)-1H-benzimidazol-2-ylmethyl]-butyramide |
| 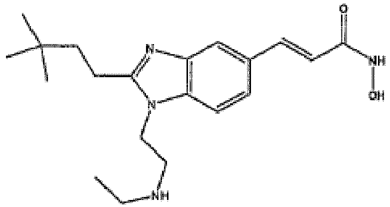 | 3-[1-(2-ethylamino-ethyl)-2-(3,3-dimethyl-butyl)-1H-benzimidazol-5-yl]-N-hydroxy-acrylamide |
| 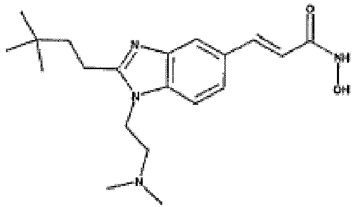 | 3-[2-(3,3-Dimethyl-butyl)-1-(2-Dimethylaminoethyl)-1H-benzimidazol-5-yl]-N-hydroxy-acrylamide |
| 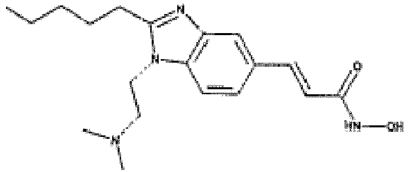 | 3-[1-(2-Dimethylamino-ethyl)-2-pentyl-1H-benzimidazol-5-yl]-N-hydroxy-acrylamide |
| 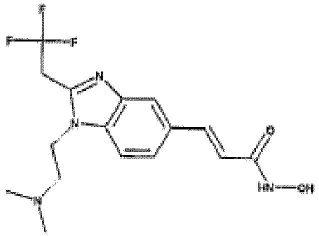 | 3-[1-(2-Dimethylamino-ethyl)-2-(2,2,2-trifluoroethyl)-1 H-benzimidazol-5-yl]-N-hydroxy-acrylamide |
| 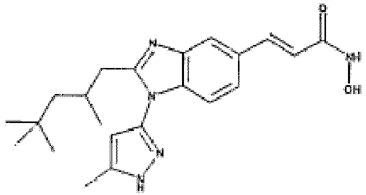 | N-Hydroxy-3-[1-(5-methyl-1H-pyrazol-3-yl)-2-(2,4,4-trimethyl-pentyl)-1H-benzimidazol-5-yl]-acrylamide |
| 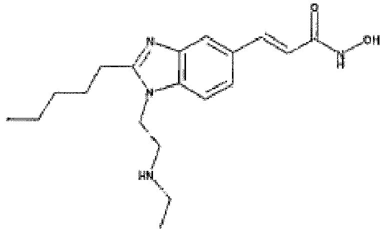 | 3-[1-(2-Ethylamino-ethyl)-2-pentyl-1H-benzimidazol-5-yl]-N-hydroxy-acrylamide |
| 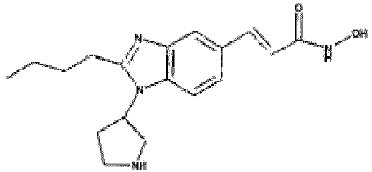 | 3-(2-Butyl-1-pyrrolidin-3-yl-1H-benzimidazol-5-yl)-N-hydroxy-acrylamide |
| 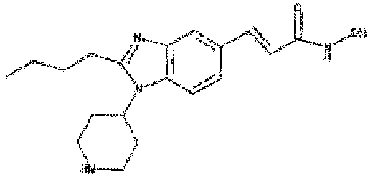 | 3-(2-Butyl-1-piperidin-4-yl-1H-benzimidazol-5-yl)-N-hydroxy-acrylamide |
| 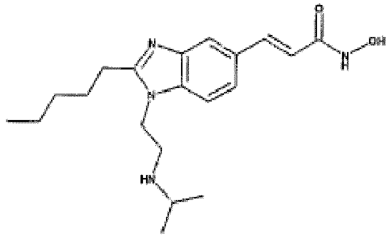 | N-Hydroxy-3-[1-(2-isopropylamino-ethyl)-2-pentyl-1H-benzimidazol-5-yl]-acrylamide |
| 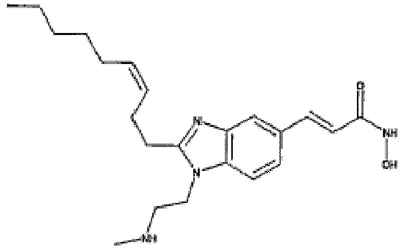 | N-Hydroxy-3-[1-(2-methylamino-ethyl)-2-non-3-enyl-1H-benzimidazol-5-yl]-acrylamide |
| 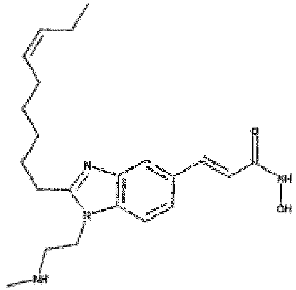 | N-Hydroxy-3-[1-(2-methylamino-ethyl)-2-non-6-enyl-1H-benzimidazol-5-yl]-acrylamide |
| 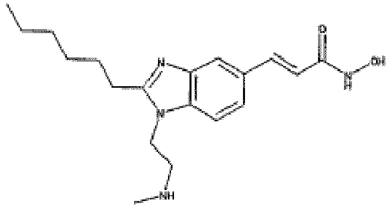 | 3-[2-Hexyl-1-[1-(2-methylamino-ethyl)-1H-benzimidazol-5-yl]-N-hydroxy-acrylamide |
| 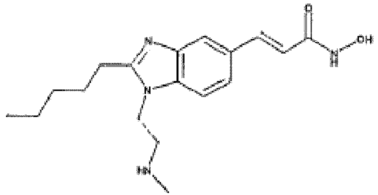 | N-Hydroxy-3-[1-(2-methylamino-ethyl)-2-pentyl-1H-benzimidazol-5-yl]-acrylamide |
| 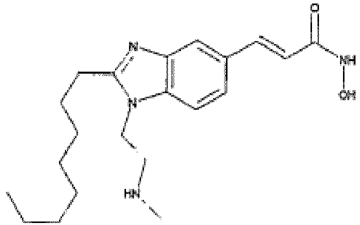 | N-Hydroxy-3-[1-(2-methylamino-ethyl)-2-octyl-1H-benzimidazol-5-yl]-acrylamide |
| 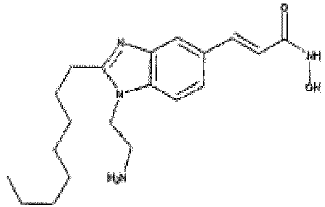 | 3-[1-(2-Amino-ethyl)-2-octyl-1H-benzimidazol-5- yl]-N-hydroxy-acrylamide |
| 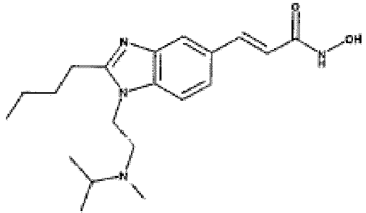 | 3-{2-Butyl-1-[2-(isopropyl-methyl-amino)-ethyl]-1H-benzimidazol-5-yl}-N-hydroxy-acrylamide |
| 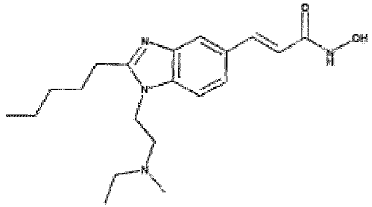 | 3-{1[2-(Ethyl-methyl-amino)-ethyl]-2-pentyl-1H-benzimidazol-5-yl)-N-hydroxy-acrylamide |
| 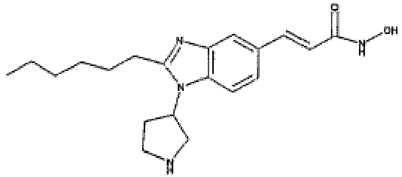 | 3-(2-Hexyl-1-pyrrolidin-3-yl-1H-benzimidazol-5--hydroxy-acrylamide |
| 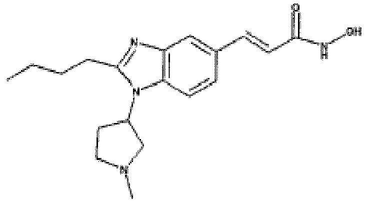 | 3-[2-Butyl-1-(1-methyl-pyrrolidin-3-yl)-1H-benzimidazol-5-yl]-N-hydroxy-acrylamide |
| 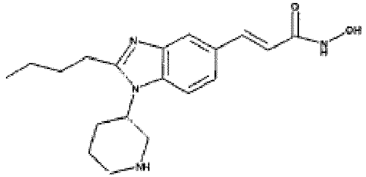 | 3-(2-Butyl-1-piperidin-3-yl-1H-benzimidazol-5--hydroxy-acrylamide |
| 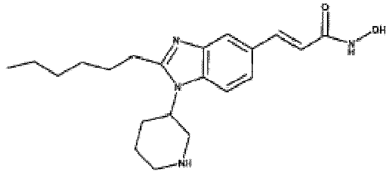 | 3-(2-Hexyl-1-piperidin-3-yl-1H-benzimidazol-5--hydroxy-acrylamide |
| 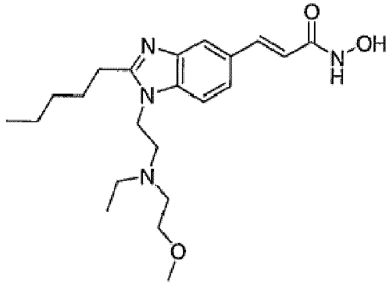 | 3-(1-{2-[Ethyl-(2-methoxy-ethyl)-amino]-ethyl}-2-pentyl-1H-benzimidazol-5-yl)-N-hydroxy-acrylamide |
| 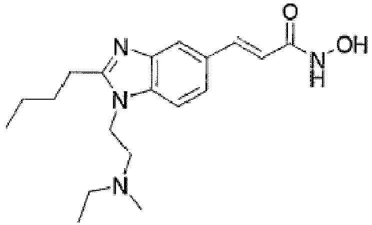 | 3-{2-Butyl-1-[2-(ethyl-methyl-ammo)-ethyl]-1H-benzimidazol-5-yl}-N-hydroxy-acrylamide |
| 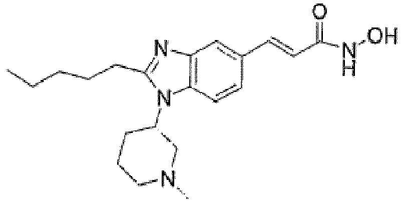 | N-Hydroxy-3-[1-(1-methyl-piperidin-3-yl)-2-pentyl-1H-benzimidazol-5-yl]-acrylamide |
| 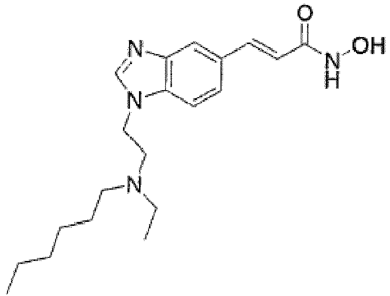 | 3-{1-[2-(Ethyl-hexyl-amino)-ethyl]-1H-benzimidazol-5-yl}-N-hydroxy-acrylamide |
| 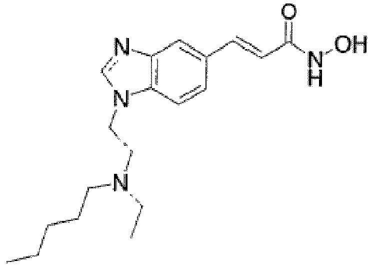 | 3-{1-[2-(Ethyl-pentyl-amino)-ethyl]-1H-benzimidazol-5-yl}-N-hydroxy-acrylamide |
| 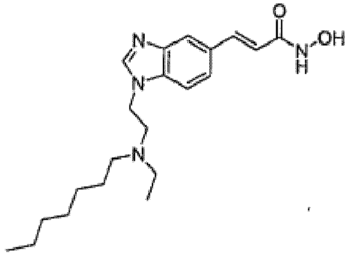 | 3-{1-[2-(Ethyl-heptyl-amino)-ethyl]-1H-benzimidazol-5-yl}-N-hydroxy-acrylamide |
| 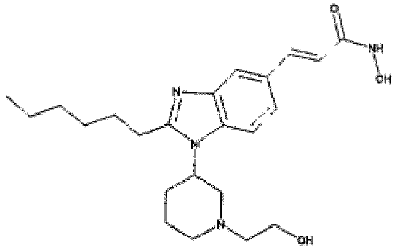 | (*E*)-3-(2-hexyl-1-(1-(2-hydroxyethyl)piperidin-3-yl)-1H-benzo[d]imidazol-5-yl)-N-hydroxyacrylamide |
| 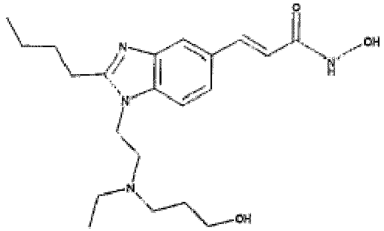 | 3-(2-Butyl-1-{2-ethyl-(3-hydroxy-propyl)-amino]-ethyl}-1H-benzimidazol-5-yl)-N-hydroxy-acrylamide |
| 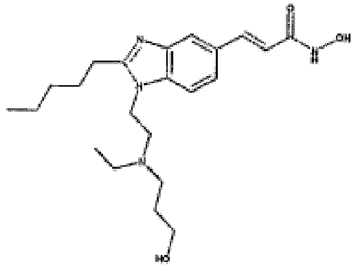 | 3-(1-{2-[Ethyl-(3-hydroxy-propyl)-ammo]-ethyl}-2-pentyl-1H-benzimidazol-5-yl)-N-hydroxy acrylamide |
| 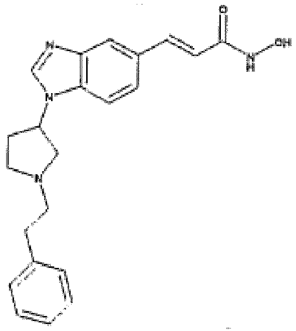 | (E)-N-hydroxy-3-(1-(1-phenethylpyrrolidin-3-yl)-1H-benzo [d]imidazol-5-yl)acrylamide |
| 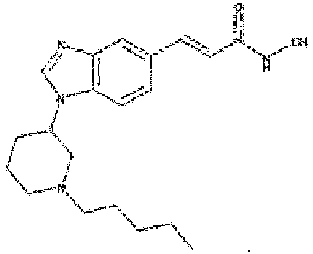 | (E)-N-hydroxy-3-(1-(1-pentylpiperidin-3-yl)-1H-benzo[d]imidazol-5-ypacrylamide |
| 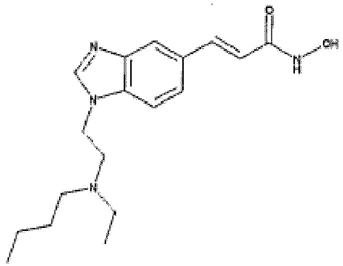 | 3-{1-2-Butyl-ethyl-aminoyethyl]-1H-benzimidazol-5-yl}-N-hydroxy-acrylamide |
| 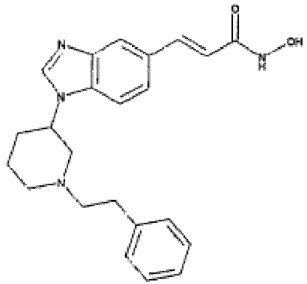 | (E)-N-hydroxy-3-(1-(1-phenethylpiperidin-3-yl)-1H-benzo [d]imidazol-5-yl)acrylamide |
| 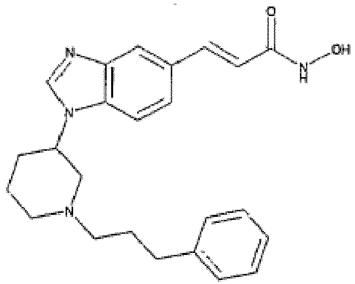 | (E)-N-hydroxy-3-(1-(1-(3-phenylpropyl)piperidin-3-yl)-1H -benzo[d]imidazol-5-yl)acrylamide |
| 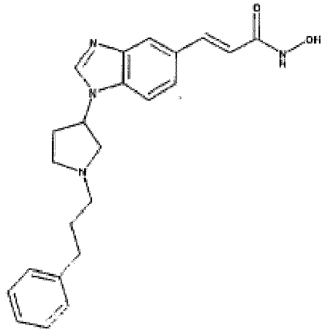 | (E)-N-hydroxy-3-(1-(1-(3-phenylpropyl)pyrrolidin-3-yl)-1H-benzo[d]imidazol-5-yl)acrylamide |
| 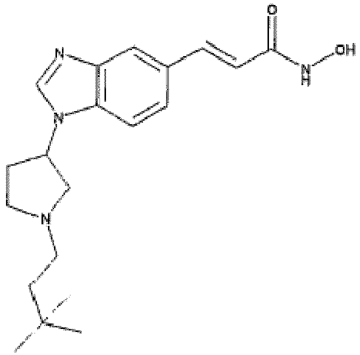 | 3-{1-[1-(3,3-Dimethyl-butyl)-pyrrolidm-3-yl]-1H-benzimidazol-5-yl}-N-hydroxy-acrylamide |
| 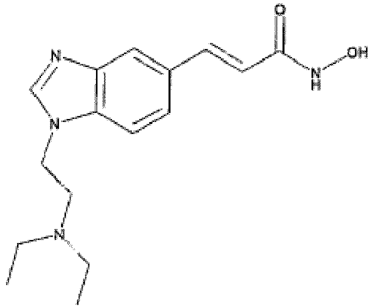 | (E)-3-(1-(2-(diethylamino)ethyl)-1H-benzo[d]imidazol-5-yl)-N-hydroxyacrylamide |
| 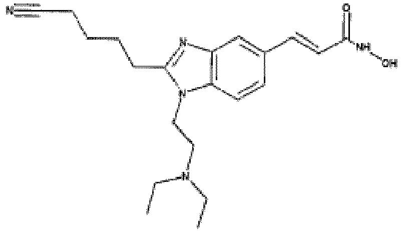 | 3-[2-(4-Cyano-butyl)-1-(2-diethylamino-ethyl)-1H-benzimidazol-5-yl]-N-hydroxy-acrylamide |
| 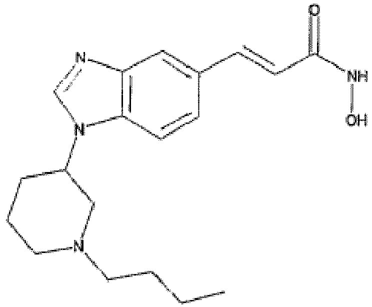 | (E)-3-(1-(1-butylpiperidin-3-yl)-1H-benzo[d]imidazol-5-yl)-N-hydroxyacrylamide |
| 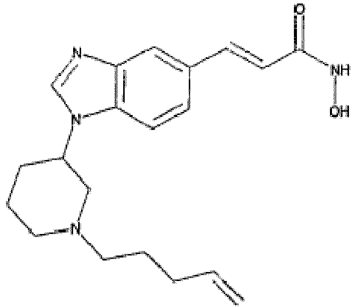 | (E)-N-hydroxy-3-(1-(1-(pent-4-enyl)piperidin-3-yl)-1H-benzo[d]imidazol-5-yl)acrylamide |
| 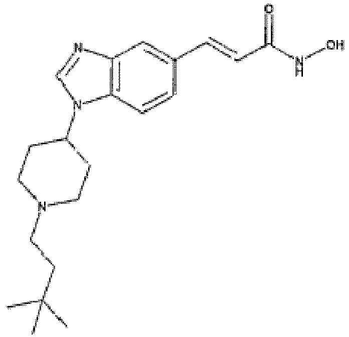 | (E)-3-(1-(1-(3,3-dimethylbutyl)piperidin-4-yl)-1H-benzo[d]imidazol-5-yl)-N-hydroxyacrylamide |
| 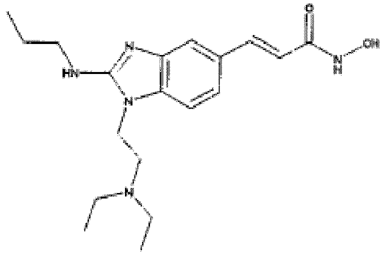 | 3-[ 1-(2-Diethylamino-ethyl)-2-propylamino-1H-benzimidazol-5-yl]-N-hydroxy-acrylamide |
| 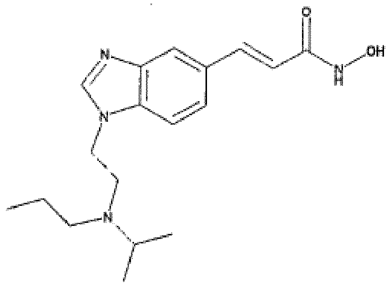 | (E)-N-hydroxy-3-(1-(2-(isopropyl(propyl)amino)ethyl)-1H-benzo[d]imidazol-5-yl)acrylamide |
| 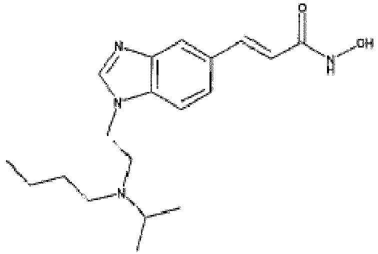 | 3-{1-[2-(Butyl-isopropyl-amino)-ethyl]-1H-benzimidazol-5-yl}-N-hydroxy-acrylamide |
| 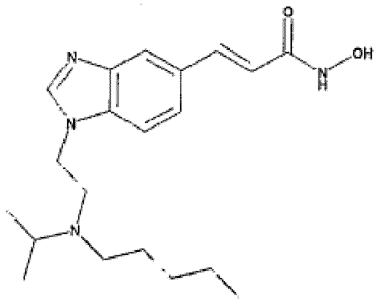 | N-Hydroxy-3-{1[2-(isopropyl-pentyl-amino)-ethyl]-1H-benzimidazol-5-yl]-acrylamide |
| 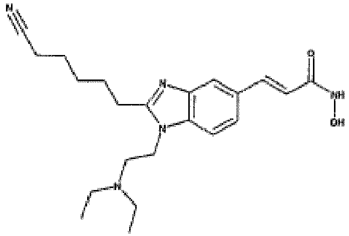 | 3-[2-(5-Cyano-pentyl)-1-(2-diethylamino-ethyl)-1H-benzimidazol-5-yl]-N-hydroxy-acrylamide |
| 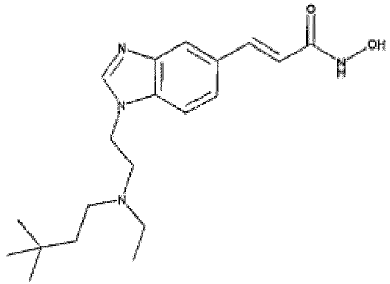 | 3-(1-{2-[(3,3-Dimethyl-butyl)-ethyl-amino]-ethyl}-1H-benzimidazol-5-yl)-N-hydroxy-acrylamide |
| 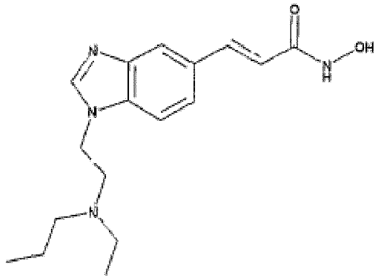 | 3-{1-[2-(Ethyl-propyl-amino)-ethyl]-1H-benzimidazol-5-yl}-N-hydroxy-acrylamide |
| 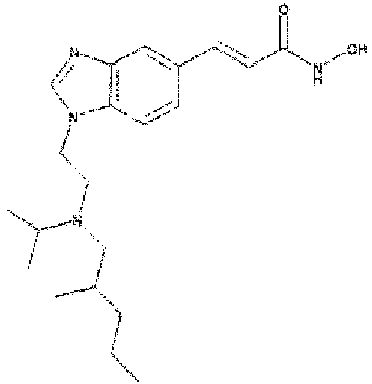 | N-Hydroxy-3-(1-{2-isopropyl-(2-methyl-pentyl)-amino]-ethyl]-1H-benzimidazol-5-yl)-acrylamide |
| 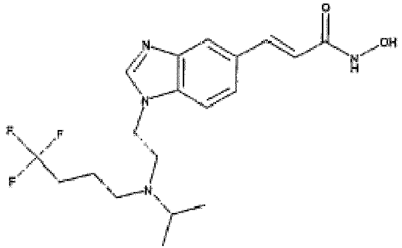 | (E)-N-hydroxy-3-(1-(2-(isopropyl(4,4,4-trifluorobutyl)amino )ethyl)-1H-benzo[d]imidazol-5-yl)acrylamide |
| 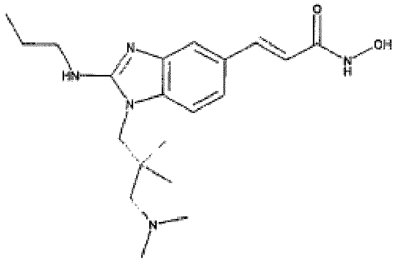 | 3-[1-(3-Dimethylamino-2,2-dimethyl-propyl)-2-propylamino-1H-benzimidazol-5-yl]-N-hydroxy-acrylamide |
| 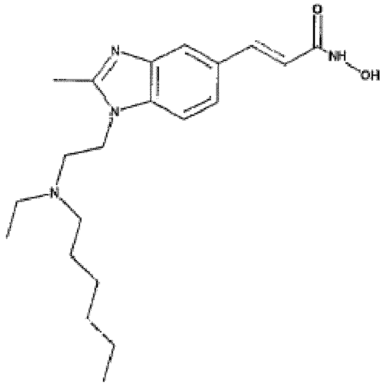 | 3-{1-[2-Ethyl-hexyl-amino)-ethyl]-2-methyl-1H-benzimidazol-5-yl]-N-hydroxy-acrylamide |
| 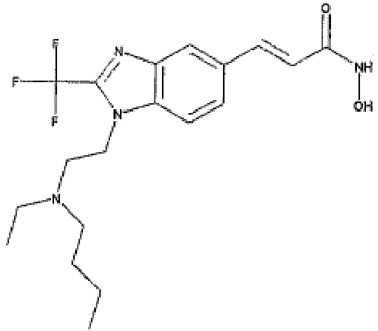 | 3-{1-[2-(Butyl-ethyl-amino)-ethyl]-2-trifluoromethyl-1H-benzimidazol-5-yl}-N-hydroxy-acrylamide |
| 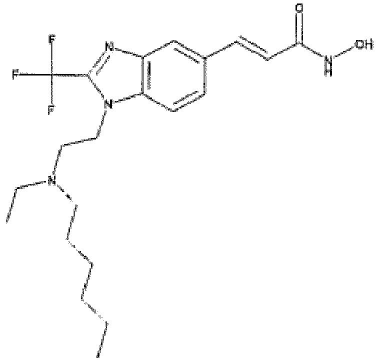 | 3-{1-[2-(Ethyl-hexyl-amino)-ethyl]-2-trifluoromethyl-1H-benzimidazol-5-yl}-N-hydroxy-acrylamide |
| 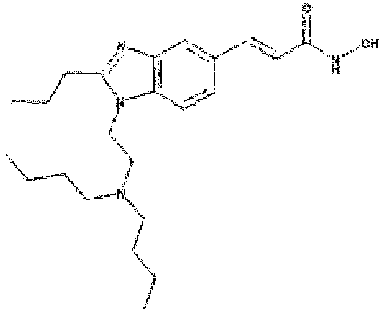 | (E)-3-(1-(2-(dibutylamino)ethyl)-2-propyl-1H-benzo[d]imidazol-5-yl)-N-hydroxyacrylamide |
| 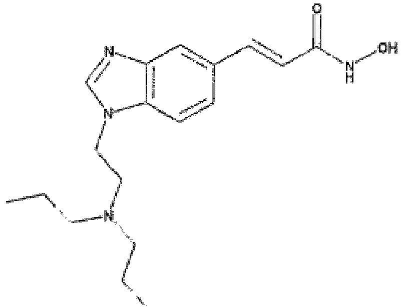 | 3-[1-(2-Dipropylamino-ethyl)-1H-benzimidazol-5-yl]-N-hydroxy-acrylamide |
| 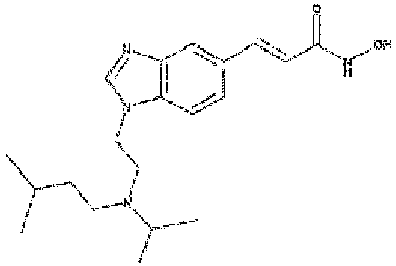 | N-Hydroxy-3-(1-{2-[isopropyl-(3-methyl-butyl)-amino]-ethyl}-1H-benzimidazol-5-yl)-acrylamide |
| 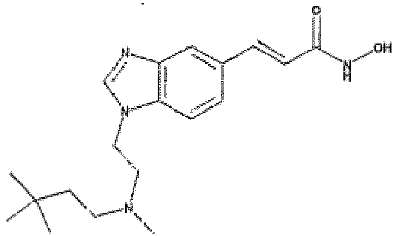 | 3-(1-{2-[(3,3-Dimethyl-butyl)-methyl-amino]-ethyl}-1H-benzimidazol-5-yl)-N-hydroxy-acrylamide |
| 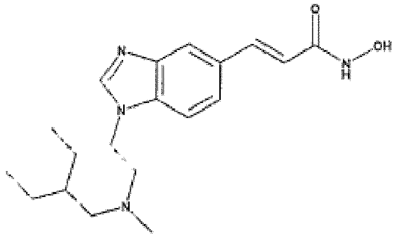 | 3-(1-{2-[(2-Ethyl-butyl)-methyl-amino]-ethyl}-1H-benzimidazol-5-yl)-N-hydroxy-acrylamide |
| 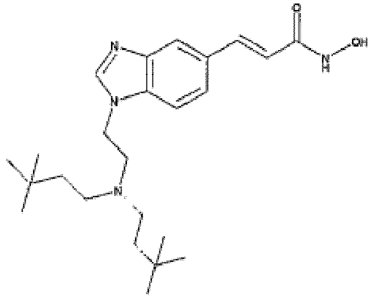 | (E)-3-(1-(2-(bis(3,3-dimethylbutyl)amino)ethyl)-1H-benzo[d]imidazol-5-yl)-N-hydroxyacrylamide |
| 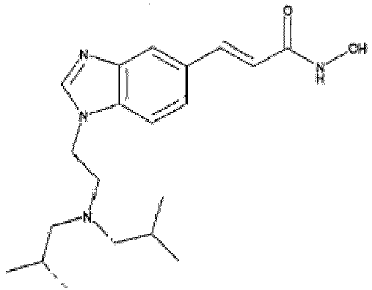 | (E)-3-(1-(2-(diisobutylamino)ethyl)-1H-benzo[d]imidazol-5-yl)-N-hydroxyacrylamide |
| 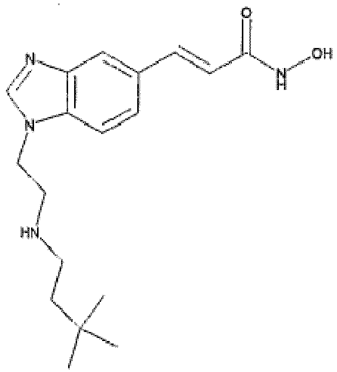 | 3-{1-[2-(3,3-Dimethyl-butylamino)-ethyl]-1H-benzimidazol-5-yl}-N-hydroxy-acrylamide |
| 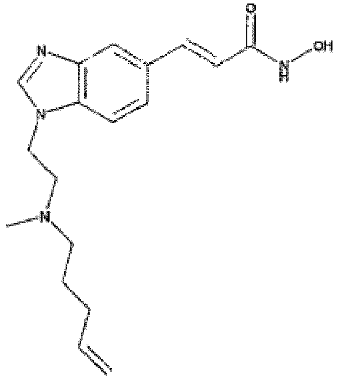 | N-Hydroxy-3-{1-[2-(methyl-pent-4-enyl-amino)-ethyl]-1H-benzimidazol-5-yl}-acrylamide |
| 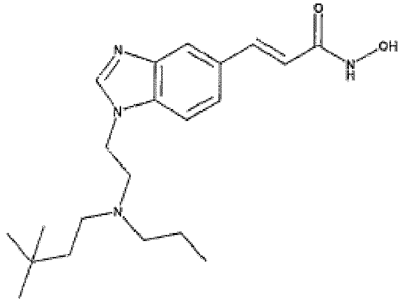 | 3-(1-{2-[(3,3-Dimethyl-butyl)-propyl-amino]-ethyl}-1H-benzimidazol-5-yl)-N-hydroxy-acrylamide |
| 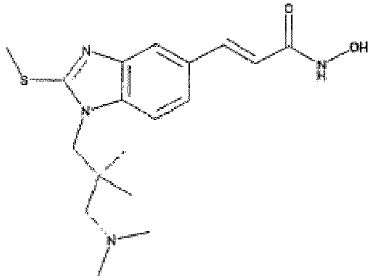 | 3-[1-(3-Dimethylamino-2,2-dimethyl-propyl)-2-methylsulfanyl-1H-benzimidazol-5-yl]-N-hydroxy-acrylamide |
| 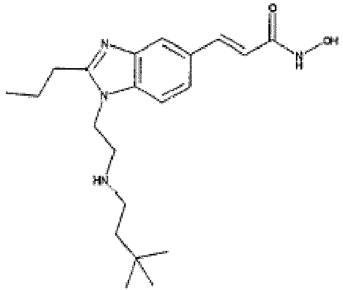 | 3-{1-[2-(3,3-Dimethyl-butylamino)-ethyl]-2-propyl-1H-benzimidazol-5-yl}-N-hydroxy-acrylamide |
| 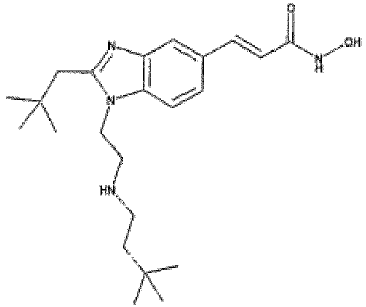 | 3-[1-[2-(3,3-Dimethyl-butylamino)-ethyl]-2-(2,2-dimethyl-propyl)-1H-benzimidazol-5-yl]-N-hydroxy-acrylamide |
| 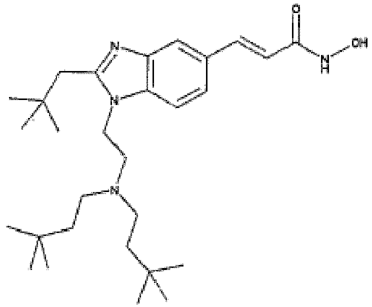 | 3-[1-{2-[Bis-(3,3-dimethyl-butyl)-amino]-ethyl)-2-(2,2-dimethyl-propyl)-1H-benzimidazol-5-yl]-N-hydroxy-acrylamide |
| 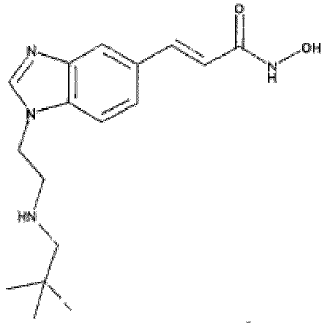 | 3-{1-[2-(2,2-Dimethyl-propylamino)-ethyl]-1H-benzimidazol-5-yl}-N-hydroxy-acrylamide |
| 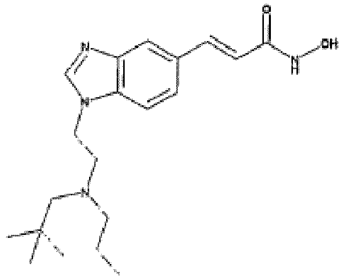 | 3-(1-{2-[(2,2-Dimethyl-propyl)-propyl-aminoethyl)-1H-benzimidazol-5-yl)-N-hydroxy-acrylamide |
| 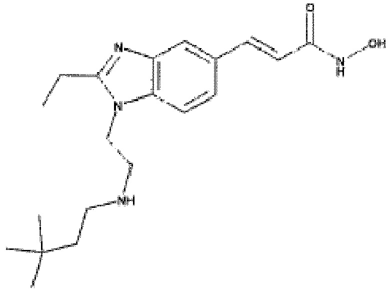 | 3-{1-[2-(3,3-Dimethyl-butylamino)-ethyl]-2-ethyl-1H-benzimidazol-5-yl)-N-hydroxy-acrylamide |
| 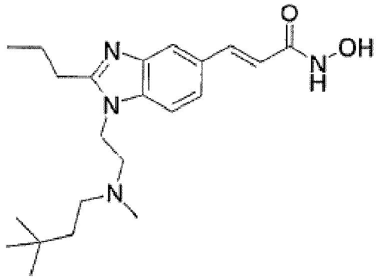 | 3-(1-{2-[(3,3-Dimethyl-butyl)-methyl-aminoethyl}-2-propyl-1H-benzimidazol-5-yl)-N-hydroxy-acrylamide |
| 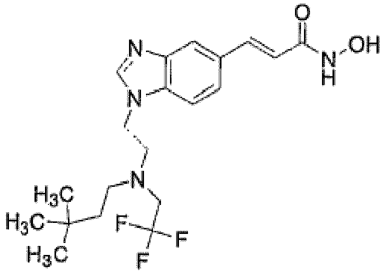 | 3-(1-{2-[(3,3-Dimethyl-butyl)-(2,2,2-trifluoroethyl)-aminoFethyl}-1H-benzimidazol-5-yl)-N-hydroxy-acrylamide |
| 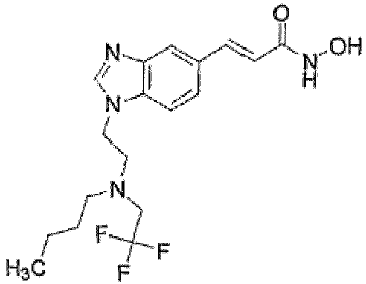 | 3-(1-{2-[Butyl-(2,2,2-trifluoro-ethyl)-amino]-ethyl}-1H-benzimidazol-5-yl)-N-hydroxy-acrylamide |

Compounds of formula (I) described herein include the disclosure found in international application No.: PCT/SG2006/000217, entitled "HETEROCYCLIC COMPOUNDS", filed on August 1, 2006, which is incorporated herein in its entirety by reference. The compounds of formula (I) and the embodiments disclosed herein inhibit histone deacetylases. In certain embodiments, the histone deacetylase inhibitor interacts with and/or reduces the activity of more than one known histone deacetylase in the cell, which can either be from the same class of histone deacetylase or different class of histone deacetylase. In some other embodiments, the histone deacetylase inhibitor interacts and reduces the activity of predominantly one histone deacetylase, for example HDAC-1, HDAC-2, HDAC-3 or HDAC-8 which belongs to Class I HDAC enzymes [De Ruijter A.J.M. et al, Biochem. J., 370, 737-749 (2003)]. In some embodiments, the compounds of formula (I) have significant anti-proliferative effects and promote differentiation, cell cycle arrest in the G1 or G2 phase, and induce apoptosis.

### DNA Hypomethylating Agents

Any suitable hypomethylating agent may be used in combination with a compound of formula (I). DNA hypomethylating agents for use in the methods provided herein include but are not limited to 5-azacytidine (azacitidine), 5-azadeoxycytidine (decitabine), SGI-110, zebularine and procaine. In certain specific embodiments, the DNA hypomethylating agent is 5-azacytidine (azacitidine).

### Methods

Provided herein are methods of treating a disease or disorder associated with dysregulation of histone deacetylase, comprising administering to a subject in need thereof an effective amount of (i) a DNA hypomethylating agent, and (ii) a compound of formula (I). In some embodiments, the DNA hypomethylating agent acts additively with a compound of formula (I). In some embodiments, the DNA hypomethylating agent acts synergistically with a compound of formula (I). In some embodiments, a compound of formula (I) is a compound of formula (Id), (Ie), or (If).

Some embodiments provided herein describe methods of treatment of a disorder caused by, associated with or accompanied by disruptions of cell proliferation and/or angiogenesis including administration of a therapeutically effective amount of a DNA hypomethylating agent and a compound of formula (I). In some embodiments, a compound of formula (I) is a compound of formula (Id), (Ie), or (If).

Also provided herein in some embodiments are agents for the treatment of a disorder caused by, associated with or accompanied by disruptions of cell proliferation and/or angiogenesis. In some embodiments, the agents are a DNA hypomethylating agent and a compound of formula (I). In some embodiments, a compound of formula (I) is a compound of formula (Id), (Ie), or (If).

Some embodiments described herein relate to the use of a DNA hypomethylating agent and a compound of formula (I) in the preparation of a medicament for the treatment of a disorder caused by, associated with or accompanied by disruptions of cell proliferation and/or angiogenesis. In one embodiment, the disorder is a proliferative disorder. In a specific embodiment, the disorder is a cancer. In some embodiments, the combination therapy of a DNA hypomethylating agent and a compound of formula (I) show low toxicity. In some embodiments, the combination therapy of a DNA hypomethylating agent and a compound of formula (I) show potent anti-proliferative activity.

Other embodiments described herein provide a method of treatment of a disorder, disease or condition that can be treated by the inhibition of histone deacetylase including administration of a therapeutically effective amount of a DNA hypomethylating agent and a compound of formula (I). In some embodiments, a compound of formula (I) is a compound of formula (Id), (Ie), or (If).

Also described herein are agents for the treatment of a disorder, disease or condition that can be treated by the inhibition of histone deacetylase. In one embodiment the agent is an anticancer agent. In some embodiments, the agents are a DNA hypomethylating agent and a compound of formula (I). In some embodiments, a compound of formula (I) is a compound of formula (Id), (Ie), or (If).

Some embodiments described herein provide a method for inhibiting cell proliferation including administration of an effective amount of a DNA hypomethylating agent and a compound according to formula (I).

Provided herein in certain embodiments is a method of treating chemoresistant cancer comprising administering to a subject in need thereof an effective amount of a DNA hypomethylating agent and a compound of formula (I). In some embodiments, the cancer is refractory, non-responsive or resistant to chemotherapy. In some embodiments, the cancer is refractory, non-responsive or resistant to haploidentical stem cell transplantation. In some embodiments, the cancer is resistant to azacitidine, decitabine, SGI-110, lenalidomide, TXA-127, or combinations thereof. In some embodiments, the cancer is resistant to azacitidine, decitabine, lenalidomide, TXA-127, or combinations thereof.

In one embodiment the disorder is selected from the group consisting of but not limited to cancer (e.g. breast cancer, colon cancer, prostate cancer, pancreatic cancer, leukemia, lymphomas, ovarian cancers, neuroblastomas, melanoma). In another embodiment the disorder is a proliferative disorder. In one embodiment the proliferative disorder is cancer. The cancer can include solid tumors or hematologic malignancies.

In some embodiments, the methods described herein are useful in treating various cancers including but not limited to bone cancers including Ewing's sarcoma, osteosarcoma, chondrosarcoma and the like, brain and CNS tumours including acoustic neuroma, neuroblastomas, glioma and other brain tumours, spinal cord tumours, breast cancers including ductal adenocarcinoma, metastatic ductal breast carcinoma, colorectal cancers, advanced colorectal adenocarcinomas, colon cancers, endocrine cancers including adenocortical carcinoma, pancreatic cancer, pituitary cancer, thyroid cancer, parathyroid cancer, thymus cancer, multiple endocrine neoplasma, gastrointestinal cancers including stomach cancer, esophageal cancer, small intestine cancer, liver cancer, extra hepatic bile duct cancer, gastrointestinal carcinoid tumour, gall bladder cancer, genitourinary cancers including testicular cancer, penile cancer, prostate cancer, gynaecological cancers including cervical cancer, ovarian cancer, vaginal cancer, uterus/endometrium cancer, vulva cancer, gestational trophoblastic cancer, fallopian tube cancer, uterine sarcoma, head and neck cancers including oral cavity cancer, lip cancer, salivary gland cancer, larynx cancer, hypopharynx cancer, orthopharynx cancer, nasal cancer, paranasal cancer, nasopharynx cancer, leukemias including childhood leukemia, acute lymphocytic leukemia, acute myeloid leukemia, chronic lymphocytic leukemia, chronic myeloid leukemia, hairy cell leukemia, acute promyelocytic leukemia, plasma cell leukemia, erythroleukemia,myelomas, haematological disorders including myelodysplastic syndromes, myeloproliferative disorders, aplastic anemia, Fanconi anemia, Waldenstroms Macroglobulinemia, lung cancers including small cell lung cancer, non-small cell lung cancer, mesothelioma, lymphomas including Hodgkin's disease, non-Hodgkin's lymphoma, cutaneous T-cell lymphoma, peripheral T-cell lymphoma, AIDS related Lymphoma, B-cell lymphoma, Burkitt's lymphoma, eye cancers including retinoblastoma, intraocular melanoma, skin cancers including melanoma, non-melanoma skin cancer, squamous cell carcinoma, merkel cell cancer, soft tissue sarcomas such as childhood soft tissue sarcoma, adult soft tissue sarcoma, Kaposi's sarcoma, urinary system cancers including kidney cancer, Wilms tumour, bladder cancer, urethral cancer, and transitional cell cancer.

In some embodiments, the disease or disorder associated with dysregulation of histone deacetylase is cancer. In some embodiments, the cancer is a hematological malignancy. In some embodiments, wherein the hematological malignancy is acute myeloid leukemia (AML), chronic myeloid leukemia (CML), chronic myelomonocytic leukemia, thrombolytic leukemia, a myelodysplastic syndrome (MDS), a myeloproliferative disorder, refractory anemia, a preleukemia syndrome, a lymphoid leukemia, lymphoma, non-Hodgkin's lymphoma, or an undifferentiated leukemia. In some specific embodiments, the cancer is myelodysplastic syndrome (MDS) or acute myeloid leukemia (AML). Non-limiting examples of non-Hodgkin's lymphoma include diffuse large B-cell lymphoma (DLBCL), mantle cell lymphoma (MCL), and chronic lymphocytic leukemia (CLL).

Other exemplary cancers that may be treated by the methods described herein include but are not limited to leukemias such as erythroleukemia, acute promyelocytic leukemia, acute myeloid leukemia, acute lymophocytic leukemia, acute T-cell leukemia and lymphoma such as B-cell lymphoma (e.g. Burkitt's lymphoma), cutaneous T-cell lymphoma (CTCL), and peripheral T-cell lymphoma.

Certain exemplary cancers that may be treated by the methods described herein include solid tumors and hematologic malignancies. In another embodiment, preferred cancers that may be treated with the compounds of the present invention are colon cancer, prostate cancer, hepatoma and ovarian cancer.

### Doses

The amount of a DNA hypomethylating agent and a compound of formula (I) will be dependent on the subject treated. In some instances where the subject is a human, the dose will normally be determined by the prescribing physician with the dosage generally varying according to the age, sex, diet, weight, general health and response of the individual patient, the severity of the patient's symptoms, the precise indication or condition being treated, the severity of the indication or condition being treated, time of administration, route of administration, the disposition of the composition, rate of excretion, and the discretion of the prescribing physician. In some embodiments, the total dosage for the day is divided and administered in portions during the day if desired. In some embodiments, combinational applications in which the combination therapy described herein is not the sole therapy, allows for the administration of lesser amounts of a DNA hypomethylating agent and a compound of formula (I).

In some embodiments, the dosage of a compound of formula (I) ranges from about 0.01 to 300 mg per kilogram of body weight per day. In other embodiments, the dosage of a compound of formula (I) ranges from 0.1 to 100 mg per kilogram of body weight per day, from 0.2 to 80 mg per kilogram of body weight per day, and from 0.2 to 50 mg per kilogram of body weight per day.

In specific embodiments, an effective amount of a compound of formula (I) is from about 5 mg to about 1000 mg, from about 5 mg to about 120 mg, from about 10 mg to about 1000 mg, from about 10 mg to about 900 mg, from about 10 mg to about 800 mg, from about 10 mg to about 700 mg, from about 10 mg to about 600 mg, from about 10 mg to about 500 mg, from about 10 mg to about 400 mg, from about 10 mg to about 300 mg, from about 10 mg to about 250 mg, from about 10 mg to about 200 mg, from about 10 mg to about 150 mg, from about 10 mg to about 125 mg, from about 10 mg to about 120 mg, from about 10 mg to about 100 mg, from about 10 mg to about 90 mg, from about 10 mg to about 80 mg, from about 10 mg to about 70 mg, from about 10 mg to about 60 mg, from about 10 mg to about 50 mg, from about 10 mg to about 40 mg, from about 10 mg to about 30 mg, from about 30 mg to about 1000 mg, from about 30 mg to about 500 mg, from about 30 mg to about 400 mg, from about 30 mg to about 300 mg, from about 30 mg to about 200 mg, from about 30 mg to about 250 mg, from about 30 mg to about 225 mg, from about 30 mg to about 200 mg, from about 30 mg to about 175 mg, from about 30 mg to about 150 mg, from about 30 mg to about 125 mg, from about 30 mg to about 120 mg, from about 30 mg to about 115 mg, from about 30 mg to about 110 mg, from about 30 mg to about 100 mg, from about 30 mg to about 90 mg, from about 30 mg to about 80 mg, from about 30 mg to about 70 mg, from about 30 mg to about 60 mg, from about 30 mg to about 50 mg, from about 40 mg to about 100 mg, from about 40 mg to about 80 mg, from about 50 mg to about 70 mg, from about 60 mg to about 500 mg, from about 60 mg to about 250 mg, from about 60 mg to about 200 mg, from about 60 mg to about 150 mg, from about 30 mg to about 125 mg, from about 30 mg to about 120 mg, from about 100 mg to about 500 mg, from about 100 mg to about 400 mg, from about 100 mg to about 300 mg, from about 100 mg to about 200 mg, less than 1000 mg, less than 900 mg, less than 800 mg, less than 700 mg, less than 600 mg, less than 500 mg, less than 400 mg, less than 300 mg, less than 275 mg, less than 250 mg, less than 225 mg, less than 200 mg, less than 175 mg, less than 150 mg, less than 125 mg, less than 100 mg, less than 90 mg, less than 80 mg, less than 70 mg, less than 65 mg, less than 60 mg, less than 55 mg, less than 50 mg, less than 40 mg, less than 30 mg, less than 20 mg, less than 10 mg, about 1000 mg, about 950 mg, about 900 mg, about 850 mg, about 800 mg, about 750 mg, about 700 mg, about 650 mg, about 600 mg, about 550 mg, about 500 mg, about 450 mg, about 400 mg, about 350 mg, about 300 mg, about 250 mg, about 200 mg, about 190 mg, about 180 mg, about 170 mg, about 160 mg, about 150 mg, about 140 mg, about 130 mg, about 120 mg, about 110 mg, about 100 mg, about 95 mg, about 90 mg, about 85 mg, about 80 mg, about 75 mg, about 70 mg, about 65 mg, about 60 mg, about 55 mg, about 50 mg, about 45 mg, about 40 mg, about 35 mg, about 30 mg, about 25 mg, about 20 mg, about 15 mg, about 10 mg, about 8 mg, about 5 mg, about 2 mg, or about 1 mg. In certain specific embodiment, an effective amount of a compound of formula (I) administered according to any of the methods described herein is about 60 mg.

In specific embodiments, an effective amount of DNA hypomethylating agent is from about 5 mg/m² to about 1000 mg/m², from about 5 mg/m² to about 125 mg/m², from about 10 mg/m² to about 1000 mg/m², from about 10 mg/m² to about 800 mg/m², from about 10 mg/m² to about 700 mg/m², from about 10 mg/m² to about 600 mg/m², from about 10 mg/m² to about 500 mg/m², from about 10 mg/m² to about 400 mg/m², from about 10 mg/m² to about 350 mg/m², from about 10 mg/m² to about 300 mg/m², from about 10 mg/m² to about 200 mg/m², from about 10 mg/m² to about 175 mg/m², from about 10 mg/m² to about 150 mg/m², from about 10 mg/m² to about 125 mg/m², from about 10 mg/m² to about 115 mg/m², from about 10 mg/m² to about 100 mg/m², from about 10 mg/m² to about 80 mg/m², from about 10 mg/m² to about 60 mg/m², from about 10 mg/m² to about 20 mg/m², from about 5 mg/m² to about 20 mg/m², from about 50 mg/m² to about 500 mg/m², from about 50 mg/m² to about 400 mg/m², from about 10 mg/m² to about 350 mg/m², from about 50 mg/m² to about 300 mg/m², from about 50 mg/m² to about 250 mg/m², from about 50 mg/m² to about 225 mg/m², from about 50 mg/m² to about 200 mg/m², from about 50 mg/m² to about 175 mg/m², from about 50 mg/m² to about 150 mg/m², from about 50 mg/m² to about 125 mg/m², from about 50 mg/m² to about 100 mg/m², from about 50 mg/m² to about 90 mg/m², from about 50 mg/m² to about 80 mg/m², from about 60 mg/m² to about 80 mg/m², from about 75 mg/m² to about 250 mg/m², from about 75 mg/m² to about 200 mg/m², from about 75 mg/m² to about 150 mg/m², from about 75 mg/m² to about 125 mg/m², less than 1000 mg/m², less than 900 mg/m², less than 800 mg/m², less than 700 mg/m², less than 600 mg/m², less than 500 mg/m², less than 400 mg/m², less than 350 mg/m², less than 300 mg/m², less than 275 mg/m², less than 250 mg/m², less than 225 mg/m², less than 200 mg/m², less than 175 mg/m², less than 150 mg/m², less than 125 mg/m², less than 115 mg/m², less than 100 mg/m², less than 90 mg/m², less than 80 mg/m², less than 70 mg/m², less than 60 mg/m², less than 50 mg/m², less than 40 mg/m², less than 30 mg/m², less than 20 mg/m², less than 10 mg/m², about 1000 mg/m², about 900 mg/m², about 800 mg/m², about 700 mg/m², about 600 mg/m², about 500 mg/m², about 400 mg/m², about 350 mg/m², about 300 mg/m², about 250 mg/m², about 225 mg/m², about 200 mg/m², about 175 mg/m², about 150 mg/m², about 140 mg/m², about 130 mg/m², about 125 mg/m², about 115 mg/m², about 100 mg/m², about 90 mg/m², about 95 mg/m², about 90 mg/m², about 85 mg/m², about 80 mg/m², about 75 mg/m², about 70 mg/m², about 65 mg/m², about 60 mg/m², about 50 mg/m², about 55 mg/m², about 45 mg/m², about 40 mg/m², about 35 mg/m², about 30 mg/m², about 25 mg/m², about 20 mg/m², about 15 mg/m², about 11 mg/m², about 10 mg/m², about 5 mg/m², or about 2 mg/m². In certain specific embodiment, an effective amount of a DNA hypomethylating agent administered according to any of the methods described herein is about 75 mg/m².

### Administration

In some embodiments, a compound of formula (I) and a DNA hypomethylating agent are administered to a subject (e.g., a human) by any acceptable modes for enteral administration such as oral or rectal, or by parenteral administration such as subcutaneous, intramuscular, intravenous and intradermal routes. In some embodiments, injection is bolus or via constant or intermittent infusion. In various embodiments, the combination of a compound of formula (I) and a DNA hypomethylating agent is selectively toxic or more toxic to rapidly proliferating cells, e.g. cancerous tumors, than to normal cells.

The compounds of the present invention can be administered alone or in the form of a pharmaceutical composition in combination with a pharmaceutically acceptable carrier, diluent or excipient. The compounds of the invention, while effective themselves, are typically formulated and administered in the form of their pharmaceutically acceptable salts as these forms are typically more stable, more easily crystallised and have increased solubility.

In some embodiments, a compound of formula (I) and a DNA hypomethylating agent are used in the form of pharmaceutical compositions which are formulated depending on the desired mode of administration. In some embodiments, a pharmaceutical composition includes a compound of formula (I) and a pharmaceutically acceptable carrier, diluent or excipient. In other embodiments, a pharmaceutical composition includes a DNA hypomethylating agent and a pharmaceutically acceptable carrier, diluent or excipient. In certain embodiments, a pharmaceutical composition includes a compound of formula (I), a DNA hypomethylating agent, and at least one pharmaceutically acceptable carrier, diluents or excipient. In certain specific embodiments, the compound of formula (I) is 3-[2-butyl-1-(2-diethylamino-ethyl)-1H-benzimidazol-5-yl]-N-hydroxy-acrylamide. In certain specific embodiments, the DNA hypomethylating agent is 5-azacitidine. In certain specific embodiments, the DNA hypomethylating agent is 5-azadeoxycytidine.

Some embodiments provided herein describe pharmaceutical compositions for parenteral injection comprising pharmaceutically acceptable sterile aqueous or nonaqueous solutions, dispersions, suspensions or emulsions as well as sterile powders for reconstitution into sterile injectable solutions or dispersions just prior to use. Non-limiting examples of suitable aqueous and nonaqueous carriers, diluents, solvents or vehicles include water, ethanol, polyols (such as glycerol, propylene glycol, polyethylene glycol, and the like), and suitable mixtures thereof, vegetable oils (such as olive oil), and injectable organic esters such as ethyl oleate. In some embodiments, proper fluidity is maintained, for example, by the use of coating materials such as lecithin, by the maintenance of the required particle size in the case of dispersions, and by the use of surfactants.

In some embodiments, provided herein are compositions containing adjuvants such as preservative, wetting agents, emulsifying agents, and dispersing agents. In some embodiments, various antibacterial and antifungal agents, for example, paraben, chlorobutanol, phenol sorbic acid, and the like are included to prevent the action of microorganisms. In some embodiments, the pharmaceutical composition includes isotonic agents such as sugars, sodium chloride, and the like. In some embodiments, prolonged absorption of the injectable pharmaceutical form is brought about by the inclusion of agents that delay absorption such as aluminium monostearate and gelatin.

In some embodiments, for more effective distribution, the active agents are incorporated into slow release or targeted delivery systems such as polymer matrices, liposomes, and microspheres.

In certain embodiments, the injectable formulations is sterilized, for example, by filtration through a bacterial-retaining filter, or by incorporating sterilizing agents in the form of sterile solid compositions that are dissolved or dispersed in sterile water or other sterile injectable medium just prior to use.

Solid dosage forms for oral administration include capsules, tablets, pills, powders, and granules. In such solid dosage forms, the active compound is mixed with at least one inert, pharmaceutically acceptable excipient or carrier such as sodium citrate or dicalcium phosphate and/or a) fillers or extenders such as starches, lactose, sucrose, glucose, mannitol, and silicic acid, b) binders such as, for example, carboxymethylcellulose, alginates, gelatin, polyvinylpyrrolidone, sucrose, and acacia, c) humectants such as glycerol, d) disintegrating agents such as agar-agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. In the case of capsules, tablets and pills, the dosage form may also comprise buffering agents.

In some embodiments, solid compositions comprise fillers in soft and hard-filled gelatin capsules using such excipients as lactose or milk sugar as well as high molecular weight polyethylene glycols and the like.

In some instances, the solid dosage forms of tablets, dragees, capsules, pills, and granules are prepared with coatings and shells such as enteric coatings and other coatings well known in the pharmaceutical formulating art. In some embodiments, the solid dosage forms optionally contain opacifying agents and release the active ingredient(s) only, or preferentially, in a certain part of the intestinal tract, optionally, in a delayed manner. Examples of embedding compositions which can be used include polymeric substances and waxes.

In some embodiments, the compounds are incorporated into slow release or targeted delivery systems such as polymer matrices, liposomes, and microspheres.

In some instances, the active compounds are in microencapsulated form, if appropriate, with one or more of the above-mentioned excipients.

Liquid dosage forms for oral administration include pharmaceutically acceptable emulsions, solutions, suspensions, syrups and elixirs. In some instances, the liquid dosage forms contains inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethyl formamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof.

In some instances, the oral compositions also include adjuvants such as wetting agents, emulsifying and suspending agents, sweetening, flavoring, and perfuming agents.

In some embodiments, any compound of formula (I) is administered intravenously, subcutaneously, or orally. In certain embodiments, a compound of formula (I) is administered orally. In certain specific embodiments, 3-[2-butyl-1-(2-diethylamino-ethyl)-1H-benzimidazol-5-yl]-N-hydroxy-acrylamide is administered orally. In certain specific embodiments, 3-[2-butyl-1-(2-diethylamino-ethyl)-1H-benzimidazol-5-yl]-N-hydroxy-acrylamide is administered intravenously.

In some embodiments, a DNA hypomethylating agent is administered intravenously, subcutaneously, or orally. In certain embodiments, a DNA hypomethylating agent is administered intravenously. In other embodiments, a DNA hypomethylating agent is administered subcutaneously. In certain specific embodiments, 5-azacitidine is administered intravenously. In other specific embodiments, 5-azacitidine is administered subcutaneously. In certain specific embodiments, 5-azadeoxycytidine is administered intravenously. In other specific embodiments, 5-azadeoxycytidine is administered subcutaneously.

Some embodiments provided herein describe a combination therapy comprising a compound of formula (I) and a DNA hypomethylating agent, wherein the compound of formula (I) and the DNA hypomethylating agent are administered in combination with each other. In some instances, the compound of formula (I) and the DNA hypomethylating agent are administered simultaneously. In other instances, the compound of formula (I) and the DNA hypomethylating agent are administered sequentially. In other instances, the compound of formula (I) and the DNA hypomethylating agent are administered within the same week.

In some embodiments, the compound of formula (I) is administered daily, every other day, every other day 3 times a week, every 3 days, every 4 days, every 5 days, every 6 days, weekly, bi-weekly, 3 times a week, 4 times a week, 5 times a week, 6 times a week, once a month, twice a month, 3 times a month, once every 2 months, once every 3 months, once every 4 months, once every 5 months, or once every 6 months. In some embodiments, the DNA hypomethylating agent is administered daily, every other day, every other day 3 times a week, every 3 days, every 4 days, every 5 days, every 6 days, weekly, bi-weekly, 3 times a week, 4 times a week, 5 times a week, 6 times a week, once a month, twice a month, 3 times a month, once every 2 months, once every 3 months, once every 4 months, once every 5 months, or once every 6 months.

In some instances, the compound of formula (I) or the DNA hypomethylating agent is optionally given continuously; alternatively, the dose of drug being administered is temporarily reduced or temporarily suspended for a certain length of time (i.e., a "drug holiday"). The length of the drug holiday optionally varies between 2 days and 1 year, including by way of example only, 2 days, 3 days, 4 days, 5 days, 6 days, 7 days, 8 days, 9 days, 10 days, 12 days, 15 days, 20 days, 28 days, 35 days, 50 days, 70 days, 100 days, 120 days, 150 days, 180 days, 200 days, 250 days, 280 days, 300 days, 320 days, 350 days, or 365 days. The dose reduction during a drug holiday includes from 10%-100%, including, by way of example only, 10%, 15%, 20%, 25%, 30%, 35%, 40%, 45%, 50%, 55%, 60%, 65%, 70%, 75%, 80%, 85%, 90%, 95%, or 100%.

Some embodiments provided herein describe a combination therapy that is used or administered in combination with one or more additional drug (s) that are chemotherapeutic drugs or HDAC inhibitor drugs and/or procedures (e.g. surgery, radiotherapy) for the treatment of the disorder/diseases mentioned. In some embodiments, the additional drug(s) are administered in the same formulation or in separate formulations. In some embodiments, if administered in separate formulations, the combination therapy is administered sequentially or simultaneously (as a combined preparation) with the additional drug(s).

### Kits

Some embodiments provided herein describe a pharmaceutical pack or kit comprising one or more containers filled with a compound of formula (I) and one or more containers filled with a DNA hypomethylating agent. In some embodiments, the kit comprises one container filled with a compound of formula (I) and a DNA hypomethylating agent. In some embodiments, the kit comprises a container having a unit dosage of the agent(s). In certain embodiments, the kits include one or more compositions comprising a compound of formula (I) and a DNA hypomethylating agent (including lyophilized compositions), which can be diluted further prior to use or they can be provided at the concentration of use, where the vials may include one or more dosages. Conveniently, in the kits, single dosages can be provided in sterile vials so that the physician can employ the vials directly, where the vials will have the desired amount and concentration of agent(s). Associated with such container(s) can be various written materials such as instructions for use, or a notice in the form prescribed by a governmental agency regulating the manufacture, use or sale of pharmaceuticals or biological products, which notice reflects approval by the agency of manufacture, use or sale for human administration.

In some embodiments, the kit comprises a container filled with 3-[2-butyl-1-(2-diethylamino-ethyl)-1H-benzimidazol-5-yl]-N-hydroxy-acrylamide and 5-azacitidine. In other embodiments, the kit comprises a container filled with 3-[2-butyl-1-(2-diethylamino-ethyl)-1H-benzimidazol-5-yl]-N-hydroxy-acrylamide and 5-azadeoxycytidine. In some embodiments, the kit comprises one or more containers filled with 3-[2-butyl-1-(2-diethylamino-ethyl)-1H-benzimidazol-5-yl]-N-hydroxy-acrylamide and one or more containers filled with 5-azacitidine. In other embodiments, the kit comprises one or more containers filled with 3-[2-butyl-1-(2-diethylamino-ethyl)-1H-benzimidazol-5-yl]-N-hydroxy-acrylamide and one or more containers filled with 5-azadeoxycytidine.

### EXAMPLES

### Example 1. Treatment of Hematological Malignancies

### Human Clinical Trial of the Safety and Efficacy of Combination Therapy for Hematological Malignancies

Study Design: This pilot phase II study was conducted as an extension study in the context of a phase I trial of 3-[2-butyl-1-(2-diethylamino-ethyl)-1H-benzimidazol-5-yl]-N-hydroxy-acrylamide in hematological malignancies to determine the efficacy and safety of the combination of 3 -[2-butyl-1 -(2-diethylamino-ethyl)-1 H-benzimidazol-5 -yl] -N-hydroxy-acrylamide (60 mg orally every other day 3 times a week for 3 consecutive weeks) and 5-azacitidine (75 mg/m² IV daily x 5 every 3 to 6 weeks) given in 4-week cycles to patients with intermediate-2 or high risk MDS.

Nine patients (6 women) were accrued between May 2011 and September 2011. The median age of the patients was 64 years (range, 22-73), WBC 2.4x10⁹/dL (0.7-9.3), Hg 10g/dL (8.2-11), platelets 31x10⁹/dL (14-269), and bone marrow blasts 7% (0%-18%). Seven (78%) patients had therapy-related myelodysplastic syndrome (MDS) with a history of prior chemotherapy/radiotherapy exposure (3 breast cancer, 2 non-Hodgkin's lymphoma, 1 breast and ovarian cancer, and 1 melanoma). Three patients had failed prior therapy: decitabine and haploidentical stem cell transplantation (SCT; n=1), lenalidomide (n=1), and decitabine and TXA-127 (n=1). All patients carried cytogenetic abnormalities: complex (n=4, 3 including -7 and 1 with -5), -7 (n=3, one of them with +8), t(6;9) (n=1), and t(14;16) and del(20) (n=1). Two patients with -7 also carried gene mutations: 1 in CEBPa and 1 IDH2^{R140Q}. Patients received a median of 4 cycles.

Results: All 9 patients were evaluable. The overall response rate (ORR; defined as rate of complete response (CR) + CR with incomplete platelet recovery (Cri) + rate of partial response (PR)) is 8/9 (89%) and the CR + CRi rate is 7/9 (78%). Five (56%) patients achieved a complete cytogenetic response, including the patient carrying IDH2R140Q, in whom such mutation became undetectable. Eight-week mortality was 0%. Only 1 (11%) patient died, unrelated to study drug (after allogeneic-SCT). The median duration of response was 45 days (0-229). Reasons for discontinuation were: transition to allogeneic-SCT (n=5), no availability of 3-[2-butyl-1-(2-diethylamino-ethyl)-1H-benzimidazol-5-yl]-N-hydroxy-acrylamide by sponsor (n=2), no response (n=1), and progression to AML (n=1). The combination was well tolerated. All toxicities were grade 1 or 2. The most frequent toxicities were fatigue and nausea (56% each).

The combination of 3-[2-butyl-1-(2-diethylamino-ethyl)-1H-benzimidazol-5-yl]-N-hydroxy-acrylamide and 5-azacitidine was very well tolerated in patients with MDS. The preliminary ORR of 89% is very encouraging, considering that most patients in this study had high-risk cytogenetics and/or had treatment related MDS, both subsets of MDS with very poor prognosis.

### Example 2. Combination Treatment for Acute Myeloid Leukemia

### Human Clinical Trial of the Safety and Efficacy of 3-[2-Butyl-1-(2-diethylaminoethyl)-1H-benzimidazol-5-yl]-N-hydroxy-acrylamide and 5-Azacitidine for Treatment of Acute Myeloid Leukemia (AML)

Study Design: This study will be a Phase II study in acute myeloid leukemia patients. Patients must not have received treatment for their cancer within 2 weeks of beginning the trial. Treatments include the use of chemotherapy, hematopoietic growth factors, and biologic therapy such as monoclonal antibodies. Patients must have recovered from all toxicities (to grade 0 or 1) associated with previous treatment. All subjects are evaluated for safety and all blood collections for pharmacokinetic analysis are collected as scheduled. All studies are performed with institutional ethics committee approval and patient consent.

Phase II: Patients receive 3-[2-butyl-1-(2-diethylamino-ethyl)-1H-benzimidazol-5-yl]-N-hydroxy-acrylamide (orally every other day 3 times a week for 4 consecutive weeks) and 5-azacitidine (intravenously daily x 5 every 4 weeks). Treatment repeats every 4 weeks for 2-6 courses in the absence of disease progression or unacceptable toxicity. After completion of 2 courses of study therapy, patients who achieve a complete or partial response may receive an additional 4 courses. Patients who maintain stable disease for more than 2 months after completion of 6 courses of study therapy may receive an additional 6 courses at the time of disease progression, provided they meet original eligibility criteria.

Blood Sampling Serial blood is drawn by direct vein puncture before and after administration of compound 31. Venous blood samples (5 mL) for determination of serum concentrations are obtained at about 10 minutes prior to dosing and at approximately the following times after dosing: days 1, 8, 15, and 22. Each serum sample is divided into two aliquots. All serum samples are stored at -20°C. Serum samples are shipped on dry ice.

Pharmacokinetics: Patients undergo plasma/serum sample collection for pharmacokinetic evaluation before beginning treatment and at days 1, 8, 15, and 22. Pharmacokinetic parameters are calculated by model independent methods on a Digital Equipment Corporation VAX 8600 computer system using the latest version of the BIOAVL software. The following pharmacokinetics parameters are determined: peak serum concentration (Cₘₐₓ); time to peak serum concentration (tₘₐₓ); area under the concentration-time curve (AUC) from time zero to the last blood sampling time (AUC₀₋₇₂) calculated with the use of the linear trapezoidal rule; and terminal elimination half-life (t_{1/2}), computed from the elimination rate constant. The elimination rate constant is estimated by linear regression of consecutive data points in the terminal linear region of the log-linear concentration-time plot. The mean, standard deviation (SD), and coefficient of variation (CV) of the pharmacokinetic parameters are calculated for each treatment. The ratio of the parameter means (preserved formulation/non-preserved formulation) is calculated.

Patient Response to combination therapy: Patient response is assessed via imaging with X-ray, CT scans, MRI, and imaging is performed prior to beginning the study and at the end of the first cycle, with additional imaging performed every four weeks or at the end of subsequent cycles. Imaging modalities are chosen based upon the cancer type and feasibility/availability, and the same imaging modality is utilized for similar cancer types as well as throughout each patient's study course. Response rates are determined using the RECIST criteria. (Therasse et al, J. Natl. Cancer Inst. 2000 Feb 2; 92(3):205-16; http://ctep.cancer.gov/forms/TherasseRECISTJNCI.pdf). Patients also undergo cancer/tumor biopsy to assess changes in progenitor cancer cell phenotype and clonogenic growth by flow cytometry, Western blotting, and IHC, and for changes in cytogenetics by FISH. After completion of study treatment, patients are followed periodically for 4 weeks.

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that methods and structures within the scope of these claims and their equivalents be covered thereby.

### Example 3. Combination Treatment for Acute Myeloid Leukemia

### Human Clinical Trial of the Safety and Efficacy of 3-[2-Butyl-1-(2-diethylaminoethyl)-1H-benzimidazol-5-yl]-N-hydroxy-acrylamide and 5-azadeoxycytidine for Treatment of Acute Myeloid Leukemia (AML)

Study Design: This study will be a Phase II study in acute myeloid leukemia patients. Patients must not have received treatment for their cancer within 2 weeks of beginning the trial. Treatments include the use of chemotherapy, hematopoietic growth factors, and biologic therapy such as monoclonal antibodies. Patients must have recovered from all toxicities (to grade 0 or 1) associated with previous treatment. All subjects are evaluated for safety and all blood collections for pharmacokinetic analysis are collected as scheduled. All studies are performed with institutional ethics committee approval and patient consent.

Phase II: Patients receive 3-[2-butyl-1-(2-diethylamino-ethyl)-1H-benzimidazol-5-yl]-N-hydroxy-acrylamide (orally every other day 3 times a week for 4 consecutive weeks) and 5-azadeoxycytidine (intravenously daily x 5 every 4 weeks). Treatment repeats every 4 weeks for 2-6 courses in the absence of disease progression or unacceptable toxicity. After completion of 2 courses of study therapy, patients who achieve a complete or partial response may receive an additional 4 courses. Patients who maintain stable disease for more than 2 months after completion of 6 courses of study therapy may receive an additional 6 courses at the time of disease progression, provided they meet original eligibility criteria.

Blood Sampling Serial blood is drawn by direct vein puncture before and after administration of compound 31. Venous blood samples (5 mL) for determination of serum concentrations are obtained at about 10 minutes prior to dosing and at approximately the following times after dosing: days 1, 8, 15, and 22. Each serum sample is divided into two aliquots. All serum samples are stored at -20°C. Serum samples are shipped on dry ice.

Pharmacokinetics: Patients undergo plasma/serum sample collection for pharmacokinetic evaluation before beginning treatment and at days 1, 8, 15, and 22. Pharmacokinetic parameters are calculated by model independent methods on a Digital Equipment Corporation VAX 8600 computer system using the latest version of the BIOAVL software. The following pharmacokinetics parameters are determined: peak serum concentration (Cₘₐₓ); time to peak serum concentration (tₘₐₓ); area under the concentration-time curve (AUC) from time zero to the last blood sampling time (AUC₀₋₇₂) calculated with the use of the linear trapezoidal rule; and terminal elimination half-life (t_{1/2}), computed from the elimination rate constant. The elimination rate constant is estimated by linear regression of consecutive data points in the terminal linear region of the log-linear concentration-time plot. The mean, standard deviation (SD), and coefficient of variation (CV) of the pharmacokinetic parameters are calculated for each treatment. The ratio of the parameter means (preserved formulation/non-preserved formulation) is calculated.

Patient Response to combination therapy: Patient response is assessed via imaging with X-ray, CT scans, MRI, and imaging is performed prior to beginning the study and at the end of the first cycle, with additional imaging performed every four weeks or at the end of subsequent cycles. Imaging modalities are chosen based upon the cancer type and feasibility/availability, and the same imaging modality is utilized for similar cancer types as well as throughout each patient's study course. Response rates are determined using the RECIST criteria. (Therasse et al, J. Natl. Cancer Inst. 2000 Feb 2; 92(3):205-16; http://ctep.cancer.gov/forms/TherasseRECISTJNCI.pdf). Patients also undergo cancer/tumor biopsy to assess changes in progenitor cancer cell phenotype and clonogenic growth by flow cytometry, Western blotting, and IHC, and for changes in cytogenetics by FISH. After completion of study treatment, patients are followed periodically for 4 weeks.

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that methods and structures within the scope of these claims and their equivalents be covered thereby.

### Example 4. Combination Treatment for Myelodysplastic Syndrome (MDS)

### Human Clinical Trial of the Safety and Efficacy of 3-[2-Butyl-1-(2-diethylaminoethyl)-1H-benzimidazol-5-yl]-N-hydroxy-acrylamide and 5-azadeoxycytidine for Treatment of myelodysplastic syndrome (MDS)

Study Design: This study will be a Phase II study in myelodysplastic syndrome patients. Patients must not have received treatment for their cancer within 2 weeks of beginning the trial. Treatments include the use of chemotherapy, hematopoietic growth factors, and biologic therapy such as monoclonal antibodies. Patients must have recovered from all toxicities (to grade 0 or 1) associated with previous treatment. All subjects are evaluated for safety and all blood collections for pharmacokinetic analysis are collected as scheduled. All studies are performed with institutional ethics committee approval and patient consent.

Phase II: Patients receive 3-[2-butyl-1-(2-diethylamino-ethyl)-1H-benzimidazol-5-yl]-N-hydroxy-acrylamide (orally every other day 3 times a week for 4 consecutive weeks) and 5-azadeoxycytidine (intravenously daily x 5 every 4 weeks). Treatment repeats every 4 weeks for 2-6 courses in the absence of disease progression or unacceptable toxicity. After completion of 2 courses of study therapy, patients who achieve a complete or partial response may receive an additional 4 courses. Patients who maintain stable disease for more than 2 months after completion of 6 courses of study therapy may receive an additional 6 courses at the time of disease progression, provided they meet original eligibility criteria.

Blood Sampling Serial blood is drawn by direct vein puncture before and after administration of compound 31. Venous blood samples (5 mL) for determination of serum concentrations are obtained at about 10 minutes prior to dosing and at approximately the following times after dosing: days 1, 8, 15, and 22. Each serum sample is divided into two aliquots. All serum samples are stored at -20°C. Serum samples are shipped on dry ice.

Pharmacokinetics: Patients undergo plasma/serum sample collection for pharmacokinetic evaluation before beginning treatment and at days 1, 8, 15, and 22. Pharmacokinetic parameters are calculated by model independent methods on a Digital Equipment Corporation VAX 8600 computer system using the latest version of the BIOAVL software. The following pharmacokinetics parameters are determined: peak serum concentration (Cₘₐₓ); time to peak serum concentration (tₘₐₓ); area under the concentration-time curve (AUC) from time zero to the last blood sampling time (AUC₀₋₇₂) calculated with the use of the linear trapezoidal rule; and terminal elimination half-life (t_{1/2}), computed from the elimination rate constant. The elimination rate constant is estimated by linear regression of consecutive data points in the terminal linear region of the log-linear concentration-time plot. The mean, standard deviation (SD), and coefficient of variation (CV) of the pharmacokinetic parameters are calculated for each treatment. The ratio of the parameter means (preserved formulation/non-preserved formulation) is calculated.

Patient Response to combination therapy: Patient response is assessed via imaging with X-ray, CT scans, MRI, and imaging is performed prior to beginning the study and at the end of the first cycle, with additional imaging performed every four weeks or at the end of subsequent cycles. Imaging modalities are chosen based upon the cancer type and feasibility/availability, and the same imaging modality is utilized for similar cancer types as well as throughout each patient's study course. Response rates are determined using the RECIST criteria. (Therasse et al, J. Natl. Cancer Inst. 2000 Feb 2; 92(3):205-16; http://ctep.cancer.gov/forms/TherasseRECISTJNCI.pdf). Patients also undergo cancer/tumor biopsy to assess changes in progenitor cancer cell phenotype and clonogenic growth by flow cytometry, Western blotting, and IHC, and for changes in cytogenetics by FISH. After completion of study treatment, patients are followed periodically for 4 weeks.

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that methods and structures within the scope of these claims and their equivalents be covered thereby.

This invention includes the following aspects:
1. A method of treating a disease or disorder associated with dysregulation of histone deacetylase, comprising administering to a subject in need thereof an effective amount of
   (i) a DNA hypomethylating agent; and
   (ii) a compound of formula (Id): 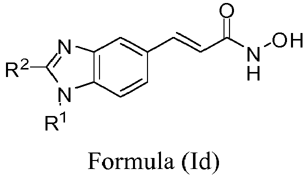 wherein
      R¹ is a group having the formula:

         -(CR²⁰R²¹)*ₘ*-(CR²²R²³)*ₙ*-(CR²⁴R²⁵)*ₒ*-NR²⁶R²⁷;
      R² is alkyl, fluoroalkyl, cyano, C₂-C₆alkenyl, C₂-C₆alkynyl, or heteroalkyl optionally substituted with =O;
      each R²⁰, R²¹, R²², R²³, R²⁴, and R²⁵ is independently H or methyl;
      each R²⁶ and R²⁷ is independently H, hydroxylalkyl, or alkyl; and
      m, n, and o are independently integers of 0, 1, 2, 3, or 4;
      or a pharmaceutically acceptable salt or prodrug thereof.
2. The method of paragraph 1, wherein the compound of formula (I) has the structure: 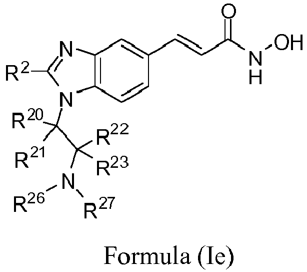 or 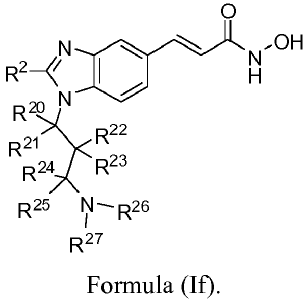
3. The method of either of paragraphs 1 or 2, wherein R²⁶ and R²⁷ are independently H or alkyl.
4. The method of any one of paragraphs 1-3, wherein R²⁶ and R²⁷ are independently H, methyl, ethyl, isopropyl, propyl, butyl, isobutyl, pentyl, hexyl or heptyl.
5. The method of any one of paragraphs 1-4, wherein R¹ has the structure 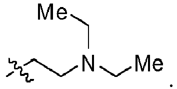
6. The method of any one of paragraphs 1-5, wherein R² is ethyl, 1-methyl-ethyl, 2,2,2-trifluoroethyl, propyl, 2-methyl-propyl, 2,2-dimethyl-propyl, 3,3,3-trifluoro-propyl, butyl, 3,3-dimethyl-butyl, pentyl, 2,4,4-trimethyl-pentyl, hexyl or octyl.
7. The method of any one of paragraphs 1-6, wherein R² is butyl.
8. The method of any one of paragraphs 1-7, wherein the compound of formula (I) has the structure: 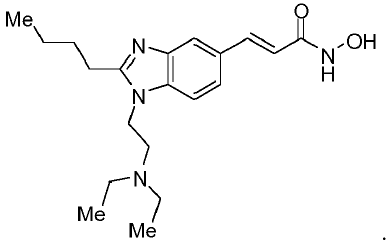
9. The method of any one of paragraphs 1-8, wherein the DNA hypomethylating agent is 5-azacytidine (azacitidine), 5-azadeoxycytidine (decitabine), SGI-110, zebularine or procaine.
10. The method of any one of paragraphs 1-9, wherein the DNA hypomethylating agent is 5-azacytidine (azacitidine).
11. The method of any one of paragraphs 1-10, wherein the disease or disorder is cancer.
12. The method of paragraph 11, wherein the cancer is a hematological malignancy.
13. The method of paragraph 12, wherein the hematological malignancy is acute myeloid leukemia (AML), chronic myeloid leukemia (CML), chronic myelomonocytic leukemia, thrombolytic leukemia, a myelodysplastic syndrome (MDS), a myeloproliferative disorder, refractory anemia, a preleukemia syndrome, a lymphoid leukemia, lymphoma, non-Hodgkin's lymphoma or an undifferentiated leukemia.
14. The method of any one of paragraphs 11-13, wherein the cancer is myelodysplastic syndrome (MDS) or acute myeloid leukemia (AML).
15. The method of any one of paragraphs 11-14, wherein the cancer is refractory, non-responsive or resistant to chemotherapy and/or haploidentical stem cell transplantation.
16. The method of paragraph 15, wherein the cancer is resistant to azacitidine, decitabine, lenalidomide, TXA-127, or combinations thereof.
17. The method of any one of paragraphs 1-16, wherein the compound of formula (I) is administered in an amount from 5 mg to 120 mg.
18. The method of any one of paragraphs 1-17, wherein the compound of formula (I) is administered in an amount of about 60 mg.
19. The method of any one of paragraphs 1-18, wherein the DNA hypomethylating agent is administered in an amount from 5 mg/m² to 125 mg/m².
20. The method of any one of paragraphs 1-19, wherein the DNA hypomethylating agent is administered in an amount of 75 mg/m².
21. The method of any one of paragraphs 1-20, wherein the compound of formula (I) is administered orally and the hypomethylating agent is administered intravenously or subcutaneously.
22. A method of treating chemoresistant cancer comprising administering to a subject in need thereof an effective amount of
   (i) a DNA hypomethylating agent; and
   (ii) a compound of formula (Id): 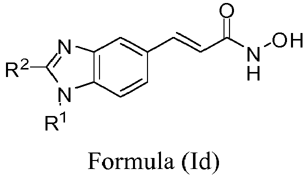 wherein
      R¹ is a group having the formula:

         -(CR²⁰R²¹)*ₘ*-(CR²²R²³)*ₙ*-(CR²⁴R²⁵)*ₒ*-NR²⁶R²⁷;
      R² is alkyl, fluoroalkyl, cyano, C₂-C₆alkenyl, C₂-C₆alkynyl, or heteroalkyl optionally substituted with =O;
      each R²⁰, R²¹, R²², R²³, R²⁴, and R²⁵ is independently H or methyl;
      each R²⁶ and R²⁷ is independently H, hydroxylalkyl, or alkyl; and
      m, n, and o are independently integers of 0, 1, 2, 3, or 4;
      or a pharmaceutically acceptable salt or prodrug thereof.
23. The method of paragraph 22, wherein the cancer is a hematological malignancy.
24. The method of any one of paragraphs 22-23, wherein the cancer is myelodysplastic syndrome (MDS) or acute myeloid leukemia (AML).
25. The method of any one of paragraphs 22-24, wherein the cancer is resistant to azacitidine, decitabine, lenalidomide, TXA-127, or combinations thereof.
26. The method of any one of paragraphs 22-25, wherein the compound of formula (I) has the structure: 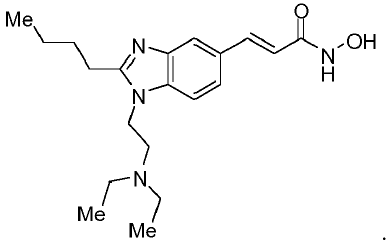
27. The method of any one of paragraphs 22-26, wherein the DNA hypomethylating agent is 5-azacytidine (azacitidine).
28. A kit comprising one or more containers filled with a DNA hypomethylating agent and one or more containers filled with a compound of formula (Id): 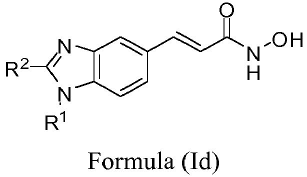 wherein
   R¹ is a group having the formula:

      -(CR²⁰R²¹)*ₘ*-(CR²²R²³)*ₙ*-(CR²⁴R²⁵)*ₒ*-NR²⁶R²⁷;
   R² is alkyl, fluoroalkyl, cyano, C₂-C₆alkenyl, C₂-C₆alkynyl, or heteroalkyl optionally substituted with =O;
   each R²⁰, R²¹, R²², R²³, R²⁴, and R²⁵ is independently H or methyl;
   each R²⁶ and R²⁷ is independently H, hydroxylalkyl, or alkyl; and
   m, n, and o are independently integers of 0, 1, 2, 3, or 4;
   or a pharmaceutical composition thereof.
29. The kit of paragraph 28, wherein the DNA hypomethylating agent is 5-azacytidine (azacitidine) and the compound of formula (I) has the structure:

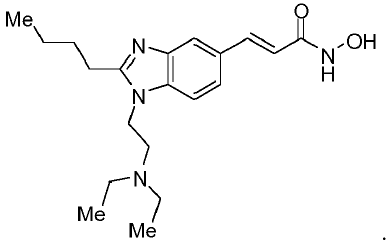

## Claims

1. A combination of azacitidine and pracinostat of formula: 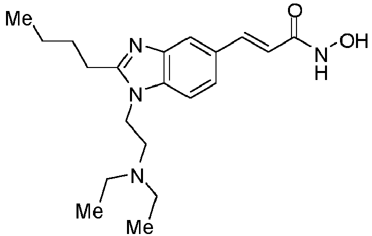 for use in treating patients with myelodysplastic syndrome (MDS); wherein pracinostat is administered in an amount from about 5 mg to about 120 mg.

2. A combination of azacitidine and pracinostat for use according to claim 1, wherein pracinostat is administered in an amount from about 30 mg to about 60 mg.

3. A combination of azacitidine and pracinostat for use according to claim 1, wherein pracinostat is administered in an amount of about 45 mg.

4. A combination of azacitidine and pracinostat for use according to claim 1, wherein pracinostat is administered in an amount of about 60 mg.

5. A combination of azacitidine and pracinostat for use according to any one of claims 1 to 4, wherein azacitidine is administered in an amount from about 5 mg/m² to about 125 mg/m².

6. A combination of azacitidine and pracinostat for use according to any one of claims 1 to 5, wherein azacitidine is administered in an amount of about 75 mg/m².

7. A combination of azacitidine and pracinostat for use according to any one of claims 1 to 6, wherein pracinostat is administered orally and azacitidine is administered intravenously or subcutaneously.

8. A combination of azacitidine and pracinostat for use according to any one of claims 1 to 7, wherein the MDS is intermediate-2 or high risk MDS.

9. A combination of azacitidine and pracinostat for use according to any one of claims 1 to 8, wherein the MDS is intermediate-2 MDS.

10. A combination of azacitidine and pracinostat for use according to any one of claims 1 to 8, wherein the MDS is high risk MDS.

11. A combination of azacitidine and pracinostat for use according to any one of claims 1 to 10, wherein the patients have high-risk cytogenetics.

12. A combination of azacitidine and pracinostat for use according to any one of claims 1 to 11, wherein the MDS is treatment related MDS.
